# EUROPEAN PATENT SPECIFICATION

(11) **EP 3 416 662 B1**
(45) Date of publication and mention of the grant of the patent: **29.07.2020**
(21) Application number: 17710054.2
(22) Date of filing: 16.02.2017
(51) Int. Cl.: A61K 35/28, A61P 35/00

(54) **METHODS AND COMPOSITIONS FOR TREATING CANCERS AND NEOPLASMS**
VERFAHREN UND ZUSAMMENSETZUNGEN ZUR BEHANDLUNG VON KARZINOMEN UND NEOPLASMEN
MÉTHODES ET COMPOSITIONS POUR LE TRAITEMENT DE CANCERS ET DE NÉOPLASMES

(30) Priority: 18.02.2016 US 201662296621 P
(43) Date of publication of application: 26.12.2018
(73) Proprietor: Pluristem Ltd., 31905 Haifa (IL)
(72) Inventor: ABERMAN, Zami, 4060541 Tel-Mond (IL); OFIR, Rachel, 1794000 Adi (IL); ALLEN, Hoshea Yissachar, 9963018 Bet Shemesh (IL); GILERT, Ariel, 3475912 Haifa (IL); SHRAGA HELED, Niva, 2242407 Nahariya (IL)
(74) Representative: Breuer Friedrich Hahner Patentanwälte PartG mbB
(86) International application number: PCT/IB2017/050868
(87) International publication number: WO 2017/141181

(56) References cited:
- WO-A1-2011/094181
- WO-A1-2016/098061
- WO-A1-2016/151476
- WO-A2-2007/011693
- WO-A2-2008/019148
- WO-A2-2009/045360
- WO-A2-2010/026575
- JEFFREY L. SPEES ET AL: "Mechanisms of mesenchymal stem/stromal cell function", STEM CELL RESEARCH & THERAPY, vol. 7, no. 1, 31 August 2016 (2016-08-31) , XP055371669, DOI: 10.1186/s13287-016-0363-7
- PAZ L. GONZÁLEZ ET AL: "Chorion Mesenchymal Stem Cells Show Superior Differentiation, Immunosuppressive, and Angiogenic Potentials in Comparison With Haploidentical Maternal Placental Cells : Chorion MSCs Outmatch Other Placental Cells", STEM CELLS TRANSLATIONAL MEDICINE : SCTM, vol. 4, no. 10, 13 August 2015 (2015-08-13), pages 1109-1121, XP055371771, Durham ISSN: 2157-6564, DOI: 10.5966/sctm.2015-0022
- ADI LAHIANI ET AL: "Human PLacental eXpanded (PLX) mesenchymal-like adherent stromal cells confer neuroprotection to nerve growth factor (NGF)-differentiated PC12 cells exposed to ischemia by secretion of IL-6 and VEGF", BIOCHIMICA ET BIOPHYSICA ACTA. MOLECULAR CELL RESEARCH., vol. 1853, no. 2, 1 February 2015 (2015-02-01), pages 422-430, XP055371957, NL ISSN: 0167-4889, DOI: 10.1016/j.bbamcr.2014.11.009

## Description

### FIELD

Described herein are methods of anti-tumor therapy using placental cells.

### SUMMARY

Previous work has established that the act of culturing adherent stromal cells (ASC) under 3D conditions produces ASC with heretofore undescribed properties and characteristics. Described herein are methods of using ASC for treatment, prevention, and inhibition of growth of cancers, tumors, and neoplasms.

In certain embodiments, the described ASC have been prepared by culturing in 2-dimensional (2D) culture, 3-dimensional (3D) culture, or a combination thereof. Non-limiting examples of 2D and 3D culture conditions are provided in the Detailed Description and in the Examples. Alternatively or in addition, the cells have been treated, in some embodiments, with pro-inflammatory cytokines; and/or are a placental cell preparation. In certain embodiments, the placental cell preparation is predominantly fetal cells; predominantly maternal cells; or a mixture of fetal and maternal cells, which is, in more specific embodiments, enriched for fetal cells or enriched for maternal cells. The term "ASC", except where indicated otherwise, may refer, in various embodiments, to adherent stromal cells either before or after incubation with pro-inflammatory cytokines. In still other embodiments, ASC refers to adherent stromal cells that have not been incubated with pro-inflammatory cytokines.

Alternatively or in addition, the cells are mesenchymal-like ASC, which exhibit a marker pattern similar to mesenchymal stromal cells, but do not differentiate into osteocytes, under conditions where "classical" mesenchymal stem cells (MSC) would differentiate into osteocytes. In other embodiments, the cells exhibit a marker pattern similar to MSC, but do not differentiate into adipocytes, under conditions where MSC would differentiate into adipocytes. In still other embodiments, the cells exhibit a marker pattern similar to MSC, but do not differentiate into either osteocytes or adipocytes, under conditions where mesenchymal stem cells would differentiate into osteocytes or adipocytes, respectively. The MSC used for comparison in these assays are, in some embodiments, MSC that have been harvested from bone marrow (BM) and cultured under 2D conditions. In other embodiments, the MSC used for comparison have been harvested from BM and cultured under 2D conditions, followed by 3D conditions.

In various embodiments, the described ASC are able to exert the described therapeutic effects, each of which is considered a separate embodiment, with or without the ASC themselves engrafting in the host. For example, the cells may, in various embodiments, be able to exert a therapeutic effect, without themselves surviving for more than 3 days, more than 4 days, more than 5 days, more than 6 days, more than 7 days, more than 8 days, more than 9 days, more than 10 days, or more than 14 days; or the cells survive for more than 3 days, more than 4 days, more than 5 days, more than 6 days, more than 7 days, more than 8 days, more than 9 days, more than 10 days, or more than 14 days.

Reference herein to "growth" of a population of cells is intended to be synonymous with expansion of a cell population.

Except where otherwise indicated, all ranges mentioned herein are inclusive.

Except where otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the embodiments of the invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
**FIG. 1** is a diagram of a bioreactor that can be used to prepare the cells.
**FIG. 2** contains plots of expression of stimulatory and co-stimulatory molecules on ASC. **Upper left:** Expression of CD80. **Upper right:** Expression of CD86. **Lower left.** Expression of CD40. **Lower right:** Expression of HLA-A/B/C. Negative controls were prepared with relevant isotype fluorescence molecules. Dotted, light, and heavy lines indicate marker-expression by placental ASC, BM cells, and mononuclear cells (MNC), respectively.
**FIG. 3** is a graph of a secretion profile of ASC under normoxic or hypoxic conditions.
**FIG. 4A** is a graph depicting secretion, measured by fluorescence, of various factors following incubation of ASC with TNF-a + IFN-g (unfilled bars) or control media (filled bars). **B-C** are graphs depicting fold-increase of secretion, measured by fluorescence, of GRO, IL-8, MCP-1, and RANTES **(B);** and IL-6, MCP-3, Angiogenin, Insulin-like Growth Factor Binding Protein-2 (IGFBP-2), Osteopontin, and Osteoprotegerin **(C)** following incubation of ASC with TNF-a alone, relative to incubation with control media (no cytokines).
**FIGs. 5A-B** are graphs depicting fold-increase relative to control medium (containing no cytokines) in secretion of MCP-1 **(A)** and GM-CSF **(B)** in several experiments, as measured by ELISA.
**FIGs. 6A-B** are graphs depicting secretion of various factors by TNF-a + IFN-g **(A)** or TNF-a alone **(B)** in the presence or absence of FBS. In **(A),** gray, white, and black bars indicate TNF-a + IFN-g; TNF-a + IFN-g + FBS; and control (no cytokines or serum), respectively. In **(B),** gray, white, and black bars indicate TNF-a alone; TNF-a + FBS; and control (no cytokines or serum), respectively.
**FIG. 7** is a plot showing population doubling time (PDT; vertical axis), in cells stimulated in a bioreactor with various concentration of cytokines (indicated in Table 5) for 40 hrs. (leftmost 7 groups) or 24 hrs. (rightmost 5 groups).
**FIG. 8A** is a graphical representation of the scores for each profiled gene for the breast cancer cell lines marker gene analysis. **B** is centroid plot showing the mean expression value for the 5 breast cancer cell lines for all of the genes downregulated (scores < -5) in the responsive breast cell lines, with 2 responsive breast cancer cell lines (HCC-1395 and MDA-MB-231) shown on the left, and the other 3 breast cancer cell lines (BT474, MCF7 and T47D) shown on the right. The error bars depict the standard deviation.
**FIGs. 9A-B** are tables summarizing the genes in the MHC Class I antigen processing and presentation pathway **(A)** and the cytokine signaling pathway **(B)** that are downregulated and/or exclusively mutated in each of the responsive cell lines.
**FIG. 10** is a heat map showing expression of 305 classifier genes useful for characterizing breast cancer lines as Luminal, Basal A, or Basal B by hierarchical clustering as per Neve et al.

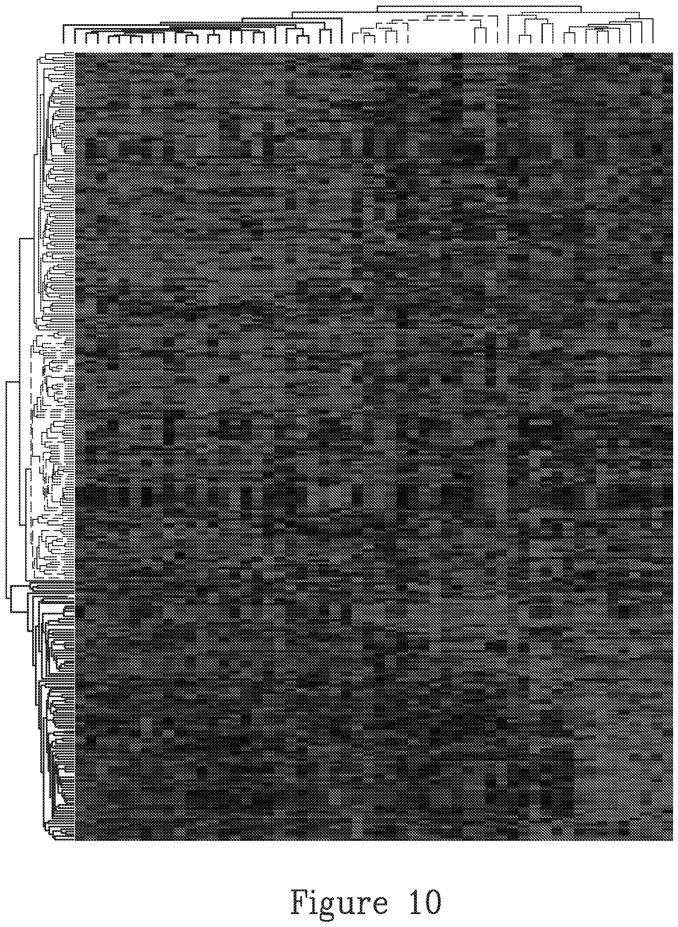

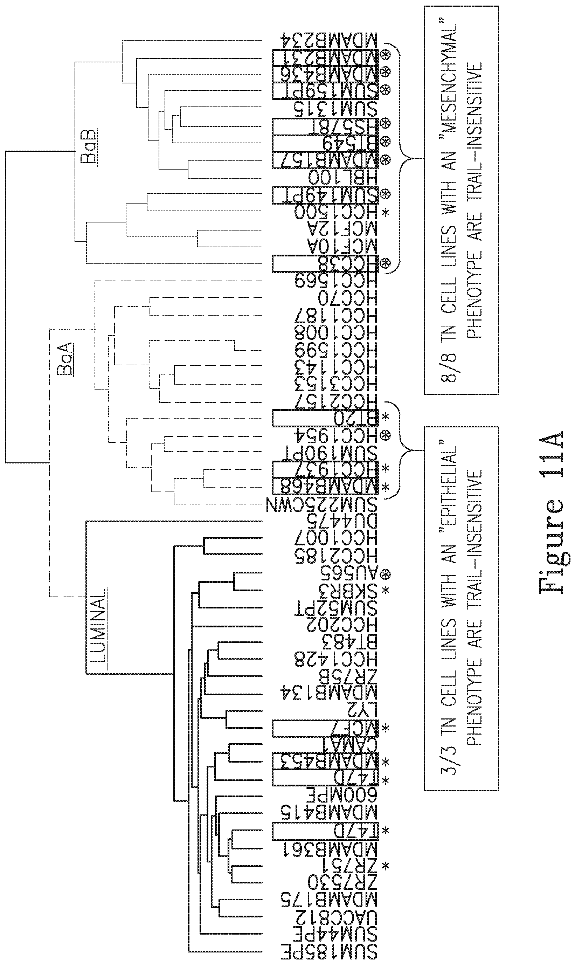

**FIG. 11A** is a classification tree corresponding to a close-up view of the top of **Fig. 10****,** and showing which breast cancer cell lines were characterized for TRAIL sensitivity and ASC sensitivity. The figure also incorporates data from Rahman et al. Plain and circled asterisks denote breast cell lines tested for TRAIL sensitivity and found to be TRAIL insensitive or TRAIL-sensitive, respectively. Enclosure in a box denotes lines that were tested for both ASC sensitivity and TRAIL sensitivity. (The first occurrence of T47D should read "BT474"). **B** depicts the data from tested breast cancer cell lines from **A** in tabular form, and also includes information on clinical sub-type, namely whether or not the cell lines are ER positive, PR positive, or Her2/neu-amplified.

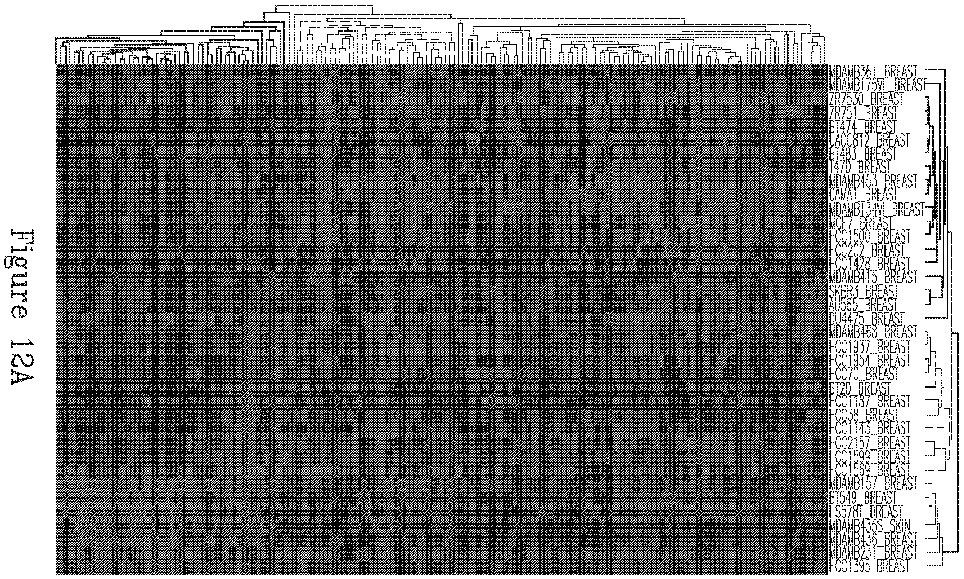

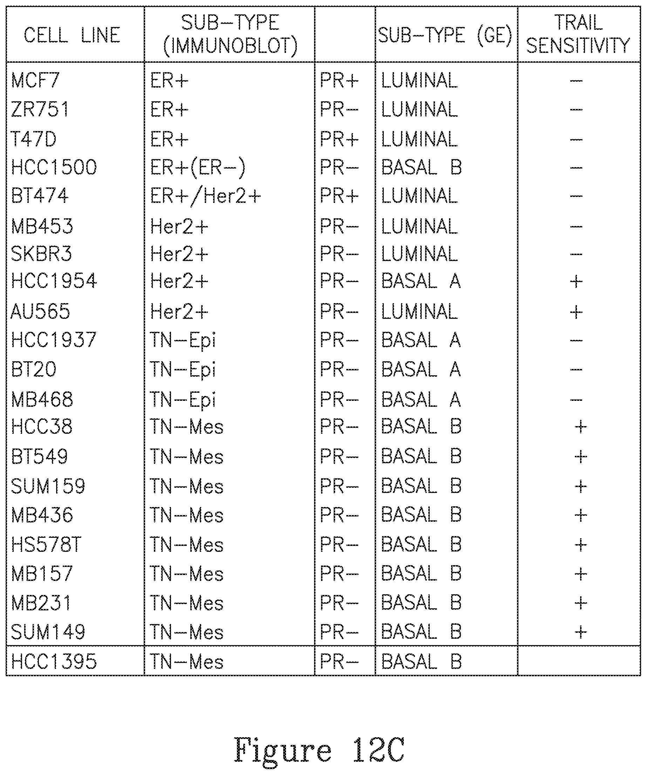

**FIG. 12A** is a heat map showing expression of 169 probe sets used for another hierarchical clustering, using data from the Cancer Cell Line Encyclopedia (CCLE). **B** is a classification tree corresponding to a close-up view of the top of **A,** showing which breast cancer cell lines were characterized for ASC sensitivity. **C** depicts the data from tested breast cancer cell lines from **B** in tabular form, also including information on clinical sub-type, namely whether or not the cell lines are ER positive, PR positive, or Her2/neu amplified. Cell lines that grouped differently from the previous analysis are circled in **B.**
**FIG. 13** is a listing **(right side)** of the pathways in which classifier genes of each section of the aforementioned hierarchical clustering analysis participate. The heatmap is reproduced on the **left side**).

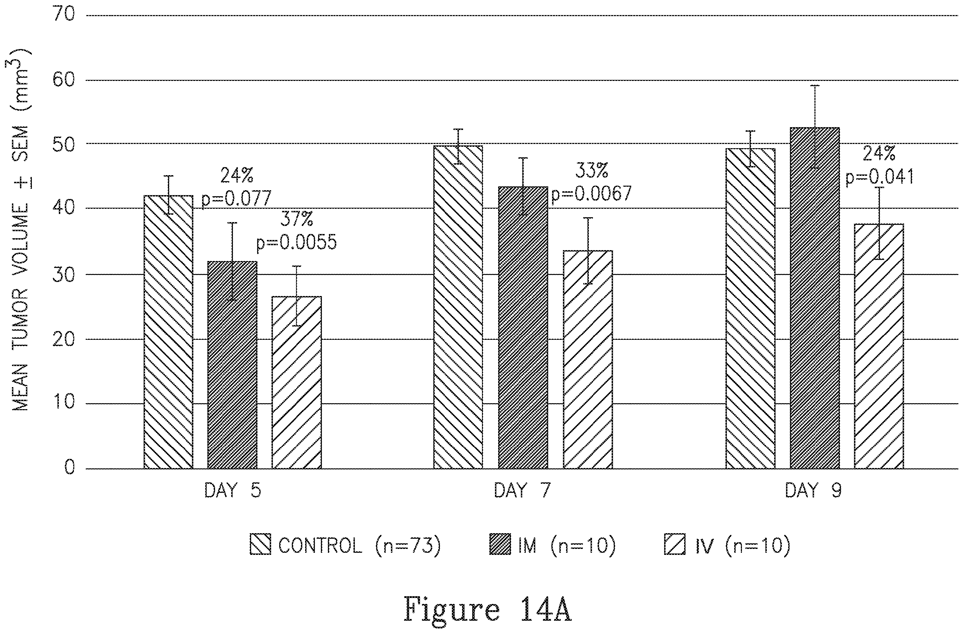

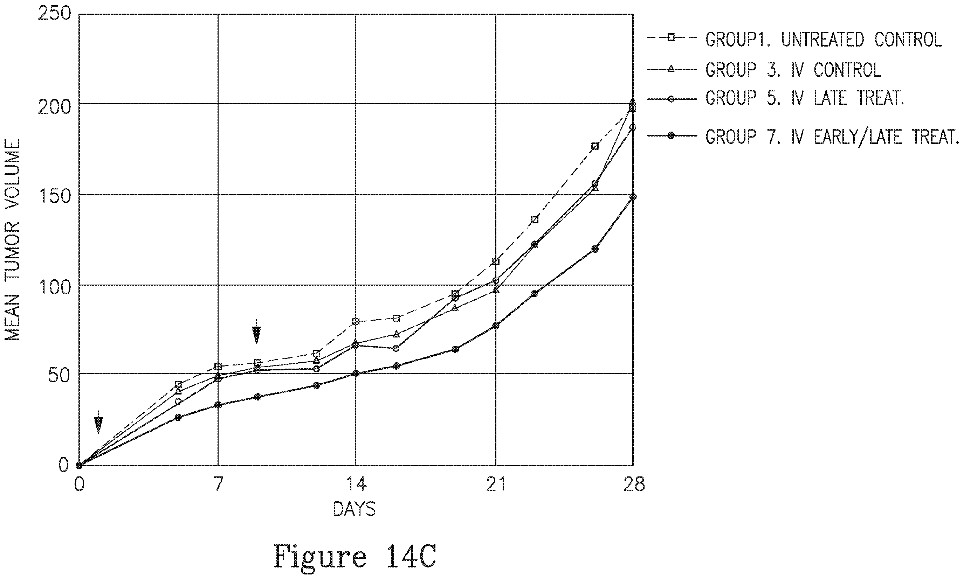

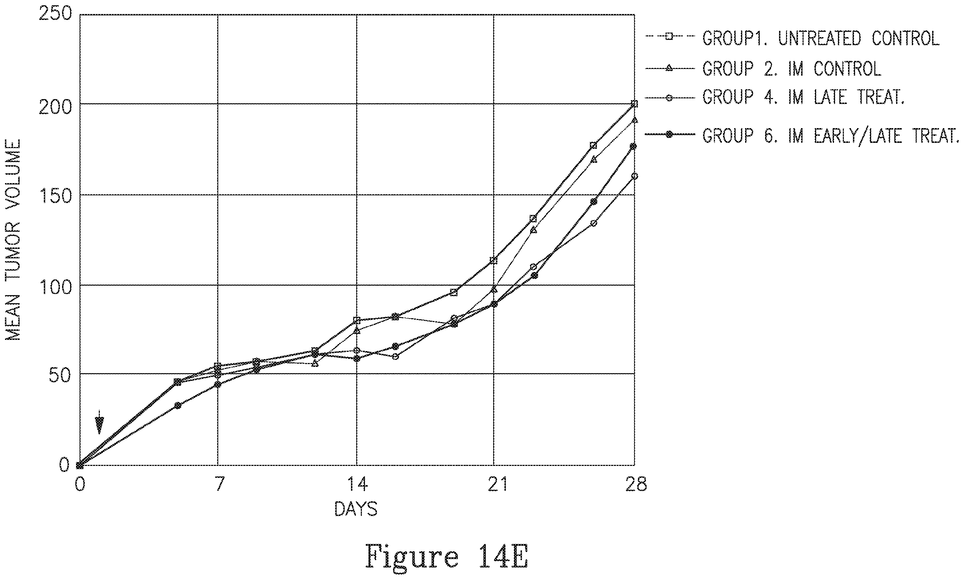

**FIG. 14A** is a bar graph showing the mean volume (mm³) of implanted tumors in mice untreated or treated with ASC IM or IV (first, second and third bars from left, respectively). Left, middle, and right bars in each series are the control, IM, and IV groups, respectively. Left, center, and right datasets depict tumor sizes at days 5, 7, and 9, respectively. **B** is a bar graph showing average tumor sizes from each timepoint for IV-injected mice, and **C** is a plot showing the same data. **D** is a bar graph showing average tumor sizes from each timepoint for IM-injected mice, and **E** is a plot showing the same data.
**FIG. 15** presents data from IM-treated mice. **A** is a plot of tumor volume (mm³; vertical axis) *vs.* time (days; horizontal axis). **B-C** are bar graphs of tumor volume (mm³; vertical axis) on day 82 in the following groups (horizontal axis, from left to right): IM-ASC, IM-vehicle, and IM-ASC/late. **D-E** are plots of percent change in tumor volume from day 47 (vertical axis) vs. time (days; horizontal axis). In **C** and **E,** outliers were removed to generate "trimmed" numbers.
**FIG. 16** is a plot of percent tumor growth inhibition (vertical axis) vs. time (days; horizontal axis), in IM-ASC-, IM-ASC-late-, IV-ASC-, and IV/IM-ASC-treated mice.

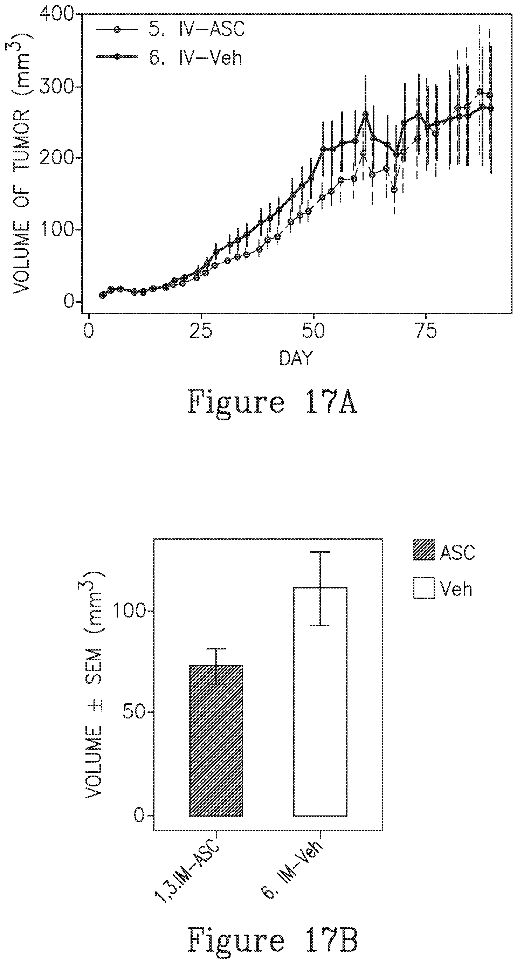

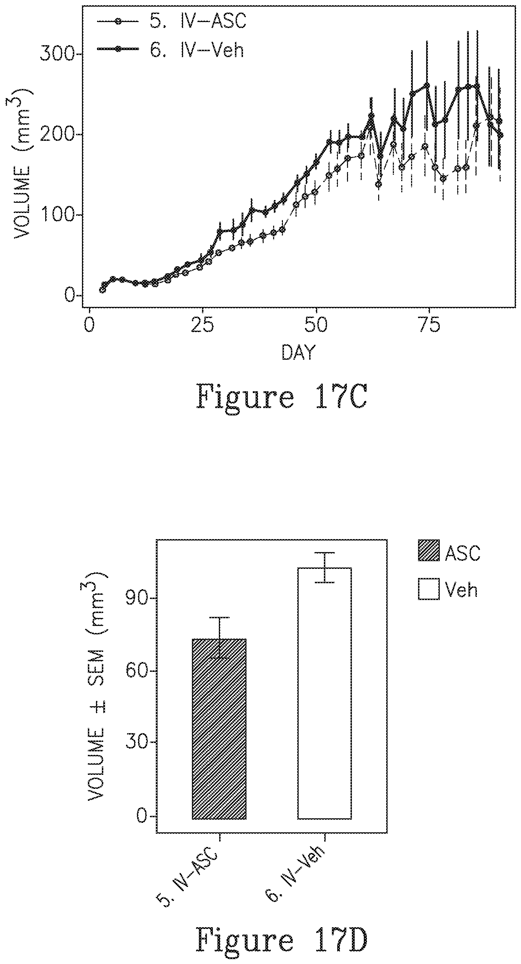

**FIG. 17** presents data from IV-treated mice. **A** and **C** are plots of tumor volume (mm³; vertical axis) *vs.* time (days; horizontal axis). **B** and **D** are bar graphs of tumor volume (mm³; vertical axis) on day 38 in mice treated with IV-ASC **(left bar)** or IV-vehicle **(right bar).** In **C** and **D,** outliers were removed to generate "trimmed" numbers.
**FIG. 18** is a bar graph of the proliferation index (vertical axis) of tumors of mice treated with IM-vehicle **(left bar)** or IM-ASC-late **(right bar).**
**FIG. 19** is a bar graph of area occupied by CD34⁺ cells (vertical axis) in tumors of mice treated with IM-vehicle **(left bar)** or IM-ASC-late **(right bar).**
**FIG. 20** is a bar graph of area occupied by CD34⁺ cells (vertical axis) in tumors of mice treated with IV-vehicle **(left bar)** or IV-ASC-late **(right bar).**
**FIGS. 21A-B** are plots showing fluorescence (vertical axis) following CyQUANT GR staining, which produces a fluorescent signal proportional to the number of cells in the plate. NCI-H460 **(A)** or MDA-MB231 **(B)** cells were seeded at initial densities of 1500, 3000, 6000 or 12,000 cells/well (shown in 4 datasets in each plot, from left to right) and 1 day later were exposed for 3 days to growth medium alone (solid circles) or ASC-CM (asterisks, triangles, and diamonds). Also plotted is fluorescence in baseline plates (squares) frozen 1 day after seeding.
**FIG. 22A** is a perspective view of a carrier (or "3D body"), according to an exemplary embodiment. **B** is a perspective view of a carrier, according to another exemplary embodiment. **C** is a cross-sectional view of a carrier, according to an exemplary embodiment.
**FIG. 23** is a theoretical plot, provided for illustrative purposes only, of the logarithm of the relative population size of a cell culture against time. µₘ is the maximal cell division rate, λ denotes the end of stationary phase, and A is the asymptote.

### DETAILED DESCRIPTION

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

In certain embodiments, there is provided a use in a method of treating a breast carcinoma in a subject in need thereof, the method comprising the step of administering to the subject a therapeutically effective amount of adherent stromal cells (ASC), thereby treating the cancer, wherein said ASC originate from placenta tissue. Also described is provided a method of treating a neoplasm in a subject in need thereof, the method comprising the step of administering to the subject the ASC. The ASC may be derived from a placenta or from adipose tissue, or from other sources as described herein. As provided herein, administration of ASC is useful in treating neoplastic growths.

More specifically, ASC were shown to inhibit growth of a variety of tumor cell lines in spheroid studies (Example 10). Furthermore, *in vivo* studies showed that administration of ASC halted or inhibited growth of implanted tumors. In a first study (Example 16), IV administration exhibited an effect of clear statistical significance, while IM administration showed at least a trend of efficacy. In a second study (Example 17), IM administration conferring a lasting effect, and IV administration conferred at least a temporary inhibition of tumor growth. Both IM and IV ASC treatment completely prevented lung metastases, and IM treatment reduced axillary lymph node metastases, which are the primary drainage lymph nodes of the mammary fat pads (Kobayashi H *et al*)*.*

Also described is a method of preventing a neoplastic growth in a subject in need thereof, the method comprising the step of administering to the subject a therapeutically effective amount of ASC, thereby preventing the neoplastic growth in the subject. Also described is a method of reducing the incidence of a neoplastic growth in a subject at risk thereof, the method comprising the step of administering to the subject the ASC. The neoplastic growth may be a cancer, a tumor, or a neoplasm.

Also described is a method of inhibiting growth of a tumor in a subject in need thereof, the method comprising the step of administering to the subject a therapeutically effective amount of ASC, thereby inhibiting growth of the tumor in the subject. Also describedis a method of inhibiting growth of a cancer, the method comprising the step of administering to the subject the ASC.

Also described is a method of inhibiting a metastasis of a tumor in a subject at risk thereof, the method comprising the step of administering to the subject a therapeutically effective amount of ASC. IAlso described is a method of inhibiting a metastasis of a cancer, the method comprising the step of administering to the subject the ASC. In certain embodiments, the subject has a primary tumor, which is inoperable. In other embodiments, the primary tumor is operable.

Also describedis a method of treating a cancer in a subject in need thereof, the method comprising the step of administering to the subject a therapeutically effective amount of CM derived from ASC, thereby treating the cancer. Also described is a method of treating a neoplasm in a subject in need thereof, the method comprising the step of administering to the subject the CM. The ASC may be derived from a placenta or from adipose tissue, or from other sources as described herein.

Also described is a method of preventing a neoplastic growth in a subject in need thereof, the method comprising the step of administering to the subject a therapeutically effective amount of CM derived from ASC, thereby preventing the neoplastic growth in the subject. Also describedis a method of reducing the incidence of a neoplastic growth in a subject at risk thereof, the method comprising the step of administering to the subject the CM. The neoplastic growth may be a cancer, a tumor, or a neoplasm.

Also describedis a method of inhibiting growth of a tumor in a subject in need thereof, the method comprising the step of administering to the subject a therapeutically effective amount of CM derived from ASC, thereby inhibiting growth of the tumor in the subject. Also describedis a method of inhibiting growth of a cancer, the method comprising the step of administering to the subject the CM.

Also described is a method of inhibiting a metastasis of a tumor in a subject at risk thereof, the method comprising the step of administering to the subject a therapeutically effective amount of CM derived from ASC. Also describedis a method of inhibiting a metastasis of a cancer, the method comprising the step of administering to the subject the CM. In certain embodiments, the subject has a primary tumor, which is inoperable. In other embodiments, the primary tumor is operable. In more specific embodiments, the tumor may be less about 200 mm³, between 50-200 mm³, between 100-200 mm³, between 125-1000 mm³, between 150-1000 mm³, between 200-1000 mm³, between 250-1000 mm³, between 300-1000 mm³, over 150 mm³, over 200 mm³, or over 300 mm³.

In each case, the described ASC may be derived from a placenta or from adipose tissue, or from other sources described herein.

Except where indicated otherwise, treatment with ASC refers to treatment of cancer cells with whole, live ASC. In alternative embodiments, the cancer cells are treated with fractions of ASC.

Except where indicated otherwise, treatment with conditioned medium (CM) refers to treatment of cancer cells with medium that has been incubated with ASC. Sometimes, the cancer cells are treated with fractions of CM that has been incubated with ASC, or with factors derived from CM that has been incubated with ASC.

The cancer can be selected from: acute lymphoblastic leukemia, adrenocortical carcinoma, AIDS-related lymphoma, anal cancer, appendix cancer, astrocytoma (childhood cerebellar or cerebral), basal cell carcinoma, bile duct cancer, bladder cancer, bone cancer, brainstem glioma, brain tumor (cerebellar astrocytoma, cerebral astrocytoma/malignant glioma, ependymoma, medulloblastoma, supratentorial primitive neuroectodermal tumor, visual pathway and hypothalamic gliomas), breast cancer, bronchial adenoma, carcinoid tumor of the lung, gastric carcinoid, other carcinoid tumors (e.g. childhood), Burkitt lymphoma, carcinoma of unknown primary, central nervous system lymphoma (e.g. primary), cerebellar astrocytoma, malignant glioma (e.g. cerebral astrocytoma), cervical cancer, chronic lymphocytic leukemia, chronic myelogenous leukemia, colon cancer, cutaneous T-cell lymphoma, desmoplastic small round cell tumor, endometrial cancer, ependymoma, esophageal cancer, Ewing's sarcoma, extracranial germ cell tumor (e.g. childhood), extragonadal germ cell tumor, extrahepatic bile duct cancer, eye cancer (e.g. intraocular melanoma, retinoblastoma), gallbladder cancer, gastric (stomach) cancer, gastrointestinal stromal tumor, germ cell tumor (e.g. childhood extracranial), gestational trophoblastic tumor, hairy cell leukemia, head and neck cancer, hepatocellular (liver) cancer, Hodgkin lymphoma, other lymphomas (AIDS-related, non-Hodgkin, primary central nervous system), hypopharyngeal cancer, intraocular melanoma, islet cell carcinoma, Kaposi sarcoma, laryngeal cancer, leukemias (e.g. acute lymphoblastic, chronic lymphocytic, chronic myelogenous, hairy cell), lip and oral cavity cancer, primary liver cancer, small cell lung cancers, non-small cell lung cancer, macroglobulinemia (Waldenstrom), malignant fibrous histiocytoma of bone, medulloblastoma (e.g. childhood), intraocular melanoma, other melanomas, Merkel cell carcinoma, mesotheliomas (e.g. adult malignant, childhood), metastatic squamous neck cancer with occult primary, mouth cancer, multiple endocrine neoplasia syndrome (e.g. in a pediatric patient), plasma cell neoplasms (e.g. multiple myeloma), mycosis fungoides, myelogenous leukemia (e.g. chronic), nasal cavity and paranasal sinus cancer, nasopharyngeal carcinoma, neuroblastoma, oral cancer, oropharyngeal cancer, osteosarcoma, ovarian cancer, ovarian epithelial cancer (e.g. surface epithelial-stromal tumor), ovarian germ cell tumor, ovarian low malignant potential tumor, islet cell pancreatic cancer, other pancreatic cancers, paranasal sinus and nasal cavity cancer, parathyroid cancer, penile cancer, pharyngeal cancer, pheochromocytoma, pineal astrocytoma, pineal germinoma, pineoblastoma and supratentorial primitive neuroectodermal tumors (childhood), pituitary adenoma, plasma cell neoplasia, pleuropulmonary blastoma, primary central nervous system lymphoma, prostate cancer, rectal cancer, renal cell carcinoma (kidney cancer), renal pelvis and ureter transitional cell cancer, retinoblastoma, rhabdomyosarcoma (childhood), salivary gland cancer, soft tissue sarcoma, uterine sarcoma, Sezary syndrome, melanoma, skin carcinoma (e.g. Merkel cell), other skin cancers, small intestine cancer, squamous cell carcinoma, supratentorial primitive neuroectodermal tumor (e.g. childhood), testicular cancer, throat cancer, thymoma (e.g. childhood), thymic carcinoma, thyroid cancer (childhood or adult), urethral cancer, endometrial uterine cancer, vaginal cancer, vulvar cancer, Waldenstrom macroglobulinemia, and Wilms tumor.

In certain embodiments, the treated tumor is sensitive to TRAIL (also known as Tumor necrosis factor ligand superfamily member 10 or Apo-2L; Uniprot accession no. P50591. Uniprot was accessed on December 29, 2015). Those skilled in the art will appreciate that the TRAIL-sensitivity of a cell or cell line can be readily determined. Exemplary protocols for doing so are described in James MA *et al* and the references cited therein. Exemplary protocols for confirming that tumor growth inhibition or death induction is TRAIL-mediated are described in the product literature for anti-TRAIL antibody [75411.11] (ab10516, Abcam), in Roux *et al,* and the references cited therein.

The cancer or neoplasm may be selected from osteosarcoma, prostate carcinoma, urothelial bladder carcinoma, renal cell adenocarcinoma, gastric adenocarcinoma, pancreatic adenocarcinoma, breast ductal carcinoma, hepatocellular carcinoma, squamous cell carcinoma, thyroid anaplastic carcinoma, lung anaplastic carcinoma, melanoma, colorectal adenocarcinoma, glioblastoma, prostate carcinoma, ovarian clear cell carcinoma, uterine sarcoma, lung adenocarcinoma, bronchoalveolar carcinoma, large cell lung carcinoma, rhabdomyosarcoma, neuroblastoma, astrocytoma, and rectum adenocarcinoma. In certain embodiments, the tumor is TRAIL-sensitive.

In certain embodiments, the tumor is a breast carcinoma, or in other embodiments, an adenocarcinoma. In certain embodiments, the breast cancer has a mesenchymal phenotype. Those skilled in the art will appreciate that breast cancer cells with a mesenchymal phenotype typically have high expression levels of Vimentin (Uniprot accession no. P08670); and Caveolin-1 (Uniprot accession no. Q03135) and Caveolin-2 (Uniprot accession nos. P51636 and Q712N7), and low levels of E-cadherin (Uniprot accession no. P12830). Alternatively or in addition, the breast tumor is TRAIL-sensitive and/or is a triple-negative (TN) tumor. Those skilled in the art will appreciate that TN breast cancer cells lack receptors for estrogen (ER; Uniprot accession no. P03372) and progesterone (PR; Uniprot accession no. P06401), and do not have an amplification in human epidermal growth factor receptor 2 (HER2; Uniprot accession no. P04626) gene copy number or expression. The presence of these receptors can be readily ascertained, for example by fluorescence-activated cell sorting. The Uniprot entries mentioned in this paragraph were accessed on December 29, 2015 or January 3, 2016.

The cancer or neoplasm that is treated, or prevented, by the described compositions can be selected from metaplasias, dysplasias, neoplasias, and leukoplakias. The cancer or neoplasm can be selected from cancers of the breast, skin, prostate, colon, bladder, cervix, uterus, stomach, lung, esophagus, larynx, oral cavity. The cancer or neoplasm can be a solid tumor, which is selected from fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma. Sometimes, the neoplasm is a papilloma of the mucous membranes.

The cancer can be non-Hodgkin lymphoma, colorectal cancer, malignant melanoma, thyroid carcinoma, non-small cell lung carcinoma, or lung adenocarcinoma. The cancer or neoplasm can be selected from non-Hodgkin lymphoma, colorectal cancer, malignant melanoma, thyroid carcinoma, and non-small cell lung carcinoma (e.g. lung adenocarcinoma).

The cancer or neoplasm can be renal cell carcinoma, melanoma, breast carcinoma, hepatocellular carcinoma, colorectal adenocarcinoma, breast adenocarcinoma, lung adenocarcinoma, large cell lung carcinoma, or rhabdomyosarcoma. The cancer or neoplasm can be selected from renal cell carcinoma, melanoma, breast carcinoma, hepatocellular carcinoma, colorectal adenocarcinoma, breast adenocarcinoma, lung adenocarcinoma, large cell lung carcinoma, and rhabdomyosarcoma. The cancer or neoplasm can be selected from renal cell carcinoma, hepatocellular carcinoma, and lung adenocarcinoma. In certain embodiments, the tumor is TRAIL-sensitive.

In the case of a solid tumor, the described ASC or pharmaceutical composition comprising same are in some embodiments administered intra-tumorally; or in other embodiments, administered to the region of the body where the tumor is located; or in other embodiments, administered to the bed of an excised tumor to prevent recurrence of the neoplasm. In other embodiments, the ASC or composition is administered intramuscularly, subcutaneously, or systemically.

Also described is the use of ASC for the manufacture of a medicament identified for treating a cancer. Also describedis the use of ASC for the manufacture of a medicament identified for treating a neoplasm. Also describedis the use of ASC for the manufacture of a medicament identified for preventing or reducing an incidence of a neoplastic growth. Also described is the use of ASC for the manufacture of a medicament identified for suppressing metastasis of a neoplastic growth. Also provided is a pharmaceutical composition for inhibiting growth of a tumor or inhibiting growth of a neoplasm, comprising the described ASC. In certain embodiments, the tumor is a breast carcinoma, or in other embodiments is an adenocarcinoma. In certain embodiments, the breast cancer has a mesenchymal phenotype. Alternatively or in addition, the breast tumor is a TN tumor.

In still another embodiment is provided an article of manufacture, comprising (a) a packaging material, wherein the packaging material comprises a label describing use in treating breast carcinoma; and (b) a pharmaceutical composition comprising ASC, wherein said ASC originate from placenta tissue. In other embodiments, a pharmaceutical agent is contained within the packaging material, and the pharmaceutical agent is effective for treatingbreast carcinoma; and the packaging material comprises a label which indicates that the pharmaceutical agent can be used for the aforementioned use(s). In some embodiments, the pharmaceutical composition is frozen. In other embodiments, the label indicates use in treating a breast carcinoma. In still other embodiments, the label indicates use in inhibiting growth of a breast carcinoma. In still other embodiments, the label indicates use in preventing or reducing an incidence of a breast carcinoma. In certain embodiments, the tumor is a breast carcinoma, or in other embodiments is an adenocarcinoma. In certain embodiments, the breast cancer has a mesenchymal phenotype. Alternatively or in addition, the breast tumor is a TN tumor.

In other embodiments is provided a method of manufacturing an anti-breast carcinoma therapeutic agent, the method comprising the steps of co-incubating ASC with cancer cells, or in other embodiments a cancer cell line, and subsequently isolating the ASC, or isolating the conditioned medium ("CM") derived from the co-incubation, wherein said ASC or CM possesses anti-cancer activity and originate from placenta tissue.

In other embodiments is provided a method of manufacturing an anti-cancer therapeutic agent, the method comprising the steps of (a) culturing cancer cells, or in other embodiments a cancer cell line, and isolating the CM from the culturing, henceforth referred to as the "cancer cell CM"; (b) incubating ASC in the cancer cell CM; and (c) isolating the ASC, wherein said ASC possess anti-breast carcinoma activity and originate from placenta tissue. Alternatively, step (c) comprises isolating the CM derived from the ASC incubation (the "ASC CM"), wherein the ASC CM possesses anti-cancer activity. In other embodiments is provided a method of manufacturing an anti-cancer therapeutic agent, the method comprising the steps of (a) incubating ASC in a cancer cell CM; and (b) isolating the ASC, wherein said ASC possess anti-breast carcinoma activity, or isolating the ASC CM, where the ASC CM possesses anti-cancer activity and originate from placenta tissue.

In still other embodiments is provided a method of manufacturing an anti-breast carcinoma therapeutic agent, the method comprising the steps of (a) culturing cancer cells, or in other embodiments a cancer cell line, and isolating a fraction of the cancer cell CM or one or more factors derived from the cancer cell CM; (b) adding the aforementioned fraction or one or more factors to a culture medium; (c) incubating ASC in the medium derived from step (c); and (d) isolating the ASC, wherein said ASC possess anti-breast carcinoma activity and originate from placenta tissue.

Methods for determining the effect of cells and solutions (e.g. CM) on the viability and replication of cancer cells are well known in the art. In some embodiments, 3D plates are utilized to house the target cancer cells, to encourage formation of cell cultures. An exemplary type of suitable plates is Elplasia™ plates, which are commercially available from Kuraray Co., Ltd. (Tokyo, JP). Use of such plates is described inter alia in Kobayashi K et al, Nakamura et al, and the references cited therein. For co-culture models, cancer cells and ASCs can be labeled with various reagents such as CellTrace dyes (i.e. CSFE (carboxyfluorescein diacetate, succinimidyl ester [Lyons et al] and CellTrace™ Violet) or QTracker™ kits (both available from ThermoFisher Scientific). Apoptosis can be detected using reagents such as Annexin V-FITC and propidium iodide (PI) or TUNEL staining, both available from Roche. CyQUANT® (Invitrogen); Calcein AM (Molecular Probes™); and RealTime GLO, CellTiter GLO, and Alamar blue (all from Promega) can be used to determine cell viability. CyQUANT® can be detected using a fluorimeter, and CellTiter GLO can be detected using a luminometer. Inhibition of replication is evidence of therapeutic efficacy. Alternatively, ASC can be differentiated from cancer cells by staining both the cancer cells and ASC, using different colors. In still other embodiments, ASCs can be labeled with known MSC markers such as anti-CD73 or ant-CD105. Kits for determining the effects of cells and solutions on the viability and replication of cancer cells are commercially available from vendors such as Bioensis Preclinical Services. (Bellevue, WA). Methods for generating spheroids of cancer cells are well known in the art, and are described, for example, in Perche F et al, 2012, Friedrich J et al, 2009, Phung YT et al 2011, Korff T et al 1998, Ivascu A et al 2006, and the references cited therein. In a non-limiting protocol, 10,000 cells are added into each well of polyHEMA-coated 96-well plates. The plates are briefly spun for 5 minutes at 800 rpm and then placed in a 37°C humidified incubator with 5% CO₂ until spheroids form. Optionally, the basement membrane extract Matrigel™ may be added to the wells, in some embodiments as described in Ivascu A et al 2006. In another non-limiting protocol, microspheroids with an average of 250 cells each can be generated using non-adhesive hydrogels cast by micromolds. 3% agarose gels (Ultrapure agarose; Invitrogen, Carlsbad, CA) are cast by using micromolds, which produces recesses on the gel surface. The gels are then equilibrated overnight with complete culture medium. Trypsinized cells are resuspended to the appropriate cell density and then pipetted onto the gels. Over 24 hours (H1299) or 48 hours (A549), cells within the recesses form aggregates and are recovered from the gels by centrifugation. Other efficacy testing methods described herein are also suitable.

Additionally, animal tumor models are well known in the art, and include, inter alia, ectopic xenograft models, orthotopic xenograft models, genetically engineered tumor models, and carcinogen-induced tumor models. Such models are described inter alia in Ruggeri BA et al, Walker JD et al, Rocha NS et al, and the references cited therein. Methods for determining efficacy of anti-cancer treatment on human subjects are also well known in the art, and include tumor imaging, measurement of tumor marker proteins, and assessment of patient wellness, for example as described in Oh WK (Urol. Oncol. 2003), Ramsey et al, and the references cited therein. Other non-limiting examples of *in vivo* models are described herein.

Those skilled in the art will appreciate that animal models for prevention of metastasis are well known in the art, and include, without limitation, those described in Yang S *et al,* Bonnomet A *et al,* and Zhang F *et al,* and the inducible PyVmT mammary tumor model described in Jones LM *et al.* Patient-derived xenograph (PDX) models can also be used, including implantation of intact fragments of tumor tissue (Zhang Y *et al*) and use of humanized mice (Morton *et al* and the references cited therein).

### Methods for preparing ASC

ASC can be propagated, in some embodiments, by using two-dimensional ("2D") culturing conditions, three-dimensional ("3D") culturing conditions, or a combination thereof. Conditions for propagating ASC in 2D and 3D culture are further described hereinbelow and in the Examples section which follows. These steps may be freely combined with any of the other described embodiments for culturing methods, characteristics of the cells, or therapeutic parameters, each of which is considered a separate embodiment.

As mentioned, in some embodiments, the cells have been propagated under 2D culturing conditions. The terms "2D culture" and "2D culturing conditions" refer to a culture in which the cells are exposed to conditions that are compatible with cell growth and allow the cells to grow in a monolayer, which is referred to as a "two-dimensional (2D) culture apparatus". Such apparatuses will typically have flat growth surfaces, in some embodiments comprising an adherent material, which may be flat or curved. Non-limiting examples of apparatuses for 2D culture are cell culture dishes and plates. Included in this definition are multi-layer trays, such as Cell Factory™, manufactured by Nunc™, provided that each layer supports monolayer culture. It will be appreciated that even in 2D apparatuses, cells can grow over one another when allowed to become over-confluent. This does not affect the classification of the apparatus as "two-dimensional".

In other embodiments, the cells have been propagated under 3D culturing conditions. The terms "3D culture" and "3D culturing conditions" refer to a culture in which the cells are exposed to conditions that are compatible with cell growth and allow the cells to grow in a 3D orientation relative to one another. The term "three-dimensional [or 3D] culture apparatus" refers to an apparatus for culturing cells under conditions that are compatible with cell growth and allow the cells to grow in a 3D orientation relative to one another. Such apparatuses will typically have a 3D growth surface, in some embodiments comprising an adherent material. Certain, non-limiting embodiments of 3D culturing conditions suitable for expansion of ASC are described in PCT Application Publ. No. WO/2007/108003.

In various embodiments, "an adherent material" refers to a material that is synthetic, or in other embodiments naturally occurring, or in other embodiments a combination thereof. In certain embodiments, the material is non-cytotoxic (or, in other embodiments, is biologically compatible). Alternatively or in addition, the material is fibrous, which may be, in more specific embodiments, a fibrous matrix, e.g. a woven fibrous matrix, a non-woven fibrous matrix, or either. In still other embodiments, the material exhibits a chemical structure that enables cell adhesion, for example charged surface-exposed moieties. Non-limiting examples of adherent materials which may be used in accordance with this aspect include polyesters, polypropylenes, polyalkylenes, poly fluoro-chloro-ethylenes, polyvinyl chlorides, polystyrenes, polysulfones, poly-L-lactic acids, cellulose acetate, glass fibers, ceramic particles, and inert metal fiber; or, in more specific embodiments, polyesters, polypropylenes, polyalkylenes, polyfluorochloroethylenes, polyvinyl chlorides, polystyrenes, polysulfones, cellulose acetates, and poly-L-lactic acids. Other embodiments include Matrigel™, an extracellular matrix component (e.g., Fibronectin, Chondronectin, Laminin), and a collagen. In more particular embodiments, the material may be selected from a polyester and a polypropylene. Non-limiting examples of synthetic adherent materials include polyesters, polypropylenes, polyalkylenes, polyfluorochloroethylenes, polyvinyl chlorides, polystyrenes, polysulfones, cellulose acetates, and poly-L-lactic acids, glass fibers, ceramic particles, and inert metal fibers. In more specific embodiments, the synthetic adherent material is selected from polyesters, polypropylenes, polyalkylenes, polyfluorochloroethylenes, polyvinyl chlorides, polystyrenes, polysulfones, cellulose acetates, and poly-L-lactic acids.

Alternatively or in addition, the described ASC have been incubated in a 2D adherent-cell culture apparatus, prior to the step of 3D culturing. In some embodiments, cells (following extraction from, in some embodiments, placenta, adipose tissue, etc.) are then subjected to prior step of incubation in a 2D adherent-cell culture apparatus, followed by the described 3D culturing steps. This step may be freely combined with any of the other described embodiments for culturing methods, characteristics of the cells, or therapeutic parameters, each of which is considered a separate embodiment.

In other embodiments, the length of 3D culturing is at least 4 days; between 4-12 days; in other embodiments between 4-11 days; in other embodiments between 4-10 days; in other embodiments between 4-9 days; in other embodiments between 5-9 days; in other embodiments between 5-8 days; in other embodiments between 6-8 days; or in other embodiments between 5-7 days. In other embodiments, the 3D culturing is performed for 5-15 cell doublings, in other embodiments 5-14 doublings, in other embodiments 5-13 doublings, in other embodiments 5-12 doublings, in other embodiments 5-11 doublings, in other embodiments 5-10 doublings, in other embodiments 6-15 cell doublings, in other embodiments 6-14 doublings, in other embodiments 6-13 doublings, or in other embodiments 6-12 doublings, in other embodiments 6-11 doublings, or in other embodiments 6-10 doublings.

According to other embodiments, the described 3D culturing is performed for at least 4 doublings, at least 5 doublings, at least 6 doublings, at least 7 doublings, at least 8 doublings, at least 9 doublings, or at least 10 doublings. In certain embodiments, cells are passaged when the culture reaches about 70-90% confluence, typically after 3-5 days (e.g., 1-3 doublings).

In certain embodiments, 3D culturing is performed in a 3D bioreactor. In some embodiments, the 3D bioreactor comprises a container for holding medium and a 3D attachment (carrier) substrate disposed therein; and a control apparatus, for controlling pH, temperature, and oxygen levels, and optionally other parameters. Alternatively or in addition, the bioreactor contains ports for the inflow and outflow of fresh medium and gases.

Examples of bioreactors include, but are not limited to, continuous stirred tank bioreactors; and New Brunswick™ CelliGen® and BIOFLO® bioreactor systems, available from Eppendorf, Inc.

As provided herein, a 3D bioreactor is capable, in certain embodiments, of 3D expansion of ASC under controlled conditions (e.g. pH, temperature and oxygen levels) and with growth medium perfusion, which in some embodiments is constant perfusion and in other embodiments is adjusted in order to maintain target levels of glucose or other components. Non-limiting embodiments of target glucose concentrations are between 400-700 mg/liter, between 450-650 mg\liter, between 475-625 mg/liter, between 500-600 mg/liter, or between 525-575 mg/liter. Alternatively or in addition, the cell cultures can be directly monitored for concentrations of lactate, glutamine, glutamate and ammonium. The glucose consumption rate and the lactate formation rate of the adherent cells enable, in some embodiments, estimation of the cellular growth rate and determination of the optimal harvest time.

In some embodiments, for example where CM is being harvested, a continuous stirred tank bioreactor is used, where a culture medium is continuously fed into the bioreactor and a product is continuously drawn out, to maintain a time-constant steady state within the reactor. A stirred tank bioreactor with a fibrous bed basket is available for example from New Brunswick Scientific Co. (Edison, NJ). Additional bioreactors that may be used, in some embodiments, are stationary-bed bioreactors; and air-lift bioreactors, where air is typically fed into the bottom of a central draught tube flowing up while forming bubbles, and disengaging exhaust gas at the top of the column. Additional possibilities are cell-seeding perfusion bioreactors with polyactive foams [as described in Wendt, D. et al., Biotechnol Bioeng 84: 205-214, (2003)] and radial-flow perfusion bioreactors containing tubular poly-L-lactic acid (PLLA) porous scaffolds [as described in Kitagawa et al., Biotechnology and Bioengineering 93(5): 947-954 (2006). Other bioreactors which can be used are described in U.S. Pat. Nos. 6,277,151; 6,197,575; 6,139,578; 6,132,463; 5,902,741; and 5,629,186. A "stationary-bed bioreactor" refers to a bioreactor in which the cellular growth substrate is not ordinarily lifted from the bottom of the incubation vessel in the presence of growth medium. For example, the substrate may have sufficient density to prevent being lifted and/or it may be packed by mechanical pressure to present it from being lifted. The substrate may be either a single body or multiple bodies. Typically, the substrate remains substantially in place during the standard perfusion rate of the bioreactor. In certain embodiments, the substrate may be lifted at unusually fast perfusion rates, for example greater than 200 rpm.

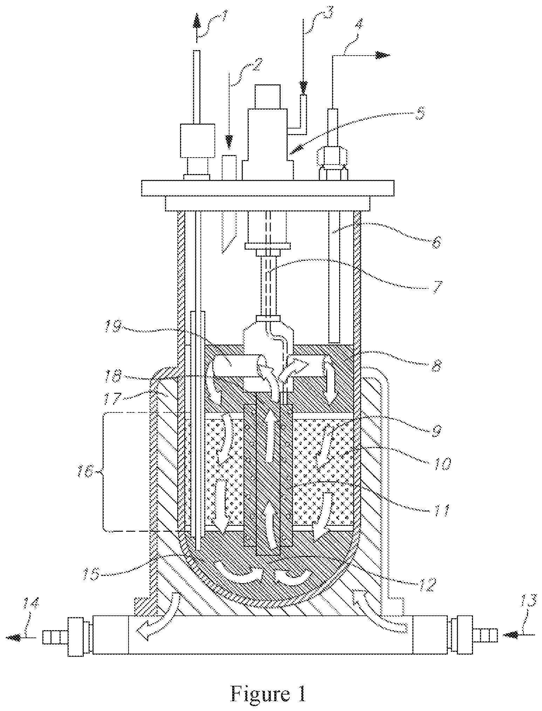

Another exemplary, non-limiting bioreactor, the Celligen 310 Bioreactor, is depicted in **Fig. 1****.** A fibrous-bed basket (16) is loaded with polyester disks (10). In some embodiments, the vessel is filled with deionized water or isotonic buffer via an external port (1 [this port may also be used, in other embodiments, for cell harvesting]) and then optionally autoclaved. In other embodiments, following sterilization, the liquid is replaced with growth medium, which saturates the disk bed as depicted in (9). In still further embodiments, temperature, pH, dissolved oxygen concentration, etc., are set prior to inoculation. In yet further embodiments, a slow stirring initial rate is used to promote cell attachment, then agitation is increased. Alternatively or addition, perfusion is initiated by adding fresh medium via an external port (2). If desired, metabolic products may be harvested from the cell-free medium above the basket (8). In some embodiments, rotation of the impeller creates negative pressure in the draft-tube (18), which pulls cell-free effluent from a reservoir (15) through the draft tube, then through an impeller port (19), thus causing medium to circulate (12) uniformly in a continuous loop. In still further embodiments, adjustment of a tube (6) controls the liquid level; an external opening (4) of this tube is used in some embodiments for harvesting. In other embodiments, a ring sparger (not visible), is located inside the impeller aeration chamber (11), for oxygenating the medium flowing through the impeller, via gases added from an external port (3), which may be kept inside a housing (5), and a sparger line (7). Alternatively or in addition, sparged gas confined to the remote chamber is absorbed by the nutrient medium, which washes over the immobilized cells. In still other embodiments, a water jacket (17) is present, with ports for moving the jacket water in (13) and out (14).

In certain embodiments, a perfused bioreactor is used, wherein the perfusion chamber contains carriers. The carriers may be, in more specific embodiments, selected from macrocarriers, microcarriers, or either. Microcarriers are well known to those skilled in the art, and are described, for example in US Patent Nos. 8,828,720, 7,531,334, 5,006,467. Microcarriers are also commercially available, for example as Cytodex™ (available from Pharmacia Fine Chemicals, Inc.), Superbeads (commercially available from Flow Labs, Inc.), and as DE-52 and DE-53 (commercially available from Whatman, Inc.). Other, non-limiting examples of microcarriers that are available commercially include alginate-based (GEM, Global Cell Solutions), dextran-based (Cytodex, GE Healthcare), collagen-based (Cultispher, Percell), and polystyrene-based (SoloHill Engineering) microcarriers.

In some embodiments, the carriers in the perfused bioreactor are packed, for example forming a packed bed, which is submerged in a nutrient medium. In certain embodiments, the microcarriers are packed inside a perfused bioreactor. Alternatively or in addition, the carriers may comprise an adherent material. In other embodiments, the surface of the carriers comprises an adherent material, or the surface of the carriers is adherent. In still other embodiments, the material exhibits a chemical structure such as charged surface exposed groups, which allows cell adhesion. Non-limiting examples of adherent materials which may be used in accordance with this aspect include a polyester, a polypropylene, a polyalkylene, a polyfluorochloroethylene, a polyvinyl chloride, a polystyrene, a polysulfone, a cellulose acetate, a glass fiber, a ceramic particle, a poly-L-lactic acid, and an inert metal fiber. In more particular embodiments, the material may be selected from a polyester and a polypropylene. In various embodiments, an "adherent material" refers to a material that is synthetic, or in other embodiments naturally occurring, or in other embodiments a combination thereof. In certain embodiments, the material is non-cytotoxic (or, in other embodiments, is biologically compatible). Non-limiting examples of synthetic adherent materials include polyesters, polypropylenes, polyalkylenes, polyfluorochloroethylenes, polyvinyl chlorides, polystyrenes, polysulfones, cellulose acetates, and poly-L-lactic acids, glass fibers, ceramic particles, and an inert metal fiber, or, in more specific embodiments, polyesters, polypropylenes, polyalkylenes, polyfluorochloroethylenes, polyvinyl chlorides, polystyrenes, polysulfones, cellulose acetates, and poly-L-lactic acids. Other embodiments include Matrigel™, an extracellular matrix component (e.g., Fibronectin, Chondronectin, Laminin), and a collagen.

Alternatively or in addition, the adherent material is fibrous, which may be, in more specific embodiments, a woven fibrous matrix, a non-woven fibrous matrix, or either. In still other embodiments, the material exhibits a chemical structure such as charged surface groups, which allows cell adhesion, e.g. polyesters, polypropylenes, polyalkylenes, polyfluorochloroethylenes, polyvinyl chlorides, polystyrenes, polysulfones, cellulose acetates, and poly-L-lactic acids. In more particular embodiments, the material may be selected from a polyester and a polypropylene.

Alternatively or in addition, the carriers comprise a fibrous material, optionally an adherent, fibrous material, which may be, in more specific embodiments, a woven fibrous matrix, a non-woven fibrous matrix, or either. Non-limiting examples of fibrous carriers are New Brunswick Scientific Fibracel® carriers, available commercially from of Eppendorf Inc, Enfield, CT, and made of polyester and polypropylene; and BioNOC II carriers, available commercially from CESCO BioProducts (Atlanta, GA) and made of PET (polyethylene terephthalate). In certain embodiments, the referred-to fibrous matrix comprises a polyester, a polypropylene, a polyalkylene, a polyfluorochloroethylene, a polyvinyl chloride, a polystyrene, or a polysulfone. In more particular embodiments, the fibrous matrix is selected from a polyester and a polypropylene.

In certain embodiments, the ASC may be incubated in a 2D apparatus, for example tissue culture plates or dishes, prior to incubation on carriers, for example packed carriers. In other embodiments, the ASC are not incubated in a 2D apparatus prior to incubation on carriers, for example packed carriers.

In other embodiments, cells are produced using a packed-bed spinner flask. In more specific embodiments, the packed bed may comprise a spinner flask and a magnetic stirrer. The spinner flask may be fitted, in some embodiments, with a packed bed apparatus similar to the Celligen™ Plug Flow bioreactor which is, in certain embodiments, packed with Fibra-cel® (or, in other embodiments, other carriers). The spinner is, in certain embodiments, batch fed (or in other alternative embodiments fed by perfusion), fitted with one or more sterilizing filters, and placed in a tissue culture incubator. In further embodiments, cells are seeded onto the scaffold by suspending them in medium and introducing the medium to the apparatus. In still further embodiments, the agitation speed is gradually increased, for example by starting at 40 RPM for 4 hours, then gradually increasing the speed to 120 RPM. In certain embodiments, the glucose level of the medium may be tested periodically (i.e. daily), and the perfusion speed adjusted maintain an acceptable glucose concentration, which is, in certain embodiments, between 400-700 mg/liter, between 450-650 mg/liter, between 475-625 mg/liter, between 500-600 mg/liter, or between 525-575 mg/liter. In yet other embodiments, at the end of the culture process, carriers are removed from the packed bed and optionally washed with isotonic buffer, and cells are processed or removed from the carriers by agitation and/or enzymatic digestion.

In certain embodiments, the 3D growth apparatus (in some embodiments the aforementioned bioreactor) contains a fibrous bed. In more specific embodiments, the fibrous bed may contain polyester, polypropylene, polyalkylene, poly fluoro-chloro-ethylene, polyvinyl chloride, polystyrene, polysulfone, or a polyamide (e.g. an aliphatic polyamide). In other embodiments, glass fibers or metal fibers (e.g. inert metal fibers) may be present; or a cellulose fiber (a non-limiting example of which is rayon) may be present.

In other embodiments, the apparatus or bioreactor contains a gel matrix. Typically, gels trap water, forming a gel phase. In other embodiments, the apparatus or bioreactor contains a hollow-fiber matrix, which is configured for the cells to grow and proliferate in the lumen of the fibers. In still other embodiments, the apparatus or bioreactor contains a packed-bed matrix, or a fluidized bed matrix, with spheres, beads, or carriers, which serve as a substrate for cell growth. The spheres or beads may be, in various embodiments, microporous, porous, or non-porous-in the latter case, the cells attach only to their surface. In yet other embodiments, the apparatus or bioreactor contains a matrix with a sponge-like configuration. In certain embodiments, the bioreactor is seeded at a concentration of between 10,000 - 2,000,000 cells / ml of medium, in other embodiments 20,000-2,000,000 cells / ml, in other embodiments 30,000-1,500,000 cells / ml, in other embodiments 40,000-1,400,000 cells / ml, in other embodiments 50,000-1,300,000 cells / ml, in other embodiments 60,000-1,200,000 cells / ml, in other embodiments 70,000-1,100,000 cells / ml, in other embodiments 80,000-1,000,000 cells / ml, in other embodiments 80,000-900,000 cells / ml, in other embodiments 80,000-800,000 cells / ml, in other embodiments 80,000-700,000 cells / ml, in other embodiments 80,000-600,000 cells / ml, in other embodiments 80,000-500,000 cells / ml, in other embodiments 80,000-400,000 cells / ml, in other embodiments 90,000-300,000 cells / ml, in other embodiments 90,000-250,000 cells / ml, in other embodiments 90,000-200,000 cells / ml, in other embodiments 100,000-200,000 cells / ml, in other embodiments 110,000-1,900,000 cells / ml, in other embodiments 120,000-1,800,000 cells / ml, in other embodiments 130,000-1,700,000 cells / ml, in other embodiments 140,000-1,600,000 cells / ml.

In still other embodiments, between 1-20 x 10⁶ cells per gram (gr) of carrier (substrate) are seeded, or in other embodiments 1.5-20 x 10⁶ cells / gr carrier, or in other embodiments 1.5-18 x 10⁶ cells / gr carrier, or in other embodiments 1.8-18 x 10⁶ cells / gr carrier, or in other embodiments 2-18 x 10⁶ cells / gr carrier, or in other embodiments 3-18 x 10⁶ cells / gr carrier, or in other embodiments 2.5-15 x 10⁶ cells / gr carrier, or in other embodiments 3-15 x 10⁶ cells / gr carrier, or in other embodiments 3-14 x 10⁶ cells / gr carrier, or in other embodiments 3-12 x 10⁶ cells / gr carrier, or in other embodiments 3.5-12 x 10⁶ cells / gr carrier, or in other embodiments 3-10 x 10⁶ cells / gr carrier, or in other embodiments 3-9 x 10⁶ cells / gr carrier, or in other embodiments 4-9 x 10⁶ cells / gr carrier, or in other embodiments 4-8 x 10⁶ cells / gr carrier, or in other embodiments 4-7 x 10⁶ cells / gr carrier, or in other embodiments 4.5-6.5 x 10⁶ cells / gr carrier.

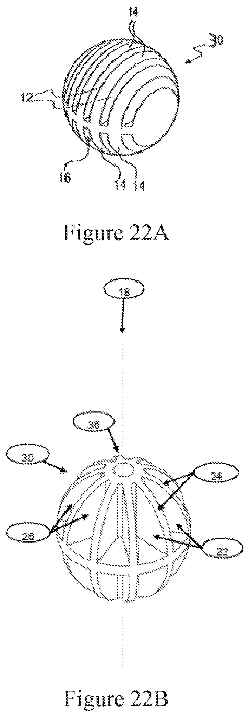

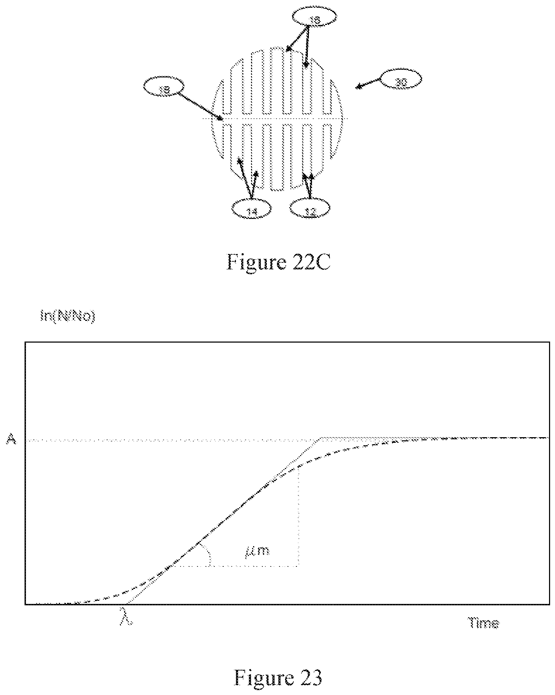

In some embodiments, with reference to **Figs. 22A-B**, and as described in WO/2014/037862, published on March 13, 2014, grooved carriers 30 are used for proliferation and/or incubation of ASC. In various embodiments, the carriers may be used following a 2D incubation (e.g. on culture plates or dishes), or without a prior 2D incubation. In other embodiments, incubation on the carriers may be followed by incubation on a 3D substrate in a bioreactor, which may be, for example, a packed-bed substrate or microcarriers; or incubation on the carriers may not be followed by incubation on a 3D substrate. Carriers 30 can include multiple two-dimensional (2D) surfaces 12 extending from an exterior of carrier 30 towards an interior of carrier 30. As shown, the surfaces are formed by a group of ribs 14 that are spaced apart to form openings 16, which may be sized to allow flow of cells and culture medium (not shown) during use. With reference to **Fig. 22C****,** carrier 30 can also include multiple 2D surfaces 12 extending from a central carrier axis 18 of carrier 30 and extending generally perpendicular to ribs 14 that are spaced apart to form openings 16, creating multiple 2D surfaces 12. In some embodiments, carriers 30 are "3D bodies" as described in WO/2014/037862.

In certain embodiments, the described carriers (e.g. grooved carriers) are used in a bioreactor. In some, the carriers are in a packed conformation.

In still other embodiments, the material forming the multiple 2D surfaces comprises at least one polymer. Suitable coatings may, in some embodiments, be selected to control cell attachment or parameters of cell biology.

In certain embodiments, the described method further comprises the subsequent step (following the described 3D incubation, which may be, in various embodiments, with or without added cytokines) of harvesting the ASC by removing the ASC from the 3D culture apparatus. In more particular embodiments, cells may be removed from a 3D matrix while the matrix remains within the bioreactor. In certain embodiments, at least about 10%, at least 12%, at least 14%, at least 16%, at least 18%, at least 20%, at least 22%, at least 24%, at least 26%, at least 28%, or at least 30% of the cells are in the S and G2/M phases (collectively), at the time of harvest from the bioreactor. Cell cycle phases can be assayed by various methods known in the art, for example FACS detection. Typically, in the case of FACS, the percentage of cells in S and G2/M phase is expressed as the percentage of the live cells, after gating for live cells, for example using a forward scatter/side scatter gate. Those skilled in the art will appreciate that the percentage of cells in these phases correlates with the percentage of proliferating cells. In some cases, allowing the cells to remain in the bioreactor significantly past their logarithmic growth phase causes a reduction in the number of cells that are proliferating.

In certain embodiments, the ASC used as an anti-cancer agent have been previously co-incubated with cancer cells, or, in other embodiments, with one or more cancer cell lines incubated in conditioned medium ("CM") derived from cancer cells or cancer cell lines, or have been incubated in medium containing a fraction of a CM derived from cancer cells or cancer cell lines. In other embodiments, the ASC used to produce CM for use as an anti-cancer agent have been co-incubated with cancer cells, or, in other embodiments, with 1 or more cancer cell lines. In some embodiments, the co-incubation is performed under conditions where the ASC and cancer cells or cell lines contact one another. Such conditions include seeding the ASC and cancer cells or cell lines in the same apparatus, in various embodiments either together, first seeding the ASC, or first seeding the cancer cells or cell lines. The co-incubation takes place, in some embodiments, in a tissue culture apparatus, or in other embodiments, in a bioreactor, which may in some embodiments comprise a 3D growth substrate.

In other embodiments, the conditions are such that the ASC and cancer cells or cell lines do not contact one another, but medium and soluble components thereof are exchanged between the two cell populations. Those skilled in the art will appreciate that various means are available to prevent contact between two cell populations while permitting exchange of medium, for example by separating the cell populations with a membrane that is permeable to fluids and factors dissolved therein, or a semi-permeable membrane that allows soluble factors smaller than a defined size to diffuse through it.

In other embodiments, the ASC used as an anti-cancer agent have been previously incubated in CM derived from cancer cells or cancer cell lines. In other embodiments, the ASC used to produce CM for use as an anti-cancer agent have been incubated in CM derived from cancer cells or cancer cell lines. In first stage of the process, in some embodiments, cancer cells are cultured, and the medium resulting from the incubation (the "cancer cell CM") is isolated. In the second stage, in various embodiments, ASC are incubated with the cancer cell CM, in a tissue culture apparatus, including but not limited to culture wells, or in other embodiments in a bioreactor, which may in some embodiments comprise a 3D growth substrate. In still other embodiments, the ASC have been exposed to inflammatory cytokines, prior to their incubation in the cancer cell CM. In other embodiments, or one or more cytokines, vitamins, or biologically active proteins are added to the cancer cell CM. In still other embodiments, the incubation of the ASC is performed under non-standard conditions, for example hypoxia or altered pH or atmospheric pressure. In still other embodiments, the CM resulting from the incubation of the ASC (the "ASC CM"), or in other embodiments the ASC themselves, is used as an anti-cancer agent. In yet other embodiments, the ASC are placental ASC that are predominantly maternal cells, or are fetal cells, or are a mixture of fetal and maternal cells.

In other embodiments, the ASC used as an anti-cancer agent have been incubated in medium containing a fraction of a CM derived from cancer cells or cancer cell lines. In other embodiments, the ASC used to produce CM for use as an anti-cancer agent have been incubated in medium containing a fraction of a CM derived from cancer cells or cancer cell lines. In the first stage, in some embodiments cancer cells are cultured, and a fraction the cancer cell CM is isolated and added to a standard culture medium. In certain embodiments, the process comprises a second stage, in which ASC are incubated with the medium generated in the first stage, for example in a bioreactor, or in culture wells.

In certain embodiments, the conditions of the aforementioned incubation are such that the cancer cells or cell lines form spheroids, or in other embodiments form microspheroids, during the co-incubation.

In various embodiments, incubation of ASC with cancer cells or cancer cell lines, or with CM derived therefrom, is performed after 3D expansion of the ASC as described herein, in the absence of cancer cells, cancer cell lines, or CM derived therefrom. In other embodiments, the entire process of 3D expansion of the ASC is performed in the presence of cancer cells, cancer cell lines, or CM derived therefrom. In still other embodiments, expansion of the ASC in the absence of cancer cells, cancer cell lines, or CM derived therefrom occurs after co-incubation of the ASC with cancer cells, cancer cell lines, or CM derived therefrom. In certain embodiments, the 2D expansion of the ASC is performed before 3D incubation of ASC without and/or with cancer cells, cancer cell lines, or CM derived therefrom.

In certain embodiments, the ASC have been exposed to inflammatory cytokines, for example while in the bioreactor used to expand them, prior to performing an additional incubation with cancer cells or cancer cell lines. In other embodiments, the ASC have been exposed to inflammatory cytokines, following (i) growing the ASC in a bioreactor, (ii) optionally harvesting them from the bioreactor, and (iii) performing an additional incubation of the ASC with cancer cells or cancer cell lines. In various embodiments, the additional incubation is performed in culture plates, optionally under non-standard conditions, for example hypoxia or altered pH or ambient pressure.

The aforementioned cancer cells that are incubated with or in proximity to ASC, or whose CM (or a fraction thereof) is incubated with ASC are, in some embodiments, those of the patient that will be treated; or in other embodiments are from the same cancer type as the tumor that will be treated; or in other embodiments are from a subpopulation of cancer cells that has undergone alterations during the course of cancer progression and/or treatment thereof. In other embodiments, the cancer cells are any other cancer cells; *e.g.* a type capable of inducing ASC to secrete therapeutic factors.

In still other embodiments, the expanded cells are harvested from the bioreactor by a process comprising vibration or agitation, for example as described in PCT International Application Publ. No. WO 2012/140519. In certain embodiments, during harvesting, the cells are agitated by oscillation at 0.7-6 Hertz, or in other embodiments 1-3 Hertz, during, or in other embodiments during and after, treatment with a protease, optionally also comprising a calcium chelator. In certain embodiments, the carriers containing the cells are agitated at 0.7-6 Hertz, or in other embodiments 1-3 Hertz, while submerged in a solution or medium comprising a protease, optionally also comprising a calcium chelator. Non-limiting examples of a protease plus a calcium chelator are trypsin, or another enzyme with similar activity, optionally in combination with another enzyme, non-limiting examples of which are Collagenase Types I, II, III, and IV, with EDTA. Enzymes with similar activity to trypsin are well known in the art; non-limiting examples are TrypLE™, a fungal trypsin-like protease, and Collagenase, Types I, II, III, and IV, which are available commercially from Thermo Fisher Scientific (Waltham MA). Enzymes with similar activity to collagenase are well known in the art; non-limiting examples are Dispase I and Dispase II, which are available commercially from Sigma-Aldrich.

In other embodiments, one or more cytokines, vitamins, or biologically active proteins are added to the medium used for the co-incubation.

In other embodiments, the co-incubation is performed under non-standard conditions, for example hypoxia or altered pH or atmospheric pressure.

In certain embodiments, the cell lines used in the co-incubation need not be the same type of cancer cell that is the therapeutic target of the obtained ASC or CM. In other embodiments, the cell lines used in the co-incubation are the same type of cancer cell that is the therapeutic target of the obtained ASC or CM

In various embodiments, the ASC used in each of the described co-incubation methods may utilize placental ASC that are predominantly maternal cells, or are predominantly fetal cells, or are a mixture of fetal cells and maternal cells. Each of these embodiments may be freely combined with the described embodiments of co-incubation of ASC with cancer cells or cancer cell lines.

### Treatment of cells with pro-inflammatory cytokines

In certain embodiments of the described methods and compositions, the composition of the medium is not varied during the course of the culturing process used to expand the cells. In other words, no attempt is made to intentionally vary the medium composition by adding or removing factors or adding fresh medium with a different composition than the previous medium. Reference to varying the composition of the medium does not include variations in medium composition that automatically occur as a result of prolonged culturing, for example due to the absorption of nutrients and the secretion of metabolites by the cells therein, as will be appreciated by those skilled in the art.

In other embodiments, the 3D culturing method used to prepare the cells comprises the sub-steps of: (a) incubating ASC in a 3D culture apparatus in a first growth medium, wherein no inflammatory cytokines have been added to the first growth medium; and (b) subsequently incubating the ASC in a 3D culture apparatus in a second growth medium, wherein one or more pro-inflammatory cytokines have been added to the second growth medium. Those skilled in the art will appreciate, in light of the present disclosure, that the same 3D culture apparatus may be used for the incubations in the first and second growth medium by simply adding cytokines to the medium in the culture apparatus, or, in other embodiments, by removing the medium from the culture apparatus and replacing it with medium that contains cytokines. In other embodiments, a different 3D culture apparatus may be used for the incubation in the presence of cytokines, for example by moving (e.g. passaging) the cells to a different incubator, before adding the cytokine-containing medium. Those skilled in the art will appreciate, in light of the present disclosure, that the ASC to be used in the described methods may be extracted, in various embodiments, from the placenta, from adipose tissue, or from other sources, as described further herein.

Reference herein to one or more "pro-inflammatory" cytokines, or "inflammatory cytokines", which are used interchangeably, implies the presence of at least one cytokine that mediates an inflammatory response in a mammalian host, for example a human host. A non-limiting list of cytokines are Interferon-gamma (IFN-gamma; UniProt identifier P01579), IL-22 (UniProt identifier Q9GZX6), Tumor Necrosis Factor-alpha (TNF-alpha; UniProt identifier P01375), IFN-alpha (IFN-α), IFN-beta (UniProt identifier P01574), IL-1alpha (UniProt identifier P01583), IL-1beta (UniProt identifier P01584), IL-17 (UniProt identifier Q5QEX9), IL-23 (UniProt identifier Q9NPF7), IL-17A (UniProt identifier Q16552), IL-17F (UniProt identifier Q96PD4), IL-21 (UniProt identifier Q9HBE4), IL-13 (UniProt identifier P35225), IL-5 (UniProt identifier P05113), IL-4 (UniProt identifier P05112), IL-33 (UniProt identifier O95760), IL-1RL1 (UniProt identifier Q01638), TNF-Beta (UniProt identifier P01374), IL-11 (UniProt identifier P20809), IL-9 (UniProt identifier P15248), IL-2 (UniProt identifier P60568), IL-21 (UniProt identifier Q9HBE4), Tumor Necrosis Factor-Like Ligand (TL1A; a.k.a. TNF ligand superfamily member 15; UniProt identifier 095150), IL-12 (UniProt identifiers P29459 and P29460 for the alpha- and beta subunits, respectively), and IL-18 (UniProt identifier Q14116). Additional cytokines include (but are not limited to): Leukemia inhibitory factor (LIF; UniProt identifier P15018), oncostatin M (OSM; UniProt identifier P13725), ciliary neurotrophic factor (CNTF (UniProt identifier P26441), and IL-8 (UniProt identifier P10145). All Swissprot and UniProt entries in this paragraph were accessed on July 24, 2014. Various embodiments of incubation of ASC with cytokines, including particular cytokines, combinations and amounts thereof, and particular method steps, are described in PCT/IB2016/053310 in the name of Eytan Abraham *et al.*

Except where indicated otherwise, reference to a cytokine or other protein is intended to include all isoforms of the protein. For example, IFN-α includes all the subtypes and isoforms thereof, such as but not limited to IFN-α17, IFN-α4, IFN-α7, IFN-α8, and IFN-α110. Representative, non-limiting UniProt identifiers for IFN-α are P01571, P05014, P01567, P32881 and P01566. Those skilled in the art will appreciate that even in the case of human cells, the aforementioned cytokines need not be human cytokines, since many non-human (*e.g.* animal) cytokines are active on human cells. Similarly, the use of modified cytokines that have similar activity to the native forms falls within the scope of the described methods and compositions.

In certain embodiments, the cytokine present in the described medium, or in other embodiments at least one of the cytokines present, if more than one is present, is an inflammatory cytokine that affects innate immune responses. In further embodiments, the cytokine is one of, or in other embodiments more than one, of TNF-α, IL-1-α, IL-12, IFN-α, IFN-beta, or IFN-gamma.

In other embodiments, the cytokine, or in other embodiments at least one of the cytokines, if more than one is present, is an inflammatory cytokine that affects adaptive immune responses. In further embodiments, the cytokine is one of, or in other embodiments more than one, of IL-2, IL-4, IL-5, TGF-β, or IFN-γ.

In still other embodiments, the cytokine, or in other embodiments at least one of the cytokines, if more than one is present, is a Th1 cytokine. In further embodiments, the cytokine is one of, or in other embodiments more than one, of IFN-γ, IL-22, TNF-α, IL-α, or IL-1β.

In still other embodiments, the cytokine, or in other embodiments at least one of the cytokines, if more than one is present, is a Thl7 cytokine. In further embodiments, the cytokine is one of, or in other embodiments more than one, of IL-17, IL-23, IL-17A, IL-17F, IL-21, IL-22, TNF-α, or granulocyte macrophage colony stimulating factor (GM-CSF; UniProt identifier P04141).

In yet other embodiments, the cytokine, or in other embodiments at least one of the cytokines, if more than one is present, is selected from a Th1 cytokine and a Th17 cytokine.

In still other embodiments, the cytokine, or in other embodiments at least one of the cytokines, if more than one is present, is a Th2 cytokine. In further embodiments, the cytokine is one of, or in other embodiments more than one, of IL-13, IL-5, IL-4, IL-33, IL-1RL1, TNF-α, and TNF-β. In other embodiments, the cytokine is one of, or in other embodiments more than one, of IL-13, IL-5, IL-33, IL-1RL1, TNF-Alpha, or TNF-Beta.

In yet other embodiments, the cytokine(s) is one of, or in other embodiments more than one, of IL-11, Leukemia inhibitory factor (LIF), oncostatin M (OSM), CNTF, GM-CSF, and IL-8. In further embodiments, the cytokine(s) is one or more of IL-11, LIF, OSM, CNTF, GM-CSF, or IL-8. In other embodiments, the cytokine(s) is one or more of IL-9, IL-2, and IL-21.

In other embodiments, the cytokine(s) is one of, or in other embodiments more than one, of: TNF-α, IL-1beta, or TL1A.

In yet other embodiments, the cytokine(s) is one of, or in other embodiments more than one, of IL-12, IL-18, TNF-α.

In more specific embodiments, one of the aforementioned cytokines is present in the medium in an amount of 0.1-10 ng/ml; 0.15-10 ng/ml; 0.2-10 ng/ml; 0.3-10 ng/ml; 0.4-10 ng/ml; 0.5-10 ng/ml; 0.7-10 ng/ml; 1-10 ng/ml; 1.5-10 ng/ml; 2-10 ng/ml; 3-10 ng/ml; 4-10 ng/ml; 5-10 ng/ml; 0.1-5 ng/ml; 0.2-5 ng/ml; 0.3-5 ng/ml; 0.4-5 ng/ml; 0.5-5 ng/ml; 0.7-5 ng/ml; 1-5 ng/ml; 2-5 ng/ml; 0.1-3 ng/ml; 0.2-3 ng/ml; 0.3-3 ng/ml; 0.4-3 ng/ml; 0.5-3 ng/ml; 0.6-3 ng/ml; 0.8-3 ng/ml; 1-3 ng/ml; 1.5-3 ng/ml; 0.1-2 ng/ml; 0.2-2 ng/ml; 0.3-2 ng/ml; 0.4-2 ng/ml; 0.5-2 ng/ml; 0.6-2 ng/ml; 0.8-2 ng/ml; 1-2 ng/ml; 0.5-1.5 ng/ml; 0.6-1.5 ng/ml; 0.6-1.4 ng/ml; 0.7-1.3 ng/ml; 0.8-1.2 ng/ml; 0.1-0.8 ng/ml; 0.1-0.6 ng/ml; 0.1-0.5 ng/ml; 0.1-0.4 ng/ml; 0.2-1 ng/ml; 0.2-0.8 ng/ml; 0.2-0.6 ng/ml; 0.2-0.5 ng/ml; 0.2-0.4 ng/ml; 1-100 ng/ml; 2-100 ng/ml; 3-100 ng/ml; 4-100 ng/ml; 5-100 ng/ml; 7-100 ng/ml; 10-100 ng/ml; 15-100 ng/ml; 20-100 ng/ml; 30-100 ng/ml; 40-100 ng/ml; 50-100 ng/ml; 1-50 ng/ml; 2-50 ng/ml; 3-50 ng/ml; 4-50 ng/ml; 5-50 ng/ml; 7-50 ng/ml; 10-50 ng/ml; 20-50 ng/ml; 1-30 ng/ml; 2-30 ng/ml; 3-30 ng/ml; 4-30 ng/ml; 5-30 ng/ml; 6-30 ng/ml; 8-30 ng/ml; 10-30 ng/ml; 15-30 ng/ml; 1-20 ng/ml; 2-20 ng/ml; 3-20 ng/ml; 4-20 ng/ml; 5-20 ng/ml; 6-20 ng/ml; 8-20 ng/ml; 10-20 ng/ml; 5-15 ng/ml; 6-15 ng/ml; 6-14 ng/ml; 7-13 ng/ml; 8-12 ng/ml; 9-11 ng/ml; 9.5-10.5 ng/ml; 1-10 ng/ml; 1-8 ng/ml; 1-6 ng/ml; 1-5 ng/ml; 1-4 ng/ml; 2-10 ng/ml; 2-8 ng/ml; 2-6 ng/ml; 2-5 ng/ml; 2-4 ng/ml; 10-1000 ng/ml; 20-1000 ng/ml; 30-1000 ng/ml; 40-1000 ng/ml; 50-1000 ng/ml; 70-1000 ng/ml; 100-1000 ng/ml; 150-1000 ng/ml; 200-1000 ng/ml; 300-1000 ng/ml; 400-1000 ng/ml; 500-1000 ng/ml; 10-500 ng/ml; 20-500 ng/ml; 30-500 ng/ml; 40-500 ng/ml; 50-500 ng/ml; 70-500 ng/ml; 100-500 ng/ml; 200-500 ng/ml; 10-300 ng/ml; 20-300 ng/ml; 30-300 ng/ml; 40-300 ng/ml; 50-300 ng/ml; 60-300 ng/ml; 80-300 ng/ml; 100-300 ng/ml; 150-300 ng/ml; 10-200 ng/ml; 20-200 ng/ml; 30-200 ng/ml; 40-200 ng/ml; 50-200 ng/ml; 60-200 ng/ml; 80-200 ng/ml; 100-200 ng/ml; 50-150 ng/ml; 60-15 ng/ml; 60-14 ng/ml; 70-130 ng/ml; 80-120 ng/ml; 10-100 ng/ml; 10-80 ng/ml; 10-60 ng/ml; 10-50 ng/ml; 10-40 ng/ml; 20-100 ng/ml; 20-80 ng/ml; 20-60 ng/ml; 20-50 ng/ml; or 20-40 ng/ml. In still other embodiments, when more than one cytokines is present, each of them is present in an amount independently selected from the above amounts, which may be freely combined. In various other embodiments, the amounts of each of the proinflammatory cytokines present are each within one of the above ranges.

In certain embodiments, one or more of the cytokines is TNF-alpha (TNF-α). In more specific embodiments, TNF-α is present in one of the aforementioned amounts or ranges. In more specific embodiments, the TNF-α may be the only cytokine present, or, in other embodiments, may be present together with additional inflammatory cytokines, which may be, in certain embodiments, one of the aforementioned cytokines. In some embodiments, TNF-α is present in the medium together with IFN-gamma (IFN-γ). These two cytokines may be the only added cytokines, or, in other embodiments, present with additional proinflammatory cytokines.

As mentioned, in some embodiments, TNF-α is present together with one, or in other embodiments 2, 3, 4, 5, or more than 5, of the aforementioned cytokines. In still other embodiments, TNF-α and one, or in other embodiments more than one, of the additional cytokines is each present in an amount independently selected from one of the aforementioned amounts or ranges. Each combination may be considered as a separate embodiment.

In certain embodiments, one or more of the cytokines is IFN-γ. In more specific embodiments, the IFN-γ may be the only cytokine present, or, in other embodiments, may be present together with 1, 2, 3, 4, 5, 6, 1-2, 1-3, 1-4, 1-5, or 1-6, or more than 6 other cytokines. In more specific embodiments, IFN-γ is present in 1 of the aforementioned amounts or ranges.

As mentioned, in some embodiments, IFN-γ is present together with one of the aforementioned cytokines. These two cytokines may be the only 2 added cytokines, or, in other embodiments, present with additional proinflammatory cytokines. In still other embodiments, IFN-gamma and one, or in other embodiments more than one, of the additional cytokines is each present in an amount independently selected from one of the aforementioned amounts or ranges. Each combination may be considered as a separate embodiment.

In other embodiments, the aforementioned step (a) (3D incubation in the absence of added inflammatory cytokine[s]) is performed for at least 3 days, at least 4 days, at least 5 days, at least 6 days, or at least 7 days. In other embodiments, step (a) is performed for between 3-4 days, 3-5 days, 3-6 days, 3-7 days, 4-5 days, 4-6 days, 4-7 days, 5-6 days, 5-7 days, or 6-7 days. In still other embodiments, step (a) is performed for at least 1 population doubling, at least 2 doublings, at least 3 doublings, at least 4 doublings, 1-2 doublings, 1-3 doublings, 1-4 doublings, 2-3 doublings, 2-4 doublings, or 3-4 doublings.

Alternatively or in addition, the aforementioned step (b) (3D incubation in the presence of added inflammatory cytokine[s]) is performed for a time between 6-48 hours, 8-48 hours, 10-48 hours, 12-48 hours, 14-48 hours, 16-48 hours, 20-48 hours, 6-36 hours, 8-36 hours, 10-36 hours, 12-36 hours, 14-36 hours, 16-36 hours, 20-36 hours, 24-36 hours, 28-36 hours, 6-24 hours, 8-24 hours, 10-24 hours, 12-24 hours, 14-24 hours, 16-24 hours, 20-24 hours, 8-18 hours, 10-18 hours, 12-18 hours, or 14-18 hours.

In certain embodiments, at least part of the aforementioned step (a) is performed in perfusion mode. In other embodiments, the majority of step (a) (the majority of the 3D culturing time in the absence of added inflammatory cytokines) is performed in perfusion mode. In still other embodiments, all of step (a) is performed in perfusion mode. In other embodiments, at least part of step (a) is performed in batch mode.

Alternatively or in addition, at least part of step (b) is performed in batch mode. In other embodiments, the majority of step (b) (the majority of the 3D culturing time in the presence of added inflammatory cytokine[s]) is performed in batch mode. In still other embodiments, all of step (b) is performed in batch mode. In other embodiments, at least part of step (b) is performed in perfusion mode. In certain embodiments, the majority of step (a) is performed in perfusion mode, and the majority of step (b) is performed in batch mode.

In certain embodiments, the bioreactor is connected to an external medium reservoir (*e.g.* that is used to perfuse the bioreactor) containing the desired concentration of cytokines. Alternatively or in addition, the medium in the bioreactor is spiked with one or more cytokines at the beginning of the cytokine incubation, in order to rapidly bring the cytokine concentration in the bioreactor to the desired concentration. As provided herein, spiking of the bioreactor medium enabled a reduced incubation time in the presence of cytokines, resulting in enhanced cell viability. In other embodiments, step (b) comprises the sub-steps of (i) adding a bolus of the pro-inflammatory cytokine(s) to a medium in the bioreactor, thereby generating a growth medium containing inflammatory cytokines; and (ii) operably connecting the growth medium in the bioreactor with an external reservoir comprising an additional amount of growth medium containing inflammatory cytokines.

In still other embodiments, which may be, in some embodiments, combined with the previous embodiments of incubation length and spiking, step (b) is begun when the culture is in exponential growth phase. In more specific embodiments, step (b) is begun when the culture is in the latter half of exponential growth phase. In some embodiments, the culture is still in exponential growth phase at the conclusion of step (b). In other embodiments, the culture is in late exponential growth phase at the conclusion of step (b). In some embodiments, the cells are in a bioreactor, which is, in more specific embodiments, a packed-bed bioreactor. As provided herein, cytokine treatment of ASC in exponential phase produces cells with a protein expression profile.

The terms "exponential phase" and "exponential growth phase", except where indicated otherwise, refer to a time period in which the rate of cell division is at or near the maximal value for the particular system, where the rate of cell division is expressed as the logarithm of the relative population size (ln(N/N0, where N = the number of cells, and NO = the number of cells at the time of inoculation). In a more specific definition, the rate of cell division is at least 70% of the maximal cell division rate. The maximal cell division rate may be defined as the slope of a tangent line of a plot of the logarithm of the relative population size against time. A theoretical plot, provided for illustrative purposes only, is shown in **Fig. 23****.**

Those skilled in the art will appreciate that, when cells are seeded into a culture system (for example, a bioreactor), there is often a lag phase, during which cell division is relatively slow. The end of lag phase may be mathematically defined as the X-axis intercept of the aforementioned tangent line. The lag phase is followed by exponential phase. When environmental factors become limiting, the cell division rate begins to appreciably slow. For example, the cell division rate may slow to less than 60% of its maximal value. This phase is sometimes referred to as "late exponential phase" or "late exponential growth phase". In a more specific definition, the rate of cell division during late exponential growth phase is at between 30%-60% of the maximal cell division rate. Finally, the culture reaches stationary phase, where there is no appreciable net increase in cell number.

In certain embodiments, the ASC that are exposed to cytokines are placental-derived, adipose-derived, or BM-derived ASC. Alternatively or in addition, the ASC are mesenchymal-like ASC, which exhibit a marker pattern similar to "classical" MSC, but do not differentiate into osteocytes, under conditions where "classical" MSC would differentiate into osteocytes. In other embodiments, the cells exhibit a marker pattern similar to MSC, but do not differentiate into adipocytes, under conditions where MSC would differentiate into adipocytes. In still other embodiments, the cells exhibit a marker pattern similar to MSC, but do not differentiate into either osteocytes or adipocytes, under conditions where MSC would differentiate into osteocytes or adipocytes, respectively. The MSC used for comparison in these assays are, in one embodiment, MSC that have been harvested from BM and cultured under 2D conditions. In other embodiments, the MSC used for comparison have been harvested from BM and cultured under 2D conditions, followed by 3D conditions. In more particular embodiments, the mesenchymal-like ASC are maternal cells, or in other embodiments are fetal cells, or in other embodiments are a mixture of fetal cells and maternal cells.

In certain embodiments, the ASC, following their *ex vivo* exposure to cytokines, exhibit a marker pattern similar to "classical" MSC, but do not differentiate into osteocytes, under conditions where "classical" MSC would differentiate into osteocytes. In other embodiments, the cells exhibit a marker pattern similar to MSC, but do not differentiate into adipocytes, under conditions where MSC would differentiate into adipocytes. In still other embodiments, the cells exhibit a marker pattern similar to MSC, but do not differentiate into either osteocytes or adipocytes, under conditions where MSC would differentiate into osteocytes or adipocytes, respectively. The MSC used for comparison in these assays are, in one embodiment, MSC that have been harvested from BM and cultured under 2D conditions. In other embodiments, the MSC used for comparison have been harvested from BM and cultured under 2D conditions, followed by 3D conditions. In more particular embodiments, the mesenchymal-like ASC are maternal cells, or in other embodiments are fetal cells, or in other embodiments are a mixture of fetal cells and maternal cells.

### Optional additional preparation steps

In certain embodiments, further steps of purification or enrichment for ASC may be performed as part of the cell preparation process. Such methods include, but are not limited to, cell sorting using markers for ASC and/or, in various embodiments, mesenchymal stromal cells or mesenchymal-like ASC.

Cell sorting, in this context, refers to any procedure, whether manual, automated, etc., that selects cells on the basis of their expression of one or more markers, their lack of expression of one or more markers, or a combination thereof. Those skilled in the art will appreciate that data from one or more markers can be used individually or in combination in the sorting process.

### Buffers

Those skilled in the art will appreciate that a variety of isotonic buffers may be used for washing cells and similar uses. Hank's Balanced Salt Solution (HBSS; Life Technologies) is only one of many buffers that may be used.

Non-limiting examples of base media useful in 2D and 3D culturing include Minimum Essential Medium Eagle, ADC-1, LPM (Bovine Serum Albumin-free), F10(HAM), F12 (HAM), DCCM1, DCCM2, RPMI 1640, BGJ Medium (with and without Fitton-Jackson Modification), Basal Medium Eagle (BME-with the addition of Earle's salt base), Dulbecco's Modified Eagle Medium (DMEM-without serum), Yamane, IMEM-20, Glasgow Modification Eagle Medium (GMEM), Leibovitz L-15 Medium, McCoy's 5A Medium, Medium M199 (M199E-with Earle's sale base), Medium M199 (M199H-with Hank's salt base), Minimum Essential Medium Eagle (MEM-E-with Earle's salt base), Minimum Essential Medium Eagle (MEM-H-with Hank's salt base) and Minimum Essential Medium Eagle (MEM-NAA with non-essential AA), among numerous others, including medium 199, CMRL 1415, CMRL 1969, CMRL 1066, NCTC 135, MB 75261, MAB 8713, DM 145, Williams' G, Neuman & Tytell, Higuchi, MCDB 301, MCDB 202, MCDB 501, MCDB 401, MCDB 411, MDBC 153. In certain embodiments, DMEM is used. These and other useful media are available from GIBCO, Grand Island, N.Y., USA and Biological Industries, Bet HaEmek, Israel, among others.

In some embodiments, whether or not inflammatory cytokines are added, the medium may be supplemented with additional substances. Non-limiting examples of such substances are serum, which is, in some embodiments, fetal serum of cows or other species, which is, in some embodiments, 5-15% of the medium volume. In certain embodiments, the medium contains 1-5%, 2-5%, 3-5%, 1-10%, 2-10%, 3-10%, 4-15%, 5-14%, 6-14%, 6-13%, 7-13%, 8-12%, 8-13%, 9-12%, 9-11%, or 9.5%-10.5% serum, which may be fetal bovine serum, or in other embodiments another animal serum. In still other embodiments, the medium is serum-free.

Alternatively or in addition, the medium may be supplemented by growth factors, vitamins (e.g. ascorbic acid), salts (e.g. B-glycerophosphate), steroids (e.g. dexamethasone) and hormones, e.g., growth hormone, erythropoietin, thrombopoietin, interleukin 3, interleukin 7, macrophage colony stimulating factor, c-kit ligand/stem cell factor, osteoprotegerin ligand, insulin, insulin-like growth factor, epidermal growth factor, fibroblast growth factor, nerve growth factor, ciliary neurotrophic factor, platelet-derived growth factor, and bone morphogenetic protein.

It will be appreciated that additional components may be added to the culture medium. Such components may be antibiotics, antimycotics, albumin, amino acids, and other components known to the art for the culture of cells.

Those skilled in the art will appreciate that animal sera and other sources of growth factors are often included in growth media. In some cases, animal sera may contain inflammatory cytokines, which, in general, are not present in large amounts. Some preparations utilize a serum that is treated, for example, with charcoal, so as to remove most or all of the cytokines present. In any event, reference herein to "added cytokines", "medium containing cytokines", or the like, does not encompass the presence of cytokines present in animal sera that is customarily included in the medium.

It will also be appreciated that in certain embodiments, when the described ASC are intended for administration to a human subject, the cells and the culture medium (e.g., with the above described medium additives) are substantially xeno-free, i.e., devoid of any animal contaminants e.g., mycoplasma. For example, the culture medium can be supplemented with a serum-replacement, human serum and/or synthetic or recombinantly produced factors.

The various media described herein, i.e. (as applicable) the 2D growth medium, the first 3D growth medium, and/or the second 3D growth medium, may be independently selected from each of the described embodiments relating to medium composition. In certain embodiments, the only difference between the first and second 3D growth media is the presence of the added cytokines. In other embodiments, the first and second 3D growth media differ in other respects. In various embodiments, any medium suitable for growth of cells in a bioreactor may be used.

### Tissue sources and cell characteristics

In certain embodiments, the described ASC (e.g. prior to incubation with inflammatory cytokines, where applicable) are mesenchymal stromal cells (MSC). These cells may be isolated from many adult tissues, such as placenta, BM and adipose. In further embodiments, the cells are human MSC as defined by The Mesenchymal and Tissue Stem Cell Committee of the International Society for Cellular Therapy (Dominici et al, 2006ⁱ), based on 3 criteria: 1. Plastic-adherence when maintained in standard culture conditions (α minimal essential medium plus 20% fetal bovine serum (FBS)). 2. Expression of the surface molecules CD105, CD73 and CD90, and lack of expression of CD45, CD34, CD14 or CD11b, CD79α or CD19 and HLA-DR. 3. Differentiation into osteoblasts, adipocytes and chondroblasts *in vitro.*

Alternatively or in addition, the described ASC are mesenchymal-like ASC cells, which exhibit a marker pattern similar to "classical" MSC, but do not differentiate into osteocytes, under conditions where "classical" MSC would differentiate into osteocytes. In other embodiments, the cells exhibit a marker pattern similar to MSC, but do not differentiate into adipocytes, under conditions where MSC would differentiate into adipocytes. In still other embodiments, the cells exhibit a marker pattern similar to MSC, but do not differentiate into either osteocytes or adipocytes, under conditions where MSC would differentiate into osteocytes or adipocytes, respectively. The MSC used for comparison in these assays are, in one embodiment, MSC that have been harvested from BM and cultured under 2D conditions. In other embodiments, the MSC used for comparison have been harvested from BM and cultured under 2D conditions, followed by 3D conditions. In more particular embodiments, the mesenchymal-like ASC are maternal cells, or in other embodiments are fetal cells, or in other embodiments are a mixture of fetal cells and maternal cells.

### Placenta-derived stromal cells

Except where indicated otherwise herein, the terms "placenta", "placental tissue", and the like refer to any portion of the placenta. Placenta-derived ASC may be obtained, in various embodiments, from either fetal or, in other embodiments, maternal regions of the placenta, or in other embodiments, from both regions. More specific embodiments of maternal sources are the decidua basalis and the decidua parietalis. More specific embodiments of fetal sources are the amnion, the chorion, and the villi. In certain embodiments, tissue specimens are washed in a physiological buffer [e.g., phosphate-buffered saline (PBS) or Hank's buffer]. Single-cell suspensions can be made, in other embodiments, by treating the tissue with a digestive enzyme (see below) or/and physical disruption, a non-limiting example of which is mincing and flushing the tissue parts through a nylon filter or by gentle pipetting (Falcon, Becton, Dickinson, San Jose, CA) with washing medium. In some embodiments, the tissue treatment includes use of a DNAse, a non-limiting example of which is Benzonase from Merck. In other embodiments, placental cells may be obtained from a full-term or pre-term placenta.

In some embodiments, residual blood is removed from the placenta before cell harvest. This may be done by a variety of methods known to those skilled in the art, for example by perfusion. The term "perfuse" or "perfusion" as used herein refers to the act of pouring or passaging a fluid over or through an organ or tissue. In certain embodiments, the placental tissue may be from any mammal, while in other embodiments, the placental tissue is human.

A convenient source of placental tissue is a post-partum placenta (e.g., less than 10 hours after birth), however, a variety of sources of placental tissue or cells may be contemplated by the skilled person. In other embodiments, the placenta is used within 8 hours, within 6 hours, within 5 hours, within 4 hours, within 3 hours, within 2 hours, or within 1 hour of birth. In certain embodiments, the placenta is kept chilled prior to harvest of the cells. In other embodiments, prepartum placental tissue is used. Such tissue may be obtained, for example, from a chorionic villus sampling or by other methods known in the art. Once placental cells are obtained, they are, in certain embodiments, allowed to adhere to an adherent material (e.g., configured as a surface) to thereby isolate adherent cells. In some embodiments, the donor is 35 years old or younger, while in other embodiments, the donor may be any woman of childbearing age.

### Placental cell preparations enriched for fetal cells or maternal cells

In other embodiments, the described ASC are a placental preparation containing both maternal and fetal cells. In certain embodiments, the preparation is enriched for maternal cells. Under many standard culture conditions, maternal cells tend to dominate 2D and 3D cultures after several passages. In other embodiments, at least 60%, at least 65%, at least 70%, at least 75%, at least 80%, at least 85%, at least 90%, at least 92%, at least 95%, at least 96%, at least 97%, at least 98%, at least 99%, at least 99.5%, at least 99.7%, or at least 99.9% of the described cells are maternally-derived cells. Lack of expression of CD200, as measured by flow cytometry, using an isotype control to define negative expression, can be used as a marker of fetal cells.

Methods of preparing and characterizing maternal-derived and fetal-derived ASC are described in WO 2011/064669. In some embodiments, maternal and fetal placental ASC are identified based on genotype and/or karyotype (e.g., FISH) analysis. For example, ASC from a placenta of a male embryo can be separated into fetal and maternal cells based on karyotype analysis (i.e., XX cells are maternal while XY cells are fetal). In some embodiments, ASC derived from a fetal portion of the placenta (e.g., consisting of or comprising chorionic villi) express CD200. In other embodiments, not more than 3.5%, not more than 3%, not more than 2%, or not more than 1% of the ASC from a maternal placental cell preparation express CD200 as measured by flow cytometry using an isotype control to define negative expression.

In other embodiments, the preparation is enriched for fetal cells. In more specific embodiments, the mixture contains at least 70% fetal cells. In more specific embodiments, at least about 30%, 35%, 40%, 45%, 50%, 55%, 60%, 65%, 75%, 80%, 85%, 90%, 95%, 96%, 97%, 98%, 99% or 100% of the cells are fetal cells. Expression of CD200, as measured by flow cytometry, using an isotype control to define negative expression, can be used as a marker of fetal cells under some conditions. In yet other embodiments, at least 60%, at least 65%, at least 70%, at least 75%, at least 80%, at least 85%, at least 90%, at least 92%, at least 95%, at least 96%, at least 97%, at least 98%, at least 99%, at least 99.5%, at least 99.7%, or at least 99.9% of the described cells are fetal cells.

In still other embodiments, the preparation is a placental cell population that is a mixture of fetal and maternal cells. In more specific embodiments, the mixture contains 20-80% fetal cells; 30-80% fetal cells; 40-80% fetal cells; 50-80% fetal cells; 60-80% fetal cells; 20-90% fetal cells; 30-90% fetal cells; 40-90% fetal cells; 50-90% fetal cells; 60-90% fetal cells; 20-80% maternal cells; 30-80% maternal cells; 40-80% maternal cells; 50-80% maternal cells; 60-80% maternal cells; 20-90% maternal cells; 30-90% maternal cells; 40-90% maternal cells; 50-90% maternal cells; or 60-90% maternal cells.

### Adipose-derived stromal cells

As used herein the phrase "adipose tissue" refers to a connective tissue which comprises fat cells (adipocytes). Adipose tissue-derived ASC may be extracted, in various embodiments, by a variety of methods known to those skilled in the art, for example those described in U.S. Pat. No. 6,153,432. The adipose tissue may be derived, in other embodiments, from omental/visceral, mammary, gonadal, or other adipose tissue sites. In some embodiments, the adipose can be isolated by liposuction.

ASC may be derived from adipose tissue by treating the tissue with a digestive enzyme (non-limiting examples of which are collagenase, trypsin, dispase, hyaluronidase or DNAse); and ethylenediaminetetraacetic acid (EDTA). The cells may be, in some embodiments, subjected to physical disruption, for example using a nylon or cheesecloth mesh filter. In other embodiments, the cells are subjected to differential centrifugation directly in media or over a Ficoll™, Percoll™, or other particulate gradient (see U.S. Pat. No. 7,078,230).

### Stromal cells from other sources

ASC may be derived, for example, from placenta; adipose tissue; BM; peripheral blood; umbilical cord blood; synovial fluid; synovial membranes; spleen; thymus; mucosa (for example nasal mucosa); limbal stroma; ligaments, for example the periodontal ligament; scalp; hair follicles, testicles; embryonic yolk sac; and amniotic fluid, all of which are known to include ASC. In certain embodiments, the source of the ASC is a non-fetal source, for example maternal cells from the placenta or somatic tissue from a pediatric or adult donor, for example adipose tissue, BM, peripheral blood, umbilical cord blood, synovial fluid, synovial membranes, and ligaments such as the periodontal ligament. In some embodiments, the ASC are human ASC, while in other embodiments, they may be animal ASC. In particular embodiments, the ASC are derived from placental tissue.

### Identifying characteristics

As mentioned, in some embodiments, the described ASC do not differentiate into osteocytes, under conditions where "classical" mesenchymal stem cells would differentiate into osteocytes. In some embodiments, the conditions are incubation with a solution containing 0.1 micromolar (mcM) dexamethasone, 0.2 mM ascorbic acid, and 10 mM glycerol-2-phosphate, in plates coated with vitronectin and collagen, for 17 days. In still other embodiments, the conditions are incubation with a solution containing 10 mcM dexamethasone, 0.2 mM ascorbic acid, 10 mM glycerol-2-phosphate, and 10nM Vitamin D, in plates coated with vitronectin and collagen, for 26 days. The aforementioned solutions will typically contain cell culture medium such as DMEM + 10% serum or the like, as will be appreciated by those skilled in the art.

In other embodiments, the described ASC do not differentiate into adipocytes, under conditions where mesenchymal stem cells would differentiate into adipocytes. In some embodiments, the conditions are incubation of adipogenesis induction medium, namely a solution containing 1 mcM dexamethasone, 0.5 mM 3-Isobutyl-1-methylxanthine (IBMX), 10 mcg/ml insulin, and 100 mcM indomethacin, added on days 1, 3, 5, 9, 11, 13, 17, 19, and 21, while the medium is replaced with adipogenesis maintenance medium, namely a solution containing 10 mcg/ml insulin, on days 7 and 15, for a total of 25 days. In still other embodiments, a modified adipogenesis induction medium, containing 1 mcM dexamethasone, 0.5 mM IBMX, 10 mcg/ml insulin, and 200 mcM indomethacin, is used, and the incubation is for a total of 26 days. The aforementioned solutions will typically contain cell culture medium such as DMEM + 10% serum or the like, as will be appreciated by those skilled in the art.

In other embodiments, the described ASC exhibit a spindle shape when cultured under 2D conditions.

Alternatively or additionally, the ASC may express a marker or a collection of markers (e.g. surface marker) characteristic of MSC or mesenchymal-like stromal cells. Examples of surface markers include but are not limited to CD105 (UniProtKB Accession No. P17813), CD29 (UniProtKB Accession No. P05556), CD44 (UniProtKB Accession No. P16070), CD73 (UniProtKB Accession No. P21589), and CD90 (UniProtKB Accession No. P04216). Examples of markers expected to be absent from stromal cells are CD3 (UniProtKB Accession Nos. P09693 [gamma chain] P04234 [delta chain], P07766 [epsilon chain], and P20963 [zeta chain]), CD4 (UniProtKB Accession No. P01730), CD34 (UniProtKB Accession No. P28906), CD45 (UniProtKB Accession No. P08575), CD80 (UniProtKB Accession No. P33681), CD19 (UniProtKB Accession No. P15391), CD5 (UniProtKB Accession No. P06127), CD20 (UniProtKB Accession No. P11836), CD11B (UniProtKB Accession No. P11215), CD14 (UniProtKB Accession No. P08571), CD79-alpha (UniProtKB Accession No. B5QTD1), and HLA-DR (UniProtKB Accession Nos. P04233 [gamma chain], P01903 [alpha chain], and P01911 [beta chain]). All UniProtKB entries were accessed on July 7, 2014, except where indicated otherwise. Those skilled in the art will appreciate that the presence of complex antigens such as CD3 and HLA-DR may be detected by antibodies recognizing any of their component parts, such as, but not limited to, those described herein.

In certain embodiments, over 90% of the described ASC are positive for CD29, CD90, and CD54. In other embodiments, over 90% of the described ASC are positive for CD29, CD90, and CD54, and less than 1% of the described cells are positive for CD14, CD19, CD31, CD34, CD39, CD45, HLA-DR, and GlyA. In other embodiments, over 85% of the described cells are positive for CD29, CD73, CD90, and CD105; and over 65% of the described cells are positive for CD49. In yet other embodiments, less than 1% of the described cells are positive for CD14, CD19, CD31, CD34, CD39, CD45, HLA-DR, and GlyA; at least 30% of the cells are positive for CD200; less than 6% of the cells are positive for GlyA; and less than 20% of the cells are positive for SSEA4. In more specific embodiments, over 90% of the described cells are positive for CD29, CD90, and CD54; over 85% of the cells are positive for CD73 and CD105; and over 65% of the cells are positive for CD49. In still other embodiments, (a) over 90% of the described cells are positive for CD29, CD90, and CD54; (b) over 85% of the cells are positive for CD73 and CD105; (c) over 65% of the cells are positive for CD49; (d) less than 1% of the cells are positive for CD14, CD19, CD31, CD34, CD39, CD45, HLA-DR, GlyA; (e) at least 30% of the cells are positive for CD200; (f) less than 6% of the cells are positive for GlyA; (g) less than 50% of the cells are positive for CD56 (NCAM1; Uniprot Accession No. P13591 [accessed on February 12, 2017]); and/or (h) less than 20% of the cells are positive for SSEA4. Alternatively, more than 50% of the cells are positive for CD56. Each combination of the immediately aforementioned characteristics (a)-(h) represents a separate embodiment. In other embodiments, the described ASC that have been incubated with inflammatory cytokines exhibit the aforementioned marker expression characteristics. Various embodiments of ASC before, after, or without cytokine stimulation, including particular cell surface markers, differentiation capabilities and lack thereof, and combinations thereof, are described in PCT/IB2016/053310 in the name of Eytan Abraham *et al.*

PCT/IB2016/053310 relates to expression or secretion (as appropriate for each protein) by ASC of factors, e.g. c-kit ligand/ stem cell factor (SCF; Uniprot Accession no. P21583); Receptor-type tyrosine-protein kinase FLT3 (Flt-3; Uniprot Accession no. P36888); Aldehyde dehydrogenase X (ALDH X; Uniprot Accession no. P30837); Interleukin-6 (IL-6; UniProt No. P05231); eukaryotic translation elongation factor 2 (EEEF2); reticulocalbin 3; EF-hand calcium binding domain (RCN₂); calponin 1 basic smooth muscle (CNN1); Vascular Endothelial Growth Factor (VEGF); MCP-1, MCP2, and MCP-3 (Monocyte chemoattractant proteins 1, 2, and 3 / UniProt Nos. P13500, P80075, and P80098, respectively); GM-CSF; and/or RANTES (C-C motif chemokine 5; UniProt No. P13501). In certain embodiments, the ASC mentioned herein secrete elevated levels of factors such as SCF, Flt-3, ALDH X, IL-6, EEEF2, reticulocalbin 3, RCN₂, CNN1, VEGF, MCP-1, MCP2, MCP-3, GM-CSF, G-CSF (Granulocyte colony-stimulating factor; UniProt No. P09919), and/or HGF (Hepatocyte growth factor; UniProt No. P14210). In certain embodiments, the ASC secrete levels that are 2-fold, 3-fold, 4-fold, 5-fold, 6-fold, 8-fold, 10-fold, 15-fold, 20-fold, 30-fold, 40-fold, 50-fold, 60-fold, 80-fold, 100-fold, 150-fold, 200-fold, 300-fold, 500-fold, or 1000-fold, of 1, 2, 3, 4, 5, 6, 1 or more, 2 or more, 3 or more, 4 or more, 5 or more, or 6 or more of the aforementioned factors. Each of these factors, and each combination thereof, represents a separate embodiment. Each level of fold-increase represents a separate embodiment, and these embodiments may be freely combined with factors and combinations thereof. Each factor, combination thereof, and level of fold-increase may be freely combined with particular cell surface markers, differentiation capabilities and lack thereof, and combinations thereof.

In other embodiments, the cells do not differentiate into osteocytes, after incubation for 17 days with a solution containing 0.1 mcM dexamethasone, 0.2 mM ascorbic acid, and 10 mM glycerol-2-phosphate, in plates coated with vitronectin and collagen. In yet other embodiments, the cells exhibit lack of differentiation into osteocytes and also possess one or more of the aforementioned cell surface marker patters.

In other embodiments, the cells do not differentiate into adipocytes, after incubation in adipogenesis induction medium, namely a solution containing 1 mcM dexamethasone, 0.5 mM 3-Isobutyl-1-methylxanthine (IBMX), 10 mcg/ml insulin, and 100 mcM indomethacin, on days 1, 3, 5, 9, 11, 13, 17, 19, and 21; and replacement of the medium with adipogenesis maintenance medium, namely a solution containing 10 mcg/ml insulin, on days 7 and 15, for a total of 25 days. In yet other embodiments, the cells exhibit lack of differentiation into adipocytes and also possess one or more of the aforementioned cell surface marker patters.

In more specific embodiments, greater than 50%, in other embodiments greater than 55%, in other embodiments greater than 60%, in other embodiments greater than 65%, in other embodiments greater than 70%, in other embodiments greater than 75%, in other embodiments greater than 80%, in other embodiments greater than 85%, in other embodiments greater than 90%, in other embodiments greater than 95%, in other embodiments greater than 96%, in other embodiments greater than 97%, in other embodiments greater than 98%, in other embodiments greater than 99% of the ASC express a marker selected from CD73, CD90, CD29, and CD105, or in other embodiments 2 or more of these markers, or in other embodiments 3 or more of these markers, or in other embodiments all 4 of these markers in combination. In other embodiments, the ASC that have been incubated with inflammatory cytokines exhibit the aforementioned marker expression characteristics.

According to some embodiments, the ASC express CD200, or, in other embodiments, lack expression thereof. In still other embodiments, less than 30%, 25%, 20%, 15%, 10%, 8%, 6%, 5%, 4%, 3%, or 2%, 1%, or 0.5% of the ASC express CD200. In yet other embodiments, greater than 30%, 35%, 40%, 45%, 50%, 55%, 60%, 65%, 70%, 75%, 80%, 85%, 90%, 92%, 94%, 95%, 96%, 97%, 98%, 99%, or 99.5% of the ASC express CD200. In other embodiments, the ASC that have been incubated with inflammatory cytokines exhibit the aforementioned marker expression characteristics.

According to some embodiments, greater than 50%, in other embodiments greater than 55%, in other embodiments greater than 60%, in other embodiments greater than 65%, in other embodiments greater than 70%, in other embodiments greater than 75%, in other embodiments greater than 80%, in other embodiments greater than 85%, in other embodiments greater than 90%, in other embodiments greater than 95%, in other embodiments greater than 96%, in other embodiments greater than 97%, in other embodiments greater than 98%, in other embodiments greater than 99% of the ASC do not express a marker selected from CD3, CD4, CD45, CD80, HLA-DR, CD11b, CD14, CD19, CD34, and CD79-alpha, or in other embodiments do not express 2 or more of these markers, or in other embodiments 3 or more of these markers, or in other embodiments 4 or more of these markers, or in other embodiments 5 or more of these markers, or in other embodiments 6 or more of these markers, or in other embodiments 7 or more of these markers, or in other embodiments 8 or more of these markers, or in other embodiments 9 or more of these markers, or in other embodiments all ten of these markers. In other embodiments, the ASC that have been incubated with inflammatory cytokines exhibit the aforementioned marker expression characteristics.

In certain embodiments, the described cells have been transfected with one or more therapeutic factors, which may be, in certain embodiments, anti-tumor factors. In other embodiments, the cells need not have been transfected with any exogenous genetic material.

In still other embodiments, the ASC may be allogeneic, or in other embodiments, the cells may be autologous. In other embodiments, the cells may be fresh or, in other embodiments, frozen (e.g., cryo-preserved).

Also provided is use of conditioned media (CM) produced by the described methods, and, in other embodiments, pharmaceutical compositions comprising the described CM, for the described therapeutic indications. Those skilled in the art will appreciate that, in certain embodiments, various bioreactors may be used to prepare CM, including but not limited to plug-flow bioreactors, and stationary-bed bioreactors (Kompier R et al. Use of a stationary bed reactor and serum-free medium for the production of recombinant proteins in insect cells. Enzyme Microb Technol. 1991. 13(10):822-7). Pharmaceutical compositions comprising CM may be freely combined with any of the described embodiments for culture method steps, cell characteristics, or therapeutic parameters.

It is clarified that each embodiment of the described CM may be freely combined with each embodiment relating to a therapeutic method or pharmaceutical composition.

### Exosomes and uses thereof

Also provided herein is use of extracellular vesicles, e.g. exosomes, secreted by the described ASC, for the described therapeutic indications. Methods of isolating exosomes and other extracellular vesicles are well known in the art, and include, for example, immuno-magnetic isolation, for example as described in Clayton A et al, 2001; Mathias RA et al, 2009; and Crescitelli R et al, 2013.

In some embodiments, the extracellular vesicles are harvested from a 3D bioreactor in which the ASC have been incubated. In some embodiments, the culture in the 3D bioreactor includes inflammatory cytokines. In other embodiments, the 3D culture utilizes standard medium. Alternatively or in addition, the ASC are placenta-derived ASC, which may be, in more specific embodiments, a mixture of fetal and maternal cells, which may in further embodiments by enriched for fetal cells or for maternal cells.

Alternatively, the cells are cryopreserved following 3D culture, or in other embodiments following 2D culture, and then are thawed, after which the exosomes or other extracellular vesicles are isolated. In some embodiments, after thawing, the cells are cultured in 2D culture, from which the extracellular vesicles are harvested. In certain embodiments, the 2D culture is performed in the presence of inflammatory cytokines, which may be, in various embodiments, any of the cytokines mentioned herein. In other embodiments, the 2D culture utilizes standard medium. Alternatively or in addition, the ASC are placenta-derived ASC, which may be, in more specific embodiments, a mixture of fetal and maternal cells, which may in further embodiments by enriched in fetal cells or in maternal cells.

In other embodiments is provided a method of treating, preventing, or inhibiting growth of a cancer, a tumor, or a neoplasm, comprising the step of administering to the subject a pharmaceutical composition comprising the described exosomes. Also provided is a composition for treating, preventing, or inhibiting growth of a cancer, a tumor, or a neoplasm, comprising the described exosomes. Provided in addition is use of the described exosomes in the preparation of a medicament for treating, preventing, or inhibiting growth of a cancer, a tumor, or a neoplasm.

It is clarified that each embodiment of the described exosomes may be freely combined with each embodiment relating to a therapeutic method or pharmaceutical composition.

### Pharmaceutical compositions

The cells, CM derived therefrom, or exosomes derived therefrom, can be administered as a part of a pharmaceutical composition that further comprises one or more pharmaceutically acceptable carriers. Hereinafter, the term "pharmaceutically acceptable carrier" refers to a carrier or a diluent that does not cause significant irritation to a subject and does not abrogate the biological activity and properties of the administered cells. Examples, without limitations, of carriers are propylene glycol, saline, emulsions and mixtures of organic solvents with water. In some embodiments, the pharmaceutical carrier is an aqueous solution of saline. In other embodiments, the composition further comprises an excipient, e.g. a pharmacologically acceptable excipient. In certain embodiments, the composition is indicated for treatment of cancer, neoplasms, tumors, and/or malignancies; or in other embodiments, for suppression of metastasis of cancers and/or neoplasms.

In further embodiments, the excipient is an osmoprotectant or cryoprotectant, an agent that protects cells from the damaging effect of freezing and ice formation, which may in some embodiments be a permeating compound, non-limiting examples of which are dimethyl sulfoxide (DMSO), glycerol, ethylene glycol, formamide, propanediol, poly-ethylene glycol, acetamide, propylene glycol, and adonitol; or may in other embodiments be a non-permeating compound, non-limiting examples of which are lactose, raffinose, sucrose, trehalose, and d-mannitol. In other embodiments, both a permeating cryoprotectant and a non- permeating cryoprotectant are present. In other embodiments, the excipient is a carrier protein, a non-limiting example of which is albumin. In still other embodiments, both an osmoprotectant and a carrier protein are present; in certain embodiments, the osmoprotectant and carrier protein may be the same compound. Alternatively or in addition, the composition is frozen. The cells may be any embodiment of ASC mentioned herein, each of which is considered a separate embodiment.

Since non-autologous cells may in some cases induce an immune reaction when administered to a subject, several approaches may be utilized according to the methods provided herein to reduce the likelihood of rejection of non-autologous cells. In some embodiments, these approaches include either suppressing the recipient immune system or encapsulating the non-autologous cells in immune-isolating, semipermeable membranes before transplantation. In some embodiments, this may be done regardless of whether the ASC themselves engraft in the host. For example, the majority of the cells may, in various embodiments, not survive after engraftment for more than 3 days, more than 4 days, more than 5 days, more than 6 days, more than 7 days, more than 8 days, more than 9 days, more than 10 days, or more than 14 days.

Examples of immunosuppressive agents that may be used in the methods and compositions provided herein include, but are not limited to, methotrexate, cyclophosphamide, cyclosporine, cyclosporine A, chloroquine, hydroxychloroquine, sulfasalazine (sulphasalazopyrine), gold salts, D-penicillamine, leflunomide, azathioprine, anakinra, infliximab (REMICADE), etanercept, TNF-alpha blockers, biological agents that antagonize one or more inflammatory cytokines, and Non-Steroidal Anti-Inflammatory Drug (NSAIDs). Examples of NSAIDs include, but are not limited to acetyl salicylic acid, choline magnesium salicylate, diflunisal, magnesium salicylate, salsalate, sodium salicylate, diclofenac, etodolac, fenoprofen, flurbiprofen, indomethacin, ketoprofen, ketorolac, meclofenamate, naproxen, nabumetone, phenylbutazone, piroxicam, sulindac, tolmetin, acetaminophen, ibuprofen, Cox-2 inhibitors, and tramadol.

One may, in various embodiments, administer the pharmaceutical composition in a systemic manner. Alternatively, one may administer the pharmaceutical composition locally, for example, via injection of the pharmaceutical composition directly into a tissue region of a patient, such as, in non-limiting embodiments, intratumoral administration. In other embodiments, the cells are administered intramuscularly, intravenously (IV), subcutaneously (SC), by the intraosseous route (e.g. by intraosseous infusion), or intraperitoneally (IP), each of which is considered a separate embodiment. In still other embodiments, the pharmaceutical composition is administered intralymphatically, for example as described in United States Patent No. 8,679,834 in the name of Eleuterio Lombardo and Dirk Buscher.

In other embodiments, for injection, the described cells may be formulated in aqueous solutions, e.g. in physiologically compatible buffers such as Hank's solution, Ringer's solution, or physiological salt buffer, optionally in combination with medium containing cryopreservation agents.

Depending on the severity and responsiveness of the neoplasm to be treated, dosing can be a single or, in other embodiments, 2, 3, 4, at least 2, at least 3, at least 4, more than 4, or a plurality of administrations, with a course of treatment lasting from several days to several weeks or, in other embodiments, until alleviation of the disease state is achieved. In some embodiments, the interval between doses is between 1 hour and 10 days; in other words, the doses are spaced by a period not less than 1 hour and not more than 10 days. In other embodiments, the interval between doses is between 2 hours and 10 days; between 3 hours and 10 days; between 4 hours and 10 days; between 6 hours and 10 days; between 8 hours and 10 days; between 12 hours and 10 days; between 24 hours and 10 days; between 1-24 hours; between 2-24 hours; between 3-24 hours; between 4-24 hours; between 6-24 hours; between 8-24 hours; between 12-24 hours; between 1-5 days; between 1-10 days; between 1-15 days; between 1-20 days; between 2-5 days; between 2-10 days; between 2-15 days; between 2-20 days; between 2-30 days; between 3-10 days; between 3-15 days; between 3-20 days; between 3-30 days; or between 5-30 days.

In certain embodiments, following administration, the majority of the cells, in other embodiments more than 60%, more than 70%, more than 80%, more than 90%, more than 95%, more than 96%, more than 97%, more than 98%, or more than 99% of the cells are no longer detectable within the subject 1 month after administration.

In certain embodiments, compositions including the described preparations formulated in a compatible pharmaceutical carrier are prepared, placed in an appropriate container, and labeled for treatment of an indicated condition, for example an anti-cancer therapy. The container may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert.

The described ASC are, in other embodiments, suitably formulated as pharmaceutical compositions which can be suitably packaged as an article of manufacture. Such an article of manufacture comprises a packaging material which comprises a label for use in an anti-cancer therapy, or an anti-metastasis therapy, as described herein.

A typical dosage of the described ASC used alone ranges, in some embodiments, from about 10 million to about 500 million cells per administration, for a human subject. For example, the dosage can be, in some embodiments, 10, 20, 30, 40, 50, 60, 70, 80, 90, 100, 125, 150, 175, 200, 225, 250, 275, 300, 325, 350, 375, 400, 425, 450, 475, or 500 million cells or any amount in between these numbers. It is further understood that a range of ASC can be used including from about 10 to about 500 million cells, from about 100 to about 400 million cells, from about 150 to about 300 million cells. Accordingly, disclosed herein are therapeutic methods, the method comprising administering to a subject a therapeutically or prophylactically effective amount of ASC, wherein the dosage administered to the subject is 10, 20, 30, 40, 50, 60, 70, 80, 90, 100, 125, 150, 175, 200, 225, 250, 275, 300, 325, 350, 375, 400, 425, 450, 475, or 500 million cells or, in other embodiments, between 150 million to 300 million cells. ASC, compositions comprising ASC, and/or medicaments manufactured using ASC can be administered, in various embodiments, in a series of 1, 2, 3, 4, 5, 6, 7, 8, 9, 10, 11, 12, 13, 14, 15, 1-10, 1-15, 1-20, 2-10, 2-15, 2-20, 3-20, 4-20, 5-20, 5-25, 5-30, 5-40, or 5-50 injections, or more.

In still other embodiments is provided use of a bioreactor, comprising the described ASC, in preparing a medicament described herein. In some embodiments, the bioreactor further comprises a synthetic material that is a 3D substrate; and/or a synthetic medium; and/or inflammatory cytokines. The cells may be any embodiment of ASC mentioned herein, each of which is considered a separate embodiment.

It is clarified that each embodiment of the described ASC may be freely combined with each embodiment relating to a therapeutic method or pharmaceutical composition.

In still other embodiments, the described CM is used in any of the described therapeutic methods. Each embodiment of conditioned medium may be freely combined with each embodiment relating to a therapeutic method or pharmaceutical composition.

In certain embodiments, the subject may be administered with additional therapeutic agents or cells as part of the described methods and compositions.

In certain embodiments, the additional therapeutic agent is a chemotherapy agent. In more specific embodiments, the chemotherapy agent may be selected from alkylating and alkylating-like agents such as nitrogen mustards (e.g., chlorambucil, chlormethine, cyclophosphamide, ifosfamide, and melphalan), nitrosoureas (e.g., carmustine, fotemustine, lomustine, and streptozocin), platinum agents (i.e., alkylating-like agents) (e.g., carboplatin, cisplatin, oxaliplatin, BBR3464, and satraplatin), busulfan, dacarbazine, procarbazine, temozolomide, thioTEPA, treosulfan, and uramustine; antimetabolites such as folic acids (e.g., aminopterin, methotrexate, pemetrexed, and raltitrexed); purines such as cladribine, clofarabine, fludarabine, mercaptopurine, pentostatin, and thioguanine; pyrimidines such as capecitabine, cytarabine, fluorouracil, floxuridine, and gemcitabine; spindle poisons/mitotic inhibitors such as taxanes (e.g., docetaxel, paclitaxel, cabazitaxel) and vincas (e.g., vinblastine, vincristine, vindesine, and vinorelbine); cytotoxic/antitumor antibiotics such anthracyclines (e.g., daunorubicin, doxorubicin, epirubicin, idarubicin, mitoxantrone, pixantrone, and valrubicin), compounds naturally produced by various species of streptomyces (e.g., actinomycin, bleomycin, mitomycin, plicamycin) and hydroxyurea; topoisomerase inhibitors such as camptotheca (e.g., camptothecin, topotecan and irinotecan) and podophyllums (e.g., etoposide, teniposide); monoclonal antibodies for cancer immunotherapy such as anti-receptor tyrosine kinases (e.g., cetuximab, panitumumab, trastuzumab), anti-CD20 (e.g., rituximab and tositumomab), and others for example alemtuzumab, bevacizumab, and gemtuzumab; photosensitizers such as aminolevulinic acid, methyl aminolevulinate, porfimer sodium, and verteporfin; tyrosine kinase inhibitors such as cediranib, dasatinib, erlotinib, gefitinib, imatinib, lapatinib, nilotinib, sorafenib, sunitinib, and vandetanib; serine/threonine kinase inhibitors, (e.g., inhibitors of AbI, c-Kit, insulin receptor family member(s), EGF receptor family member(s), Akt, mTOR [e.g., rapamycin or analogs thereof, direct inhibitors of mTORC1 and/or mTORC2], Raf kinase family, phosphatidyl inositol (PI) kinases such as PI3 kinase, PI kinase-like kinase family members, cyclin dependent kinase family members, and aurora kinase family), growth factor receptor antagonists, retinoids (e.g., alitretinoin and tretinoin), altretamine, amsacrine, anagrelide, arsenic trioxide, asparaginase (e.g., pegaspargase), bexarotene, bortezomib, denileukin diftitox, estramustine, ixabepilone, masoprocol, mitotane, and testolactone, Hsp90 inhibitors, proteasome inhibitors, HDAC inhibitors, angiogenesis inhibitors, e.g., anti-vascular endothelial growth factor agents such as bevacizumab or VEGF-Trap, matrix metalloproteinase inhibitors, and pro-apoptotic agents (e.g., apoptosis inducers). In other embodiments, the additional therapeutic agent has activity against triple-negative breast cancer. Non-limiting examples of such agents are anthracycline; paclitaxel; docetaxel; eribulin; ixabepilone; capecitabine; Tigatuzumab; 3-(phenylethynyl)-1H-pyrazolo[3,4-d]pyrimidin-4-amine derivatives (Zhang CH et al); Teriflunomide; carboplatin, CB(2) cannabinoid o-quinone compounds (Morales et al); alantolactone; cabazitaxel; and dutasteride.

### Subjects

In certain embodiments, the subject treated by the described methods and compositions is a human. In other embodiments, the subject may be an animal. Alternatively or in addition, the subject has a cancer, a neoplasm, and/or a tumor. In still other embodiments, the subject has a primary tumor that is at risk of metastasis. In more specific embodiments, the primary tumor may be operable, or in other embodiments inoperable.

Also disclosed herein are kits and articles of manufacture that are drawn to reagents that can be used in practicing the methods disclosed herein. The kits and articles of manufacture can include any reagent or combination of reagent discussed herein or that would be understood to be required or beneficial in the practice of the disclosed methods, including ASC. In another aspect, the kits and articles of manufacture may comprise a label, instructions, and packaging material, for example for treating a tumor, cancer, or neoplasm; or for suppress metastasis of same.

Additional objects, advantages, and novel features of the invention will become apparent to one ordinarily skilled in the art upon examination of the following examples, which are not intended to be limiting. Additionally, each of the various embodiments and aspects of the invention as delineated hereinabove and as claimed in the claims section below finds experimental support in the following examples.

### EXAMPLES

Reference is now made to the following examples, which together with the above descriptions illustrate certain embodiments in a non-limiting fashion.

### EXAMPLE 1: PRODUCTION AND CULTURING OF ADHERENT STROMAL CELLS

The manufacturing process for the cell product consisted of 2 stages:
Stage 1, the intermediate cell stock (ICS) production, contains the following steps:
   1. Extraction of ASCs from the placenta.
   2. 2-dimensional (2D) cell growth for up to 12 population doublings.
   3. Cell concentration, formulation, filling and cryopreservation.
Stage 2, the thawing of the ICS and further culture, contains the following steps:
   1. 2D cell growth of the thawed ICS for up to 8 additional doublings.
   2. 3-dimensional (3D) cell growth in bioreactor/s and harvest from bioreactor/s up to 10 additional doublings.
   3. Downstream processing: cell concentration, washing, formulation, filling and cryopreservation.

The procedure included periodic testing of the growth medium for sterility and contamination.

Further details are provided in Example 1 of WO/2016/151476 to Pluristem Ltd.

### EXAMPLE 2: OSTEOCYTE AND ADIPOSE DIFFERENTIATION ASSAYS

### METHODS

***Bone marrow adherent cells*** - BM adherent cells were obtained as described in WO 2016/098061 to Esther Lukasiewicz Hagai and Rachel Ofir. Osteogenesis and adipogenesis assays were performed as described in WO 2016/098061.

### RESULTS

***Osteocyte induction.*** Incubation of BM-derived adherent cells in osteogenic induction medium resulted in differentiation of over 50% of the BM cells, as demonstrated by positive alizarin red staining. On the contrary, none of the placental-derived cells exhibited signs of osteogenic differentiation.

Next, a modified osteogenic medium comprising Vitamin D and higher concentrations of dexamethasone was used. Over 50% of the BM cells underwent differentiation into osteocytes, while none of the placental-derived cells exhibited signs of osteogenic differentiation.

***Adipocyte induction.*** Adipocyte differentiation of placenta- or BM-derived adherent cells in adipocyte induction medium resulted in differentiation of over 50% of the BM-derived cells, as demonstrated by positive oil red staining and by typical morphological changes (e.g. accumulation of oil droplets in the cytoplasm). In contrast, none of the placental-derived cells differentiated into adipocytes.

Next, a modified medium containing a higher indomethacin concentration was used. Over 50% of the BM-derived cells underwent differentiation into adipocytes. In contrast, none of the placental-derived cells exhibited morphological changes typical of adipocytes.

### EXAMPLE 3: MARKER EXPRESSION ONADHERENT STROMAL CELLS

### Methods (Examples 3-4)

***FACS analysis of membrane markers*** was performed as described in WO 2016/098061.

### Results

***Expression of cellular markers on isolated cells* -** the surface antigens expressed by the isolated cells were examined using monoclonal antibodies. The cells expressed CD73, CD29, and CD105, and did not express the markers CD34, CD45, CD19, CD14, and HLA-DR. More specifically, all the positive markers were expressed by more than 90% of the cells, and all the negative markers were expressed by less than 3% of the cells.

Furthermore, the cells did not express endothelial markers as shown by negative staining for the two endothelial markers CD31 and KDR. However, expression of a fibroblast-typical marker, D7-fib, was evident.

### EXAMPLE 4: HYPO-IMMUNOGENICITY OF ASC

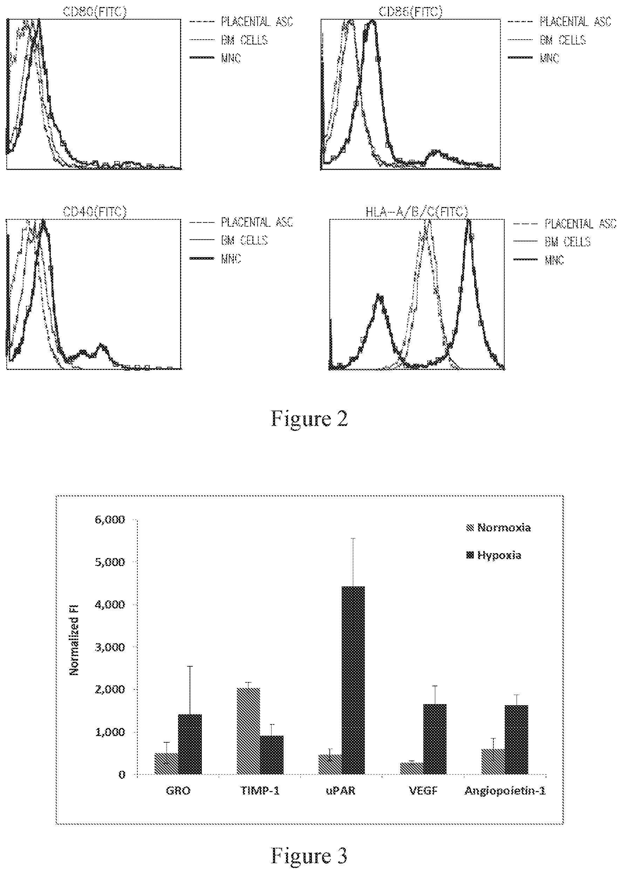

ASC were prepared as described in Example 1, and their expression of co-stimulatory molecules was measured. FACS analysis demonstrated the absence of CD80, CD86 and CD40 on the cell membranes **(****Figs. 2A-C****).** Moreover, the cells expressed low levels of HLA class I molecules, as detected by staining for HLA A/B/C **(****Fig. 2D****).** The ASC were also shown to escape allo-recognition.

### EXAMPLE 5: ASC STIMULA TE ENDOTHELIAL CELL PROLIFERA TION

### Protocol-Endothelial cell proliferation (ECP) assay:

Placental ASC were prepared as described in Example 1 and cryopreserved. 1 x 10⁶ thawed ASC were seeded in 2 ml DMEM medium. After 24 hours (hr), the medium was replaced with EBM-2 medium (Lonza Group Ltd, Basel, Switzerland), and cells were incubated under hypoxic conditions (1% O₂) for an additional 24 hr, after which the conditioned media (CM) was collected. In parallel, 750 human umbilical cord endothelial cells (HUVEC) were seeded, incubated for 24 hr, and then incubated with the CM, for 4 days under normoxic conditions at 37°C. After removal of the CM, the proliferation of the HUVEC cells was assayed using the AlamarBlue® fluorescent assay. Results are presented as the percent ECP (%ECP) observed in the absence of ASC (arbitrarily set at 100%).

### Results

ASC cultured under normoxic or hypoxic conditions were tested for protein secretion, using Cytokine (Human) Antibody Array C Series 4000 (RayBio). Secretion of several pro-angiogenic factors was up-regulated under hypoxic conditions, as shown in **Fig. 3****.**

In additional experiments, various batches of ASC were co-incubated with HUVEC cells to test their effect on ECP. Stimulation of ECP was observed, typically to levels at least 135% of the ECP observed in the absence of ASC.

### EXAMPLE 6: TREATMENT OF ASC WITH PRO-INFLAMMATORY CYTOKINES DURING 3D CULTURING

### Methods

*General experimental protocol.* ASC were obtained from the placenta and cultured under 2D conditions, then under 3D conditions, and were then harvested, all as described in Example 1, except that the last day of 3D culture (typically starting day 5 or 6) was performed in DMEM containing (or lacking [negative control]) 10 nanograms/milliliter (ng/ml) Tumor Necrosis Factor alpha (TNF-α), 10 ng/ml Interferon-Gamma (IFN-y), and/or 10% FBS (see Table 1), and the bioreactor was incubated for an additional day. Levels of secreted cytokines in the bioreactor medium were measured using the RayBio® Human Cytokine Array kit.

*Hypoxic incubation.* 1 x 10⁶ thawed ASC were seeded in 2 ml DMEM medium. After 24 hours (hr), the medium was replaced with EBM-2 medium (Lonza Group Ltd, Basel, Switzerland), and cells were incubated under hypoxic conditions (1% O₂) for an additional 24 hr, after which the CM was collected.

**Table 1. Incubation conditions that were tested.**

| Designation | Cytokines | FBS |
|---|---|---|
| 1 | None | NO |
| 2 | None | YES |
| 3 | TNF | NO |
| 4 | TNF | YES |
| 5 | TNF+IFN | NO |
| 6 | TNF+IFN | YES |

In some experiments, levels of secreted cytokines were measured in the CM from a subsequent hypoxic incubation, as described above.

*Quantitative detection of secreted proteins:* IL-6 and VEGF were quantitatively measured using the respective human immunoassay Quantikine® ELISA kits (R&D Systems).

### Results

In a series of experiments testing various conditions side-by-side, ASC were incubated in a bioreactor as described in the previous Examples. On the last day of the bioreactor incubation, the medium was replaced by medium containing or lacking added TNF-α, or TNF-α + IFN-γ, in the presence or absence of FBS. VEGF and IL-6 secretion were measured in the bioreactor medium by ELISA. Inclusion of TNF-α significantly increased secretion of VEGF, whether or not IFN-γ was present (**Table 2**).

**Table 2. Secretion of VEGF (picograms/ml [pg/ml]) by ASC under various conditions.**

| Expt. # | Cytokines | FBS | VEGF in CM / RPD* | VEGF in bioreactor medium / RPD* |
|---|---|---|---|---|
| 1 | TNF+IFN | NO | 619 / 3 | 195 / 3 |
| | None | NO | 274 / 7 | 65 / 0 |
| 2 | TNF+IFN | NO | 7540 / 1 | 151 / 3 |
| | None | NO | 3266 / 4 | 140 / 3 |
| 3 | TNF+IFN | YES | 371 / 3 | 1749 / 2 |
| | TNF | YES | 370 / 10 | 1128 / 5 |
| 4 | TNF+IFN | YES | NT (not tested) | 373 / 2 |
| | TNF | YES | NT | 348 / 8 |
| 5 | TNF+IFN | NO | 732 ± 20** | (not performed) |
| | None | NO | 650 ± 46** | (not performed) |

| | | | | |
|---|---|---|---|---|
| *In this table and throughout the document, except where indicated otherwise, RPD refers to the percentage difference between duplicate samples in the ELISA. **Indicated number is the standard deviation. | | | | |

Another experiment showed that inclusion of TNF-α significantly increased IL-6 secretion, which was further increased by IFN-γ.

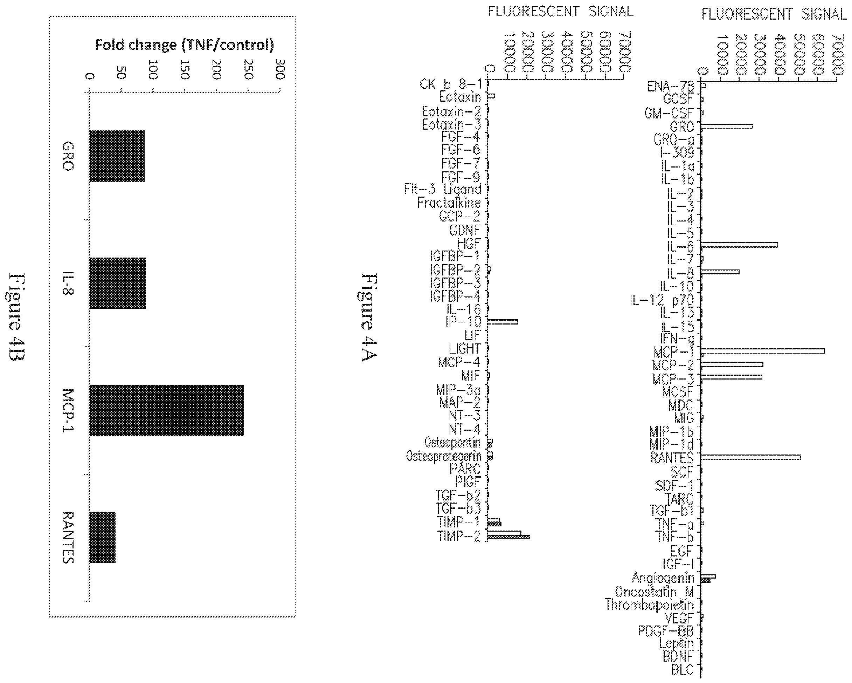

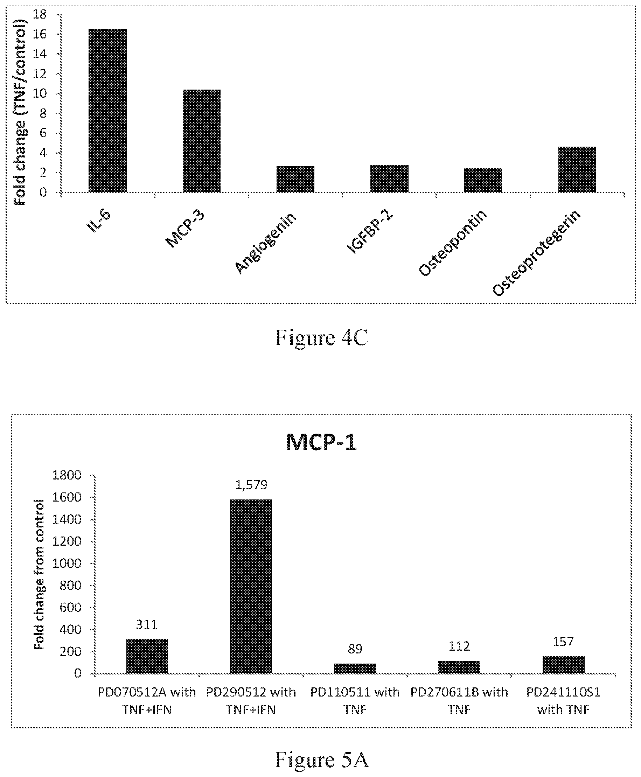

The experiments described below, through the end of this Example, were all performed on samples grown in medium lacking serum. Bioreactor media from selected samples from the aforementioned experiments were probed for expression of a panel of factors, using a fluorescence-based cytokine array assay. Increased expression of several factors, including GRO (CXCL1; Uniprot Accession No. P09341), IL-6, IL-8, MCP-1, MCP-2, MCP-3, RANTES, and IP-10 (Uniprot Accession No. P02778), was observed following cytokine incubation (**Fig. 4A**). In another experiment, TNF-α alone was compared to no added cytokines, showing increased expression of GRO, IL-8, MCP-1, RANTES, and, to a lesser extent, IL-6, MCP-3, Angiogenin, Insulin-like Growth Factor Binding Protein-2 (IGFBP-2), Osteopontin, and Osteoprotegerin (**Figs. 4B-C**).

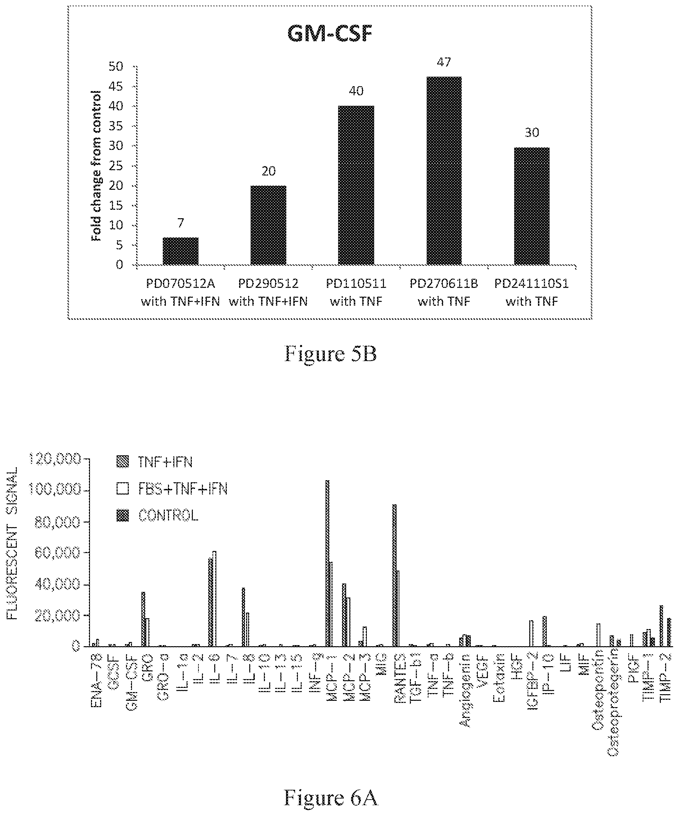

Increased expression of MCP-1 and GM-CSF in the bioreactor media was verified by quantitative ELISA in several experiments. The results showed that TNF-α + IFN-γ was more potent than TNF-α alone for MCP-1 induction (**Fig. 5A**), while TNF-α alone appeared to be slightly superior for GM-CSF induction (**Fig. 5B**). The cytokine concentrations and fold-changes relative to control medium (containing no cytokines) from the TNF-α + IFN-γ trial are shown in **Table 3** below.

**Table 3. MCP-1 and GM-CSF concentrations in bioreactor medium.**

| Expt. No. | Conditions | MCP-1 (pg/ml) (fold-increase) | GM-CSF (pg/ml) (fold-increase) |
|---|---|---|---|
| 1 | TNF+IFN | 6365.4 (311) | 6.32 (6.9) |
| | None | 20.5 | 0.91 |
| 2 | TNF+IFN | 9063.7 (1579) | 13.09 (20.0) |
| | None | 5.8 | 0.65 |

The induction of several other factors, over several experiments utilizing TNF-α + IFN-γ, or TNF-α alone, was detected by the aforementioned cytokine array. A number of proteins were consistently upregulated, as depicted in **Table 4.**

**Table 4. Fold-enrichment (relative to no-cytokine control cells) of selected proteins upon incubation with TNF-α +/- IFN-γ. Only fold-changes greater than 2 are depicted.**

| Condition/ Expt. No. | TNF + IFN / expt. 1 | TNF + IFN / expt. 2 | TNF alone / expt. 6 |
|---|---|---|---|
| Proteins | | | |
| ENA-78 | 13.0 | 11.4 | |
| GCSF | 4.6 | 3.3 | |
| GM-CSF | 3.7 | 3.1 | |
| GRO | 57.8 | 102.7 | 87 |
| GRO-a | 2.9 | 2.5 | |
| IL-2 | 3.8 | 3.2 | |
| IL-6 | 199.2 | 281.4 | 16.5 |
| IL-7 | 4.6 | 2.5 | |
| IL-8 | 32.6 | 80.5 | 88.7 |
| IL-10 | 3.2 | 3.5 | |
| IFN-g | 2.9 | 2.8 | |
| MCP-1 | 88.3 | 529.3 | 243.3 |
| MCP-2 | 88.3 | 198.5 | |
| MCP-3 | 160.7 | 18.0 | 10.4 |
| MIG | 158.2 | 3.2 | |
| RANTES | 4.4 | 452.1 | 41.3 |
| TGF-b1 | 256.7 | 3.5 | |
| VEGF | 4.3 | | |
| Eotaxin | 17.6 | 2.1 | |
| IGFBP-2 | 2.3 | | 2.8 |
| IP-10 | 75.0 | 94.7 | |
| MIF | 3.0 | 2.9 | |
| Angiogenin | | | 2.7 |
| Osteopontin | | | 2.5 |
| Osteoprotegerin | | | 4.6 |

Similar results to those presented hereinabove in this Example were obtained with ASC stimulated with a bolus of cytokines (as described hereinbelow in Example 9), except that much larger upregulation of GM-CSF was observed, expression of G-CSF, HGF, and TRAIL was tested and found to be upregulated, and TNF-α was not upregulated. The increased expression of the proteins mentioned in this Example was also confirmed on the mRNA level, using quantitative polymerase chain reaction.

### EXAMPLE 7: THE EFFECT OF SERUM ON PRO-INFLAMMATORY CYTOKINE TREATMENT OF ASC DURING 3D CULTURING

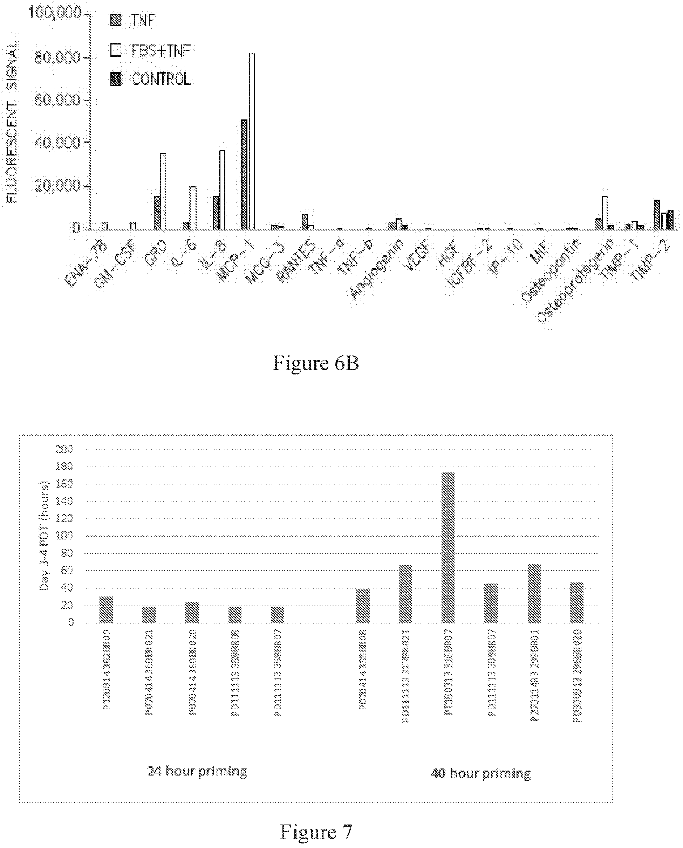

This experiment examined the effect of FBS on induction of the aforementioned panel of factors by TNF-α + IFN-γ (**Fig. 6A**) or TNF-α alone (**Fig. 6B**). A similar set of major proteins was induced in the presence or absence of FBS. For TNF-alpha alone, IL-6 appeared to be induced much more strongly in the presence of FBS than in its absence.

### EXAMPLE 8: MARKER PHENOTYPE OF ASC TREATED WITH INFLAMMATORY CYTOKINES

The marker phenotype of the ASC that had been pre-treated with pro-inflammatory cytokines was examined over several experiments. Consistently, the cells were over 90% positive for CD29, CD90, and CD54; over 85% positive for CD73 and CD105; and over 65% positive for CD49. Additionally, the cells were less than 1% positive for CD14, CD19, CD31, CD34, CD39, and CD45; less than 3% were positive for CD200; less than 6% were positive for GlyA; and less than 20% were positive for SSEA4.

### EXAMPLE 9: ALTERED CYTOKINE CONDITIONS IMPROVE CELL VITALITY

ASC were stimulated with inflammatory cytokines in a similar manner to that described in Example 6, with two exceptions: 1. The cytokine exposure was for 24 hours; and 2. Cytokines were spiked into the bioreactor medium, using a concentrated stock solution at the beginning of the 24-hr incubation, rapidly bringing the cytokine concentration up to the target. Over the following 24 hrs, fresh medium containing the target cytokine concentration was perfused into the bioreactor. The 24-hr incubation began 5 days after seeding the bioreactor, corresponding to exponential growth phase. By the conclusion of cytokine treatment, cellular growth had reached the point that the rate of doubling began to slow. The ASC were frozen. Thawed cells were seeded on tissue culture dishes, and population doubling time (PDT) was measured, by measuring and comparing cell densities in plates harvested 3 and 4 days after seeding.

The 24-hr-stimulated cells exhibited a significant reduction in PDT (**Fig. 7**). **Table 5** sets forth the conditions of each sample.

**Table 5. Conditions of samples depicted in Fig. 7.**

| **Run** | **TNF/IFN concentration** | **Hours exposure** | **PDT** |
|---|---|---|---|
| 277BR021 PT180313 | 10/10 | 40 | 49.7 |
| 296BR020 PD300913 | 10/10 | 40 | 74.2 |
| 299BR01 P270114R3 | 10/10 | 40 | 67.2 |
| 309BR07 PD111113S6 | 10/10 | 40 | 55.5 |
| 316BR07 PT180313 | 10/10 | 40 | 126.5 |
| 317BR021 PD111113S7 | 10/10 | 40 | 83 |
| 335BR08 P070414 | 10/10 | 40 | 95.2 |
| 358BR07 PD111113 | 10/10 | 24 | 19 |
| 358BR08 PD111113 | 1/1 | 24 | 19.3 |
| 360BR020 P070414 | 5/5 | 24 | 24.7 |
| 360BR021 P070414 | 10/10 | 24 | 19.4 |

### EXAMPLE 10: ASC CM AFFECTS TUMOR CELL REPLICATION AND SURVIVAL

### METHODS

*CM production:* Bioreactor incubations and subsequent cryopreservation were performed as described in Examples 1 and 6. Following these steps, 500,000 cells were seeded in multi-well plates in 4 ml DMEM supplemented with 2 mM L-glutamine and 10% FBS, in some cases with the addition of 40 ng/well IFN-γ. After 24 hours, the medium was aspirated, the cells were washed, and RPMI (without FBS, unless otherwise indicated) was added. After a 24-hr incubation, the medium was collected and centrifuged, and 5% FBS was added to the medium.

*Anti-cancer assay.* 59 cell lines were grown in medium (RPMI + 10% FBS, 2 mM L-alanyl-L-Glutamine, and 1 mM Sodium Pyruvate) and seeded in the above medium, but with 5% FBS, to form spheroids, in multi-well 3D plates (Elplasia™ plates, which contain micro-spaces on the surface that allow cells to self-assemble) pre-coated with polyhydroxyethylmethacrylate (pHEMA). CM was serially diluted 2-fold and assayed over several concentrations in triplicate. CM, neat or diluted 1:2, 1:4, or 1:8, was added 24 hours post seeding in a volume of 25 µL and was exchanged every 3 days. Controls (positive and negative) were included for every cell line. Cells were lysed and analyzed using a CellTiter-Glo® Cell Viability Assay, to determine the effects of the CM on the viability and replication of the cells. An inhibition of 20-40% relative to vehicle was statistically significant relative to the standard deviations and was defined as partial inhibition, while an inhibition of 40% or more was defined as inhibition.

### RESULTS

ASC, either maternal or mixed maternal/fetal, were produced in a bioreactor and used to prepare conditioned media (CM). CM was prepared from maternal and maternal/fetal batches of ASC, some of which were subjected to treatment prior to or during CM production (**Table 6**), and tested for the ability to inhibit replication of various cancer cell lines (**Table 7**).

**Table 6. Tested cell lines.**

| Group | Composition | Special treatment |
|---|---|---|
| 1 | maternal | TNF-α + IFN-γ on last day of bioreactor incubation as described in Example 6 |
| 2 | maternal / fetal | None |
| 3 | maternal / fetal | IFN-γ present on the first day of CM production |
| 4 | maternal / fetal | FBS present on the second day of CM production |

**Table 7. Cell lines used for anti-cancer testing.**

| Cell Line | ATCC Cat. # | Cancer Type | Organ | Organ Notes |
|---|---|---|---|---|
| 22Rv1 | CRL-2505 | Prostate carcinoma | Prostate | |
| 647-V | ACC-414 | Urothelial bladder carcinoma | Bladder | |
| 769-P | CRL-1933 | Renal cell adenocarcinoma | Kidney | clear cell renal cell carcinoma |
| 786-O | CRL-1932 | Renal cell adenocarcinoma | Kidney | clear cell renal cell carcinoma |
| A-498 | HTB-44 | Renal cell carcinoma | Kidney | |
| A549 | CCL-185 | Non-small cell carcinoma | Lung | |
| ACHN | CRL-1611 | Renal cell adenocarcinoma | Kidney | |
| AGS | CRL-1739 | Gastric adenocarcinoma | Stomach | |
| AsPC-1 | CRL-1682 | Pancreatic adenocarcinoma | Pancreas | Ductal carcinoma |
| BT474 | HTB-20 | Breast ductal carcinoma | Breast | Breast/duct; Mammary gland |
| C32 | CRL-1585 | Malignant melanoma | Skin | |
| C3A | CRL-10741 | Hepatocellular carcinoma | Liver | |
| Cal 27 | CRL-2095 | Squamous cell carcinoma | Head/Neck | Head and Neck (tongue) |
| CAL-62 | ACC 448 | Thyroid anaplastic carcinoma | Thyroid | |
| Calu-6 | HTB-56 | Lung anaplastic carcinoma | Lung | |
| CHL-1 | CRL-9446 | Melanoma | Skin | |
| Colo 205 | CCL-222 | Colorectal adenocarcinoma | Colon/ Rectum | Colon/GI |
| Colo 320 HSR | CCL-220.1 | Colorectal adenocarcinoma; Dukes' type C | Colon/ Rectum | Colon |
| COLO 829 | CRL-1974 | Melanoma; Fibroblast | Skin | |
| DBTRG-05MG | CRL-2020 | Astrocytoma | Brain | |
| DLD-1 | CCL-221 | Colorectal adenocarcinoma | Colon/ Rectum | Dukes' type C, colorectal adenocarcinoma |
| DU 145 | HTB-81 | Prostate carcinoma | Prostate | Prostate; derived from metastatic site: brain |
| ES-2 | CRL-1978 | Ovarian clear cell carcinoma | Ovary | |
| FaDu | HTB-43 | Hypopharyngeal squamous cell carcinoma | Pharynx | |
| HCC1395 | CRL-2324 | Breast carcinoma | Breast | Mammary gland, breast |
| HCT 116 | CCL-247 | Colorectal carcinoma | Colon/ Rectum | Colon |
| HCT-15 | CCL-225 | Colorectal adenocarcinoma; Dukes' type C | Colon/ Rectum | Colon |
| Hela | CCL-2 | Adenocarcinoma | Cervix | Female GU (Cervix) |
| Hep 3B2.1-7 | HB-8064 | Hepatocellular carcinoma | Liver | |
| Hep G2 | HB-8065 | Hepatocellular carcinoma | Liver | |
| HT-1376 | CRL-1472 | Urinary bladder carcinoma | Bladder | Transitional cell carcinoma |
| HT-29 | HTB-38 | Colorectal adenocarcinoma | Colon/ Rectum | Colon |
| Huh 7 | Huh7 | Hepatocellular carcinoma | Liver | |
| J82 | HTB-1 | Urinary bladder transitional cell carcinoma | Bladder | |
| LNCaP clone FGC | CRL-1740 | Prostate adenocarcinoma; metastatic | Prostate | |
| LS 174T | CL-188 | Colorectal adenocarcinoma; Dukes' type B | Colon/ Rectum | Colon |
| MCF7 | HTB-22 | Breast adenocarcinoma | Breast | Breast; mammary gland, derived from metastatic site: pleural effusion |
| MDA-MB-231 | HTB-26 | Breast adenocarcinoma | Breast | Breast; mammary gland, derived from metastatic site: pleural effusion |
| MDA-MB-453 | HTB-131 | Breast carcinoma; metastatic | Breast | Breast; mammary gland, derived from metastatic site: pericardial effusion |
| MES-SA | CRL-1976 | Uterine sarcoma | Uterus | |
| Mia PaCa-2 | CRL-1420 | Pancreatic carcinoma | Pancreas | Ductal carcinoma |
| NCI-H1792 | CRL-5859 | Lung adenocarcinoma | Lung | Lung, derived from metastatic site:pleural effusion |
| NCI-H23 | CRL-5800 | Lung adenocarcinoma, NSCL | Lung | |
| NCI-H358 | CRL-5807 | Bronchioalveolar carcinoma, NSCL | Lung | Lung; Bronchiole |
| NCI-H460 | HTB-177 | Lung carcinoma; large cell | Lung | Lung; plueral effusion |
| PC-3 | CRL-1435 | Prostate adenocarcinoma | Prostate | Prostate; derived from metastatic site, bone |
| RD | CCL-136 | Rhabdomyosarcoma | Muscle | |
| SK-MEL-3 | HTB-69 | Melanoma | Skin | Skin; derived from Metastatic Site: lymph node |
| SK-N-AS | CRL-2137 | Neuroblastoma | Brain | Brain; derived from metastatic site, BM |
| SK-OV-3 | HTB-77 | Ovarian adenocarcinoma | Ovary | Ovary; ascites |
| SNU-449 | CRL-2234 | Hepatocellular carcinoma; grade II-III/IV | Liver | |
| SW1088 | HTB-12 | Astrocytoma | Brain | |
| SW48 | CCL-231 | Colorectal adenocarcinoma; Dukes' type C, grade IV | Colon/ Rectum | Colon |
| SW480 | CCL-228 | Colorectal adenocarcinoma; Dukes' type B | Colon/ Rectum | Colon |
| SW620 | CCL-227 | Colorectal adenocarcinoma | Colon/ Rectum | Colon; derived from metastatic site: lymph node |
| T24 | HTB-4 | Carcinoma | Bladder | |
| T-47D | HTB-133 | Ductal carcinoma | Breast | Mammary gland; derived from metastatic site: pleural effusion |
| U-87 MG | HTB-14 | Astrocytoma | Brain | CNS |
| UM-UC-3 | CRL-1749 | Urinary Bladder carcinoma; transitional cell | Bladder | |

The below analysis focuses on experimental Group 1, which received CM from ASC treated with TNF-alpha / IFN-gamma. Overall:
- 12 cell lines were inhibited (<60% Proliferation) by Group 1.
- 14 cell lines were partially inhibited (60-80% Proliferation) by Group 1.
- 28 cell lines were not inhibited by the CM.
- 4 cell lines were partially stimulated (120-140% Proliferation) by Group 1.
- 1 cell line was stimulated (>140% Proliferation) by Group 1.

Several cancer types exhibited inhibition (defined as at least a 40% reduction in proliferation) by at least the highest concentration of Group 1, namely renal cell carcinoma (2/4 cell lines tested; **Table 8**), melanoma (1/4 lines), hepatocellular carcinoma (2/5 lines; **Table 9**), colorectal carcinoma (2/10), breast carcinoma (2/6 lines; **Table 10**), lung adenocarcinoma (1/1 lines; **Table 11**), large cell lung carcinoma (1/1), and rhabdomyosarcoma (1/1 lines; **Table 12**).

**Table 8. Inhibition of renal cell carcinoma proliferation by ASC.**

| % Proliferation relative to control | | | | Dose response of Group 1 treatment | | |
|---|---|---|---|---|---|---|
| | Cell Line | | | | Cell Line | |
| Group | 769-P | 786-O | | Dilution | 769-P | 786-O |
| Group 1 | 54 | 49 | | 0.125 | 105 | 86 |
| Group 2 | 77 | 71 | | 0.25 | 97 | 90 |
| Group 3 | 88 | 79 | | 0.5 | 85 | 80 |
| Group 4 | 126 | 105 | | 1 | 54 | 49 |
| RPMI | 95 | 90 | | | | |
| RPMI | 100 | 98 | | | | |

**Table 9. Inhibition of hepatocellular carcinoma proliferation by ASC.**

| % Proliferation relative to control | | | | Dose response of Group 1 treatment | | |
|---|---|---|---|---|---|---|
| | Cell Line | | | | Cell Line | |
| Group | Hep G2 | SNU-449 | | Dilution | Hep G2 | SNU-449 |
| Group 1 | 55 | 56 | | 0.125 | 92 | 103 |
| Group 2 | 48 | 67 | | 0.25 | 81 | 98 |
| Group 3 | 49 | 83 | | 0.5 | 85 | 87 |
| Group 4 | 44 | 80 | | 1 | 55 | 56 |
| RPMI | 86 | 108 | | | | |
| RPMI | 99 | 104 | | | | |

**Table 10. Inhibition of breast carcinoma proliferation by ASC.**

| % Proliferation relative to control | | | | Dose response of Group 1 treatment | | |
|---|---|---|---|---|---|---|
| | Cell Line | | | | Cell Line | |
| Group | MDA-MB-231 | HCC-1395 | | Dilution | MDA-MB-231 | HCC-1395 |
| Group 1 | 48 | 60 | | 0.125 | 82 | 76 |
| Group 2 | 56 | 72 | | 0.25 | 91 | 77 |
| Group 3 | 52 | 65 | | 0.5 | 63 | 91 |
| Group 4 | 71 | 83 | | 1 | 48 | 60 |
| RPMI | 79 | 77 | | | | |
| RPMI | 97 | 96 | | | | |

**Table 11. Inhibition of NCI-H1792 (lung adenocarcinoma) proliferation by ASC.**

| Group | % Proliferation relative to control | | Dilution | Dose response: Group 1 |
|---|---|---|---|---|
| Group 1 | 20 | | 0.125 | 88 |
| Group 2 | 35 | | 0.25 | 69 |
| Group 3 | 33 | | 0.5 | 54 |
| Group 4 | 57 | | 1 | 20 |
| RPMI | 94 | | | |
| RPMI | 90 | | | |

**Table 12. Inhibition of RD (rhabdomyosarcoma) proliferation by ASC.**

| Group | % Proliferation relative to control | | Dilution | Dose response: Group 1 |
|---|---|---|---|---|
| Group 1 | 36 | | 0.125 | 88 |
| Group 2 | 56 | | 0.25 | 80 |
| Group 3 | 52 | | 0.5 | 66 |
| Group 4 | 64 | | 1 | 36 |
| RPMI | 95 | | | |
| RPMI | 92 | | | |

### EXAMPLE 11: DIFFERENTIALLY EXPRESSED GENE ANALYSIS BETWEEN RESPONSIVE CELL LINES AND OTHER CELL LINES

To identify marker genes differentially expressed between the responsive and non-responsive cancer cell lines to ASC-TNFα/IFNγ treatment, cell lines were grouped by organ. Therefore, the cancer cell lines chosen for marker gene selection came from five organs: breast, large intestine, kidney, liver and lung. The responsive cell lines RD (rhabdomyosarcoma) and CHL-1 (melanoma) were excluded from the investigation, because there was insufficient data to ensure two cell lines for each of the two classes for cancers originating from these organs.

The two classes were assigned for each organ as follows (**Table 13**):
- Class 0: responsive cell lines (percent of control (POC) ≤ 60% with undiluted CM).
- Class 1: cell lines having a POC ≥ 79% with undiluted CM.

Marginally responsive cell lines defined as having 60% < POC < 79% were excluded from both classes.

**Table 13. The cell line matrix for ComparativeMarkerSelection input.**

| | Class 0 Cell Lines | Class 1 Cell Lines |
|---|---|---|
| Breast | HCC1395 | BT474 |
| | MDA-MB231 | MCF7 |
| | | T47D |
| Large Intestine | HT29 | COLO320 |
| | SW48 | DLD1 |
| | | HCT15 |
| | | SW480 |
| | | SW620 |
| | | HCT116 |
| Liver | HEPG2 | C3A |
| | SNU449 | HUH7 |
| Lung | NCIH1792 | A549 |
| | NCIH460 | NCIH23 |
| Kidney | 769P | ACHN |
| | 786O | A498 |

Gene expression data was obtained from the Cancer Cell Line Encyclopedia (CCLE; Barretina, J., et al), which provides access to genomic data, analysis and visualization for over 1000 cell lines. mRNA expression array data for the cancer cell lines used in the cell proliferation assay were downloaded for identifying marker genes. Prior to downloading the data sets, the raw Affymetrix CEL files from the original Affymetrix U133+2 arrays were converted to a single value for each probe set using Robust Multi-array Average (RMA) and normalized using quantile normalization. A redefined custom CDF file from the package HGU133Plus2_Hs_ENTREZG_15.0.0 from Brainarray was used for the summarization.

In order to identify and select marker genes, the ComparativeMarkerSelection module in GenePattern (Reich et al) was employed.

Genes were scored by calculating the value of the two-sided t-test for each profiled gene. Marker genes were selected if the test statistic was >5 or <-5. Positive and negative values indicate upregulated genes and downregulated genes, respectively, in the responsive cell lines. **Table 14** shows the numbers of marker genes with scores > 5 and < -5.

**Table 14. Numbers of marker genes with scores > 5 and < -5.**

| | Breast | Kidney | Large Intestine | Liver | Lung |
|---|---|---|---|---|---|
| Up | 412 | 494 | 91 | 190 | 297 |
| Down | 382 | 318 | 112 | 151 | 452 |
| Total | 794 | 812 | 203 | 341 | 749 |

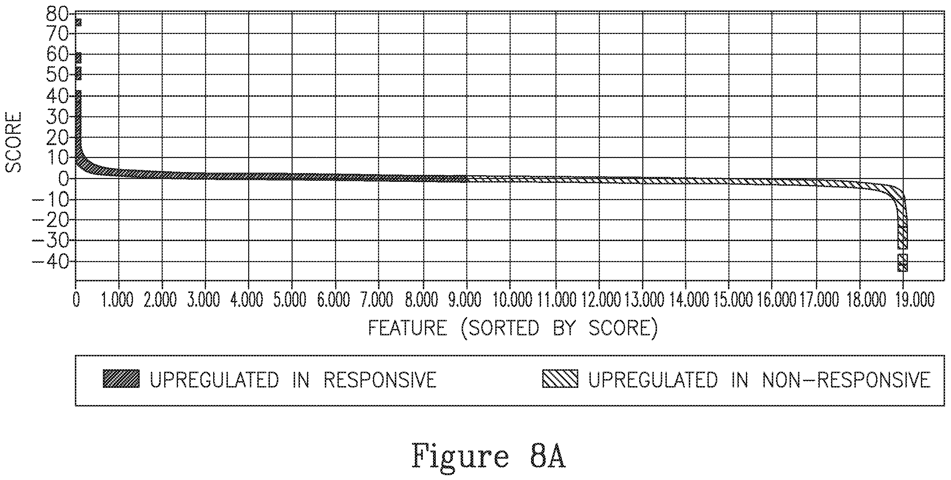

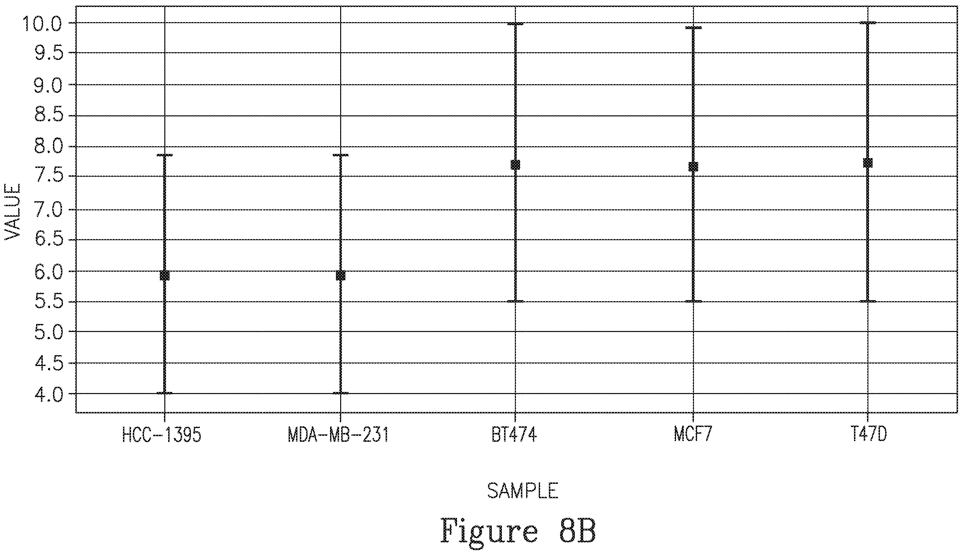

As an example, **Fig. 8A** depicts a graphical representation of the scores for each profiled gene for the breast cancer cell lines analysis. The upregulated genes in the responsive cell lines are shown on the left side of the graph, while the downregulated genes in the responsive cell lines (upregulated in the other cell lines) are shown on the right side. **Fig. 8B** is a centroid plot showing the mean expression value for the five breast cancer cell lines for all of the genes downregulated (scores < -5) in the responsive breast cell lines. The two responsive breast cancer cell lines (HCC-1395 and MDA-MB-231) are shown on the left, and the other three breast cancer cell lines (BT474, MCF7 and T47D) are shown on the right.

### EXAMPLE 12: PATHWAYS SIGNIFICANTLY PERTURBED IN RESPONSIVE LINES

To determine relevant biological pathways that are perturbed between Class 0 and Class 1 cell lines within each organ, the most statistically significantly upregulated and downregulated genes within each organ were used to probe the Reactome Pathway Database V53 (Croft et al). **Table 15** shows the number of upregulated and downregulated genes from each of the five organs that are found in at least one Reactome pathway.

**Table 15. Numbers of up- and downregulated genes found in Reactome pathways.**

| **DE Genes Responsive Cell Lines vs Other (Score > 5 and < -5)** | | | | | |
|---|---|---|---|---|---|
| | **Breast** | **Kidney** | **Large Intestine** | **Liver** | **Lung** |
| Up | 412 | 494 | 91 | 190 | 297 |
| Down | 382 | 318 | 112 | 151 | 452 |
| **Total** | 794 | 812 | 203 | 341 | 749 |
| | | | | | |
| Up-Reactome | 191 | 213 | 26 | 62 | 127 |
| Down-Reactome | 120 | 74 | 58 | 63 | 93 |
| **Total** | 311 | 287 | 84 | 125 | 220 |

The data was searched for significant pathways that were common among the organs. The following 5 pathways were in the list of the top 200 Reactome pathways in 4/5 output lists:
1. RIG-I/MDA5 mediated induction of IFN-alpha/beta pathways (R-HSA-168928)
2. Interferon Signaling (R-HSA-913531)
3. Cytokine Signaling in Immune system (R-HSA-1280215)
4. Cellular Senescence (R-HSA-2559583)
5. Deactivation of the beta-catenin trans-activating complex (R-HSA-3769402)

Next, the most statistically significant upregulated and downregulated genes were pooled from each of the five marker gene sets and used to probe the Reactome Pathway Database. The database was probed three times:
1. All upregulated genes
2. All downregulated genes
3. All upregulated and downregulated genes

The statistical cut-off for defining a biological pathway as statistically significant was an entities false discovery rate (FDR) ≤ 0.05. **Tables 16-18** depict the most statistically relevant biological pathways perturbed between Classes 0 and 1 across the 5 organs due to upregulated genes, downregulated genes, and both, respectively. The bold-faced pathways in **Table 17** survive the selection process when mutated genes are added to the analysis (**Table 20**).

**Table 16**

| **Most Statistically Significant Pathways Perturbed Due to Upregulated Genes** | | |
|---|---|---|
| **Pathway name** | **Entities pValue** | **Entities FDR** |
| Factors involved in megakaryocyte development and platelet production | 1.08E-05 | 1.59E-02 |
| Cellular Senescence | 2.06E-05 | 1.59E-02 |
| Mitochondrial biogenesis | 4.83E-05 | 2.30E-02 |
| Hemostasis | 5.95E-05 | 2.30E-02 |
| Signaling by NOTCH | 1.03E-04 | 3.18E-02 |
| Organelle biogenesis and maintenance | 1.84E-04 | 4.73E-02 |

**Table 17**

| **Most Statistically Significant Pathways Perturbed Due to Downregulated Genes** | | |
|---|---|---|
| **Pathway name** | **Entities pValue** | **Entities FDR** |
| **Interferon alpha/beta signaling** | 6.32E-09 | 8.18E-06 |
| **Cytokine Signaling in Immune system** | 3.78E-08 | 2.44E-05 |
| **Interferon Signaling** | 8.83E-08 | 3.81E-05 |
| Ion channel transport | 4.33E-05 | 1.28E-02 |
| **Interferon gamma signaling** | 4.95E-05 | 1.28E-02 |
| RIG-I/MDAS mediated induction of IFN-alpha/beta pathways | 8.29E-05 | 1.78E-02 |
| Activation of gene expression by SREBF (SREBP) | 1.23E-04 | 2.27E-02 |
| PPARA activates gene expression | 2.53E-04 | 3.99E-02 |
| **Endosomal/Vacuolar pathway** | 3.15E-04 | 3.99E-02 |
| Regulation of lipid metabolism by PPARalpha | 3.16E-04 | 3.99E-02 |
| TRAF6 mediated IRF7 activation | 3.41E-04 | 3.99E-02 |

**Table 18**

| | | |
|---|---|---|
| **Most Statistically Significant Pathways Perturbed Due to Upregulated and Downregulated Genes** | | |

| **Pathway name** | **Entities pValue** | **Entities FDR** |
|---|---|---|
| PPARA activates gene expression | 1.47E-05 | 1.82E-02 |
| Regulation of lipid metabolism by PPARalpha | 2.16E-05 | 1.82E-02 |

As can be seen, the biological pathways that show the highest statistical significance are revealed when the database is probed by the pooled set of the Class 0 downregulated genes.

### EXAMPLE 13: MUTATION ANALYSIS OF EXOMES OF RESPONSIVE AND OTHER CELL LINES

To probe the pathways of the greatest biological significance (besides the described statistical significance), somatic mutations were analyzed via full exome sequencing from the COSMIC Cancer Cell Lines Project (Forbes et al). Addition, deletion, substitution, frameshift and splice site mutations were included in the count, whereas CDS silent mutations were excluded. Data from Hep-G2, AGS, DLD1, LS-174T and SW480 did not appear in the database. Besides these cell lines, a total of >10,300 mutations were counted in the 11 responsive cell lines and >53,000 mutations in the other 43 cell lines.

The Reactome database was probed with:
- all genes mutated exclusively in the 11 responsive cell lines
- all genes mutated exclusively in the other 43 cell lines (the marginal and non-responsive lines).
- the pooled list of all genes mutated exclusively in the responsive cell lines; and all genes downregulated in the responsive cell lines (as described for Table 15 above).

The final query was the most informative, so its results are described hereinbelow.

**Table 19** shows the numbers of genes exclusively mutated in the responsive lines:

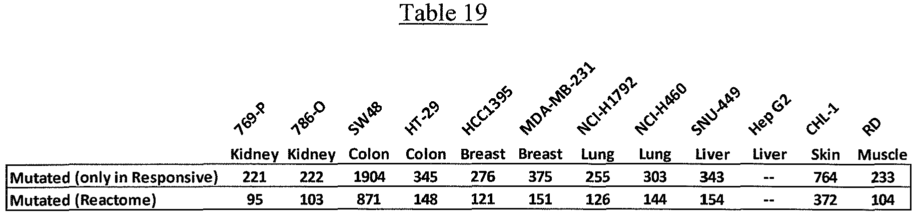

The results of the final query are shown in Table 20. The statistical cut-off was an entities FDR≤ 0.05.

**Table 20**

| Pathway name | **Entities pValue** | **Entities FDR** | **Reactions found** | **Reactions total** |
|---|---|---|---|---|
| Endosomal/Vacuolar pathway | 1.11E-16 | 9.78E-14 | 4 | 4 |
| Interferon alpha/beta signaling | 1.11E-16 | 9.78E-14 | 14 | 19 |
| Antigen Presentation: Folding, assembly and peptide loading of class I MHC | 3.77E-15 | 2.22E-12 | 13 | 14 |
| Interferon gamma signaling | 1.34E-12 | 5.89E-10 | 11 | 15 |
| Interferon Signaling | 2.77E-12 | 9.74E-10 | 41 | 50 |
| ER-Phagosome pathway | 8.61E-12 | 2.52E-09 | 3 | 5 |
| Antigen processing-Cross presentation | 3.82E-10 | 9.59E-08 | 8 | 17 |
| Class I MHC mediated antigen processing & presentation | 5.35E-08 | 1.18E-05 | 28 | 39 |
| Cytokine Signaling in Immune system | 7.45E-07 | 1.45E-04 | 200 | 285 |

This analysis showed that the responsive cancer cell lines are those cell lines that have a downregulation or a dysregulation in two significant pathways: MHC Class I antigen processing and presentation (which includes the endosomal/vacuolar, antigen presentation: folding, assembly and peptide loading of class I MHC, ER-phagosome, and antigen processing-cross presentation pathways) and cytokine signaling (which includes the interferon alpha/beta signaling, interferon gamma signaling, and interferon signaling pathways). These pathways overlap significantly with the pathways found statistically significantly in the previous analysis (**Table 17**), validating the statistical analyses and the importance of these particular pathways.

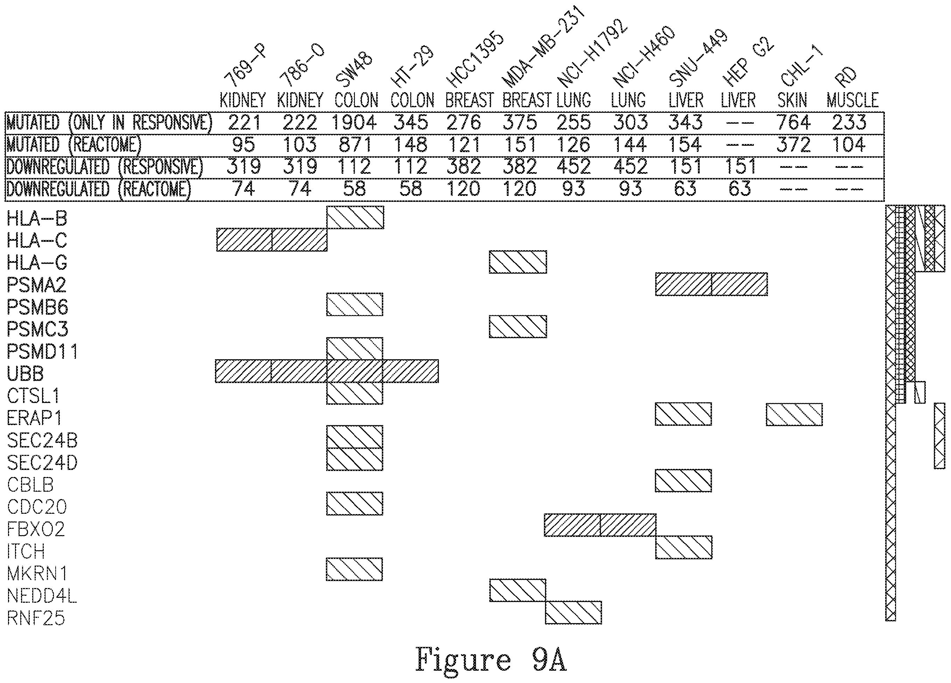

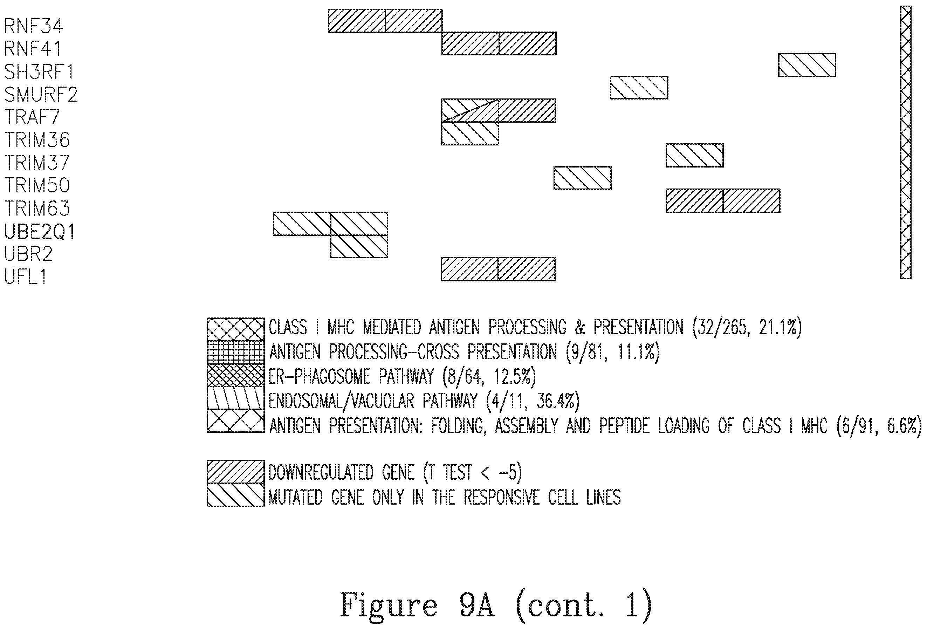

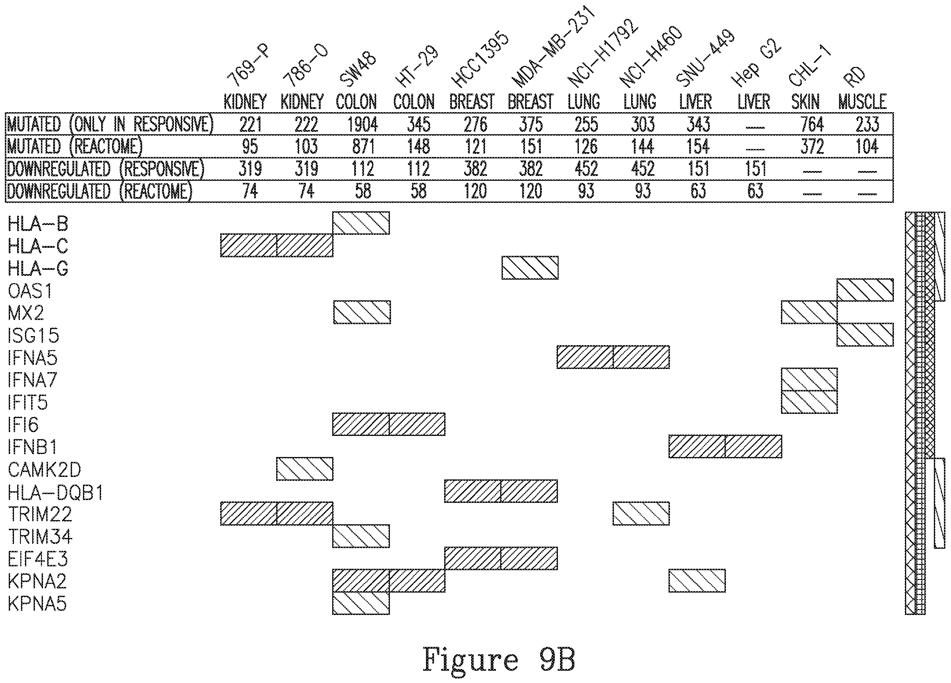

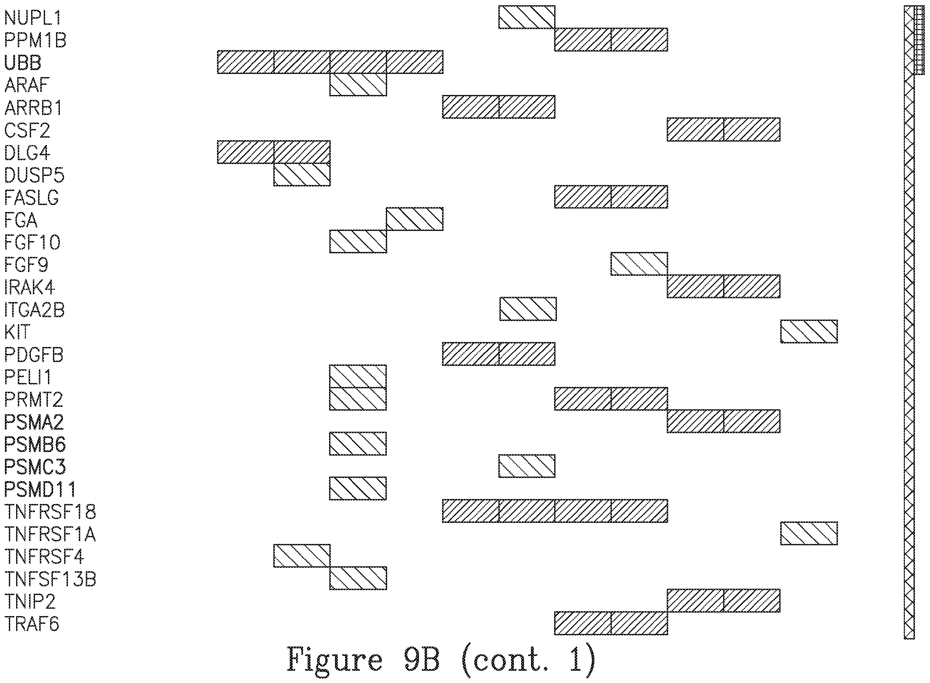

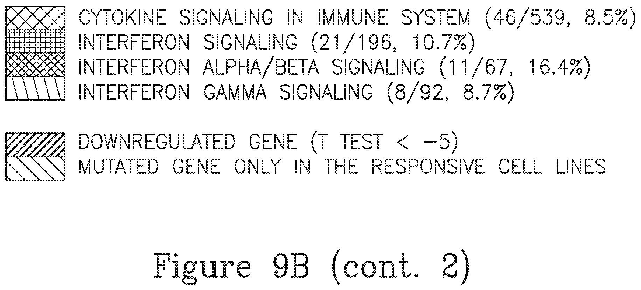

**Figs. 9A-B** summarize the genes in these pathways that are downregulated and/or exclusively mutated in each of the responsive cell lines. In **A,** the first 8 genes listed are also present in the cytokine signaling/interferon pathway. The last 19 genes, except for UBE2Q1, are involved in the ubiquitin ligase pathway. In **B,** the 3 HLA genes, UBB, and the 3 genes beginning with PSM are also present in the antigen processing/presentation pathway.

In conclusion, the above data show that cancer cell lines with downregulated or dysregulated MHC Class I antigen processing and presentation pathways and/or downregulated or dysregulated cytokine signaling pathways are sensitive to treatment with ASC.

### EXAMPLE 14: FURTHER CHARACTERIZATION OF RESPONSIVE AND NON-RESPONSIVE BREAST CANCER CELL LINES

Next, the phenotypes of responsive and non-responsive breast cancer cell lines were determined, based on expression of 305 classifier genes useful for characterizing breast cancer lines as Luminal, Basal A, or Basal B by hierarchical clustering (Neve et al). The data from the paper was downloaded and reproduced (**Fig. 10**) using the GenePattern software tool (Reich et al). Pearson Correlation Clustering was used for distance measurements for both columns and rows, and the hierarchical clustering method was pairwise average-linkage.

**Fig. 11A** depicts the top of **Fig. 10****,** showing which cell lines are characterized, which includes 5/6 cell lines tested herein for ASC sensitivity. Of the depicted lines, HCC38, SUM149PT, MDA-MB-157, BT549, HSS78T, SUM159PT, MDA-MB-436, and MDA-MB-231 were tested for TRAIL sensitivity and are TN.

**Fig. 11A** also incorporates data from Rahman et al, which tested 20 breast cancer cell lines, including 11 triple negative (TN) lines, for TRAIL sensitivity; these are marked by plain asterisks (TRAIL-insensitive) and circled asterisks (TRAIL-sensitive). Most of the TN cell lines that are TRAIL-sensitive fall in the Basal B cluster, although there are 2 that fall outside it. All 8 TRAIL-sensitive TN cell lines that are Basal B have the "mesenchymal phenotype", and all 3 TRAIL-insensitive that are Basal A have the "epithelial phenotype."

The mesenchymal phenotype is defined as having high levels of Vimentin, high levels of caveolins, and low levels of E-cadherin. The epithelial phenotype is defined as having high levels of E-cadherin, abundant keratins, and low levels of Vimentin.

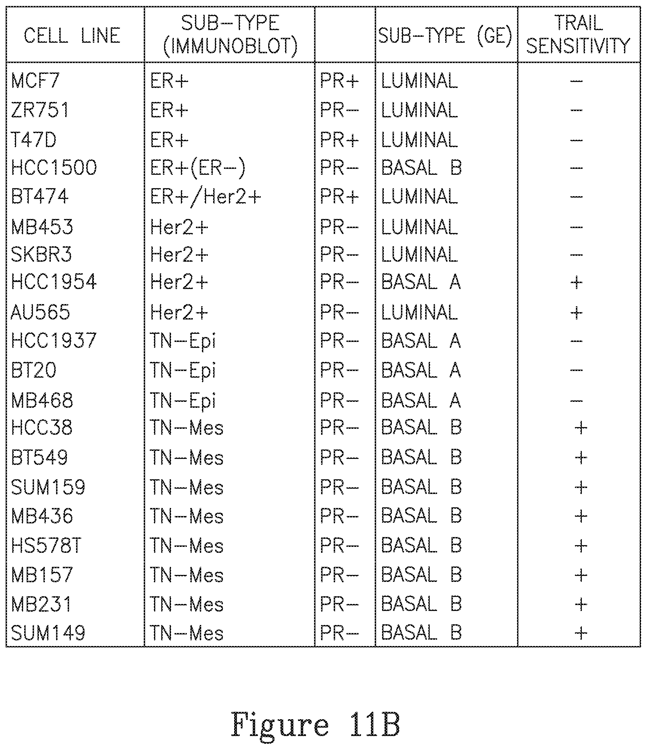

**Fig. 11B** depicts the data from tested breast cancer cell lines from **Fig. 11A** in tabular form, and also includes information on clinical sub-type, namely whether or not estrogen receptor (ER) or progesterone receptor (PR) is present, and whether Her2/neu is amplified.

In conclusion, TN breast tumors exhibit sensitivity to treatment with ASC.

HCC1395 is the only breast cancer line that was tested herein for ASC sensitivity and was not analyzed in the aforementioned hierarchical clustering analysis by Neve et al. This cell line was used to verify the hypothesis that sensitivity of breast tumors to ASC parallels a TN phenotype. Since HCC1395 was sensitive to ASC treatment, the aforementioned analysis predicts that the triple negative cell line HCC1395 is TRAIL-sensitive and falls into the Basal B cluster. This analysis required another dataset. The Cancer Cell Line Encyclopedia (CCLE; Barretina et al) was probed for breast cancer cell lines that were also in the hierarchical clustering analysis by Neve et al. Affymetrix gene expression data from 37 breast cancer cell lines was downloaded and processed as described hereinabove.

"Affy probes to gene" matches were used to identify genes in the Neve et al dataset, which were in turn used to select relevant gene expression data out of the 37 lines from the CCLE. This process yielded 169 probe sets to perform the hierarchical clustering, which yielded a similar clustering (**Fig. 12A**) to Neve et al. HCC-1395 clearly clustered together with MDA-MB-231 in the TN/Basal B group, thus verifying the hypothesis that sensitivity of breast tumors to ASC parallels TN phenotype.

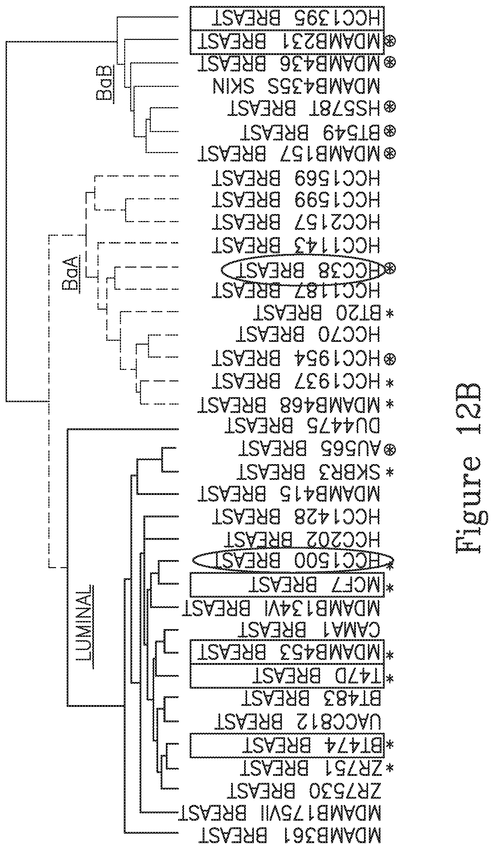

**Fig. 12B** shows the top of **Fig. 12A****.** Only 2 cell lines (circled) clustered differently than in the previous analysis. A virtually identical hierarchical clustering was obtained whether 18,000 probe sets (the number of probes in the gene expression data from the set of 37 CCLE cell lines) or 169 probe sets were used, thus verifying the clustering scheme (**Fig**. **12C**).

### EXAMPLE 15: GENES RESPONSIBLE FOR CLUSTERING OF BREAST TUMOR LINES ARE INVOLVED IN ANTIGEN PRESENTATIONAND IFN SIGNALING

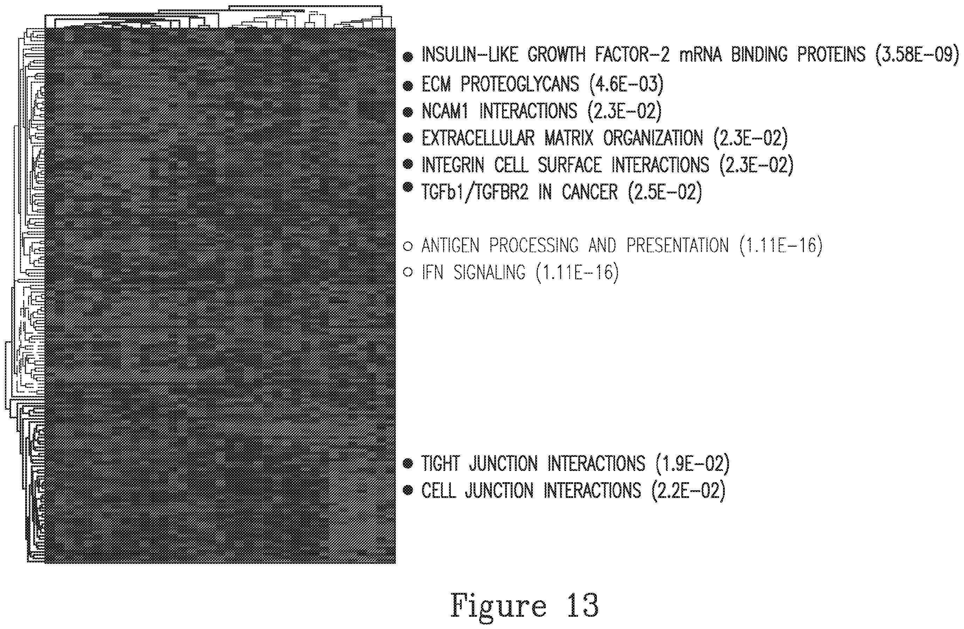

The genes identified in the aforementioned hierarchical clustering analysis by Neve *et al.* as responsible for clustering into Luminal, Basal A and Basal B were entered into the Reactome Pathway Database (each section individually) to identify pathways in which the classifier genes participate (**Fig. 13**). The middle rows section of classifier genes included HLAs and a few other antigen processing/presentation genes as well as IFN signaling pathway genes, thus further validating the aforementioned analyses and verifying that the previously-identified pathways apply to a wider spectrum of breast cancer cell lines than was originally tested by the ASC-TNFα/IFNγ.

### EXAMPLE 16: IN VIVO ANTI-TUMOR ACTIVITY OF ASC IN A TUMOR IMPLANTATION MODEL

### METHODS

93 athymic Foxn1^{nu} nude were injected (all groups) subcutaneously in the right flank with 3x10⁶ MDA-MB-231 adenocarcinoma cells in 0.2 ml PBS-this was considered day 0. The experimental groups are shown in **Table 21:**

**Table 21**

| Group num/name | Intervention | Route | Time of intervention |
|---|---|---|---|
| 1/Untreated control | None | - | - |
| 2/IM control | PlasmaLyte A | IM | Day 9 only |
| 3/IV control | PlasmaLyte A | IV | Day 9 only |
| 4/IM Late Treatment | 1x10⁶ TNF/IFN stimulated ASC | IM | Day 9 only |
| 5/IV Late Treatment | 1x10⁶ TNF/IFN stimulated ASC | IV | Day 9 only |
| 6/IM early/late treat. | 1x10⁶ TNF/IFN stimulated ASC | IM | Days 1 and 9 |
| 7/IV early/late treat. | 1x10⁶ TNF/IFN stimulated ASC | IV | Days 1 and 9 |

TNF-α + IFN-γ-stimulated ASC were suspended in a volume of 50 mcl (microliters) or 250 mcl for intramuscular (IM) or intravenous (IV) administration, respectively. On day 1, ASC were administered IM or IV, to 10 mice each in Groups 6 and 7, respectively. On day 9, 23 mice with tumor sizes outside the range of 36-88mm³ were removed from the 73 untreated mice (previously referred to as the control group), leaving 50 untreated mice. The 50 animals were assigned randomly to Groups 1, 2, 3, 4 and 5. Randomization was performed according to the size of the tumor such that each group ended up with mice having tumors approximately the same average size. Thus there were ultimately 10 mice in each group. On the same day, mice were administered either no treatment, mock IM or IV injection (Groups 1, 2, and 3, respectively); or ASC administered IM (Groups 4 and 6, receiving a first or additional treatment, respectively) or IV (Groups 5 and 7, receiving a first or additional treatment, respectively), as indicated in Table 21.

Tumor volume was measured using electronic calipers.

### RESULTS

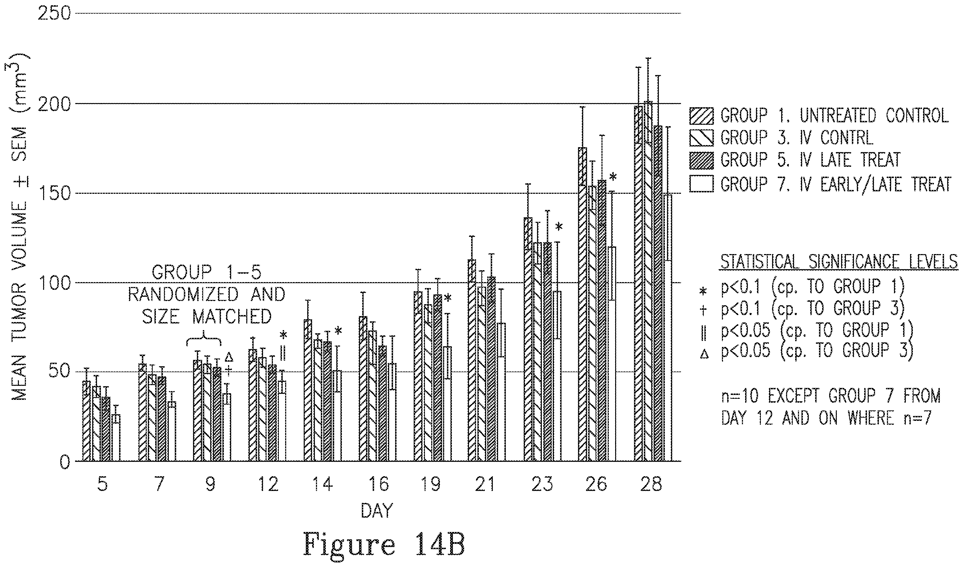

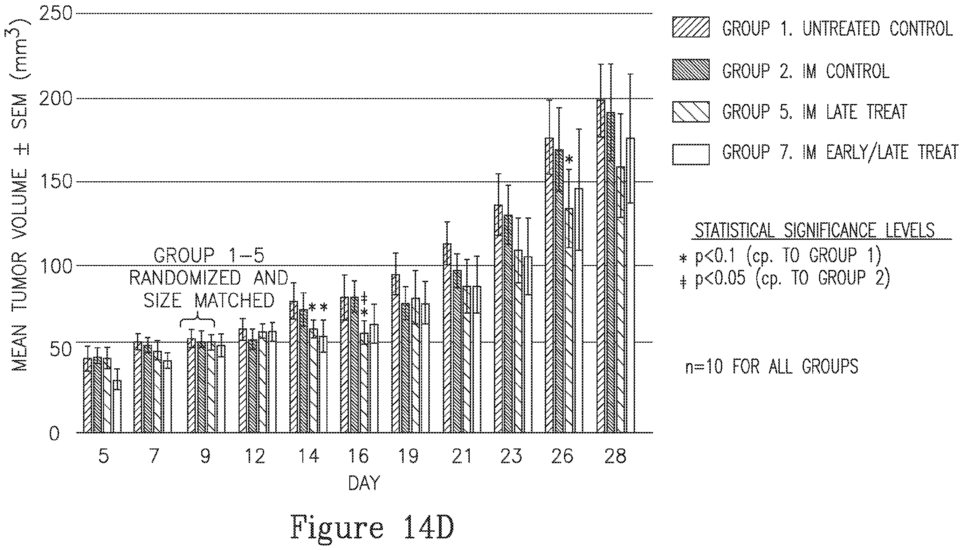

Anti-tumor effects of ASC were tested in an *in vivo* tumor implantation model. Mice receiving ASC administration on day 1 exhibited reduced tumor size. The inhibition was statistically significant when IV was compared to controls at each time point, namely at days 5, 7, and 9 (p=0.0055, 0.0067, and 0.041 by one-tailed t-test, respectively) (**Fig. 14A**). Trends of efficacy were seen in both IV-injected (**Figs. 14B-C**) and IM-injected (**Figs. 14D-E**) mice. The inhibitory effect was strongly seen when observing the fold change in tumor volume from days 12-16 (**Tables 22-23**); days 9-28 (**Table 24**); and days 9-16 (**Table 25**).

**Table 22. Fold Change in Tumor Volume from Day 12-16.**

| | Untreated Controls (Group 1) | IM Control (Group 2) | IM ASC (Late) (Group 4) | IM ASC (Early/Late) (Group 6) |
|---|---|---|---|---|
| Mean | 1.33 | 1.52 | 1.01 | 1.06 |
| Standard Deviation (SD) | 0.53 | 0.47 | 0.39 | 0.35 |
| Minimum | 0.32 | 1.00 | 0.50 | 0.52 |
| 1 st Quartile | 1.03 | 1.07 | 0.84 | 0.87 |
| 2nd Quartile (Median) | 1.34 | 1.48 | 1.00 | 1.02 |
| 3rd Quartile | 1.55 | 1.91 | 1.00 | 1.13 |
| Maximum | 2.30 | 2.31 | 1.89 | 1.75 |
| Number of Mice | 10 | 10 | 10 | 10 |

**Table 23. Fold Change in Tumor Volume from Day 12-16.**

| | Percentage of mice in each group | |
|---|---|---|
| Fold Change | IM ASC (Groups 4/6) | Control (Groups 1/2) |
| 0-0.5 | 0 | 5 |
| 0.5-1 | 65 | 25 |
| 1-1.5 | 25 | 25 |
| 1.5-2 | 10 | 30 |
| 2-2.5 | 0 | 15 |

**Table 24. Fold Change in Tumor Volume from Day 9-28. Note the lack of control mice with fold change of 0 or 0-1, in contrast to the 8% ASC-treated mice in these groups.**

| | Percentage of mice in each group | | | |
|---|---|---|---|---|
| Fold Change | Control Mice (Groups 1-3) | All ASC (Groups 4-7) | All IM ASC (Groups 4/6) | All IV ASC (Groups 5/7) |
| 0 | 0 | 3 | 0 | 6 |
| 0-1 | 0 | 5 | 10 | 0 |
| 1-2 | 13 | 14 | 15 | 12 |
| 2-3 | 30 | 27 | 20 | 35 |
| 3-4 | 13 | 19 | 25 | 12 |
| 4-5 | 30 | 16 | 10 | 24 |
| 5-6 | 3 | 5 | 10 | 0 |
| 6-7 | 7 | 5 | 10 | 0 |
| 7-8 | 0 | 3 | 0 | 6 |
| 8-9 | 3 | 3 | 0 | 6 |

**Table 25. Fold Change in Tumor Volume from Day 9-28. Note the increased number of ASC-treated mice with a fold change of < 1.5 vs. the control mice.**

| | Percentage of mice in each group | | | | | |
|---|---|---|---|---|---|---|
| Fold Change | All ASC (Gr. 4-7) | IM ASC (Gr. 4/6) | IV ASC (Gr. 5/7) | All Control (Gr. 1-3) | IM Controls (Gr. 1/2) | IV Controls (Gr. 1/3) |
| 0 | 3 | 0 | 6 | 0 | 0 | 0 |
| 0-0.5 | 0 | 0 | 0 | 3 | 5 | 5 |
| 0.5-1 | 32 | 35 | 29 | 23 | 30 | 15 |
| 1-1.5 | 41 | 35 | 47 | 33 | 15 | 45 |
| 1.5-2 | 11 | 20 | 0 | 23 | 25 | 20 |
| 2-2.5 | 11 | 10 | 12 | 13 | 20 | 10 |
| 2.5-3 | 3 | 0 | 6 | 3 | 5 | 5 |

These data confirm that ASC inhibit tumor growth *in vivo,* even in a very rapidly-growing tumor model.

### EXAMPLE 17: TESTING OF ASC IN AN ADDITIONAL IN VIVO TUMOR IMPLANTATION MODEL

### OVERVIEW

Another tumor implantation model was used to study the anti-tumor effect of ASC. In this case, the tumor cells were injected into the inguinal mammary fat pad. Subsequently, 0.5 or 1.0 x 10⁶ ASC, or PlasmaLyte (negative control; "Lyte") were injected intramuscularly (IM), intravenously (IV), or alternating IM and IV ("IV/IM").

### METHODS

3 x 10⁶ MDA-MB-231 cells were injected into the 4th (inguinal) mammary fat pad, designated Day 0, except for Group 9, which received no treatment. **Table 26** sets forth the treatments received by all groups. 3 mice died during the study, 2 in Group 5 on week 8 and 1 in Group 7 on week 11. No significant effects of ASC administration were seen on body mass.

**Table 26, Part I**

| Group num. | Group name | Number of mice | Substance injected | Route | Injection schedule |
|---|---|---|---|---|---|
| 1, 3 | IM-ASC | 20 | ASC | IM | See Part II |
| 2 | IM-PlasmaLyte | 10 | PlasmaLyte | IM | |
| 4 | IM-ASC/late | 10 | ASC | IM | |
| 5 | IV-ASC | 10 | ASC | IV | |
| 6 | IV-PlasmaLyte | 10 | PlasmaLyte | IV | |
| 7 | IV/IM-ASC | 10 | ASC | IV/IM | |
| 8 | IV/IM-PlasmaLyte | 10 | PlasmaLyte | IV/IM | |
| 9 | Naïve | 10 | - | - | |

**Table 26, Part II ("Lyte" refers to PlasmaLyte")**

| Week | 1 | 2 | 3 | 4 | 5 | 6 | 7 | 8 | 9 | 10 | 11 | 12 |
|---|---|---|---|---|---|---|---|---|---|---|---|---|
| Group no. | | | | | | | | | | | | |
| 1, 3 | IM-ASC | | | | | | | | | | | |
| 2 | IM-PlasmaLyte | | | | | | | | | | | |
| 4 | IM-PlasmaLyte | | | | | | IM-ASC | | | | | |
| 5 | IV-ASC | | | | | | | | - | IV-ASC | | - |
| 6 | IV-PlasmaLyte | | | | | | | | - | IV-Lyte | | - |
| 7 | IV-ASC | IM-ASC | IV-ASC | IM-ASC | IV-ASC | IM-ASC | IV-ASC | IM-ASC | IV-ASC | IM-ASC | IV-ASC | IM-ASC |
| 8 | IV-Lyte | IM-Lyte | IV-Lyte | IM-Lyte | IV-Lyte | IM-Lyte | IV-Lyte | IM-Lyte | IV-Lyte | IM-Lyte | IV-Lyte | IM-Lyte |
| 9 | - | | | | | | | | | | | |
| No. cells | 0.5 x 10⁶ | | | | | | 1.0 x 10⁶ | | | | | |

*Histology:* Tissue samples included xenograft tumors, lungs and lymph nodes (axillary, inguinal and lumbar). Tissue samples were immersion-fixed in buffered formalin and processed for paraffin embedding. Samples were processed as follows:
1. Xenograft tumors. Paraffin blocks of tumor samples were prepared so that standard five micrometers sections through the middle of the tumor could be prepared. Tumor sections were stained with hematoxylin-eosin for general histopathological assessment.
   1.1 Tumor cell proliferation. Tumor sections were processed for immunohistochemical (IHC) staining with antibodies to Ki67 antigen. Sections were deparaffinized and subjected to heat-induced antigen retrieval (HIER) by boiling for 20 min in 0.05% citraconic anhydride (CA; Aldrich; Cat# 125318) solution, pH 7.4. After cooling to room temperature (rt), sections were incubated for 1 hr at rt with rabbit monoclonal antibody to Ki67 (clone SP6; Abcam Cat#ab16667) diluted 1:200 in TBST (10mM Tris-HCl, 150 mM NaCl, 0.1% Tween-20, pH 7.5). Sections were washed in TBST and incubated for 30 min with horseradish peroxidase (HRP)-labeled anti-rabbit IgG polymeric reagent (ZytoCem Plus HRP-polymer anti-Rabbit; ZytoMed, Cat#ZUC032-10). Then sections were washed, and HRP activity was assayed by incubation for 3 min in TIBS buffer (0.15 M NaCl, 10 mM Tris, 5 mM imidazole; pH 7.5) containing 0.05% diaminobenzidine (DABx4HCl [Sigma; Cat#32750]), and 0.015% hydrogen peroxide. Slides were lightly counterstained with hematoxylin, coverslipped using DPX mountant (Sigma; Cat#06522), and photographed using an Olympus BX-50 microscope equipped with a QuantiFire XI CCD camera (Optronics) coupled with an RGB tunable imaging filter (CRI). 3-5 non-overlapping images were obtained per tumor sample with a 20x objective.
      Using ImageJ, images were subjected to background subtraction and color deconvolution, resulting in separation of the image into blue and brown monochrome images, showing all nuclei and Ki67-immunostained nuclei, respectively. Images were then binarized by automatic thresholding, and nuclei were separated using the "watershed" command and automatically counted by using the "analyze particles" command. The percentage of the Ki67 labeled nuclei in all images related to the same sample was used as the proliferation index.
   1.2 Tumor cell apoptosis. For detection of apoptotic cells, tumor sections were immunostained with anti-active caspase antibody. Sections were deparaffinized and subjected to HIER by boiling in 10 mM citric buffer (pH 6.0) for 20 min. After cooling for 1 hr at rt, sections were incubated for 1 hr at rt with rabbit monoclonal antibody to active caspase 3 (clone E83-77; Abcam Cat#ab32042) diluted in 1:1000 in TBST, and incubation, detection, and counterstaining were performed as described above for the anti-Ki67 antibody. To quantify apoptosis, sections of immunostained cells were manually counted in 6-10 high power (objective x40) microscopic fields.
   1.3. Tumor vascularization. To quantify tumor vascularization, sections were immunostained with rabbit monoclonal antibody to mouse CD34 (clone EP373Y, Abcam; Cat#ab81289; diluted 1:1000) to visualize endothelial cells. Immunostaining was performed as described above for anti-active caspase. The zone of the highest density of CD34⁺ (endothelial) cells ("hot spot") was photographed using a microscopic objective x20 to obtain a color digital image of 2048x2048 pixels. Using ImageJ, the area (number of pixels) stained brown with DAB was determined after color deconvolution, automatic thresholding and binarization. The percentage of the immunostained area was calculated in relation to the image size (4.19 Mpi).
2. Lung metastases. Paraffin blocks with lung specimens were subjected to exhaustive systematic sectioning, yielding sections separated by 300 µm (8-20 per lung sample), which were mounted onto glass slides. IHC staining with rabbit monoclonal anti-cytokeratin 18 (CK18) antibody (clone EPR1626; Abcam, Cat#ab133263) diluted 1:600 was used for the detection of tumor cells in lung sections subjected to HIER in TE buffer (pH 8.0). The rest of the IHC procedure was as described above.
3. Lymph node metastases. Each pair of lymph nodes (axillary, inguinal and lumbar) was embedded into one paraffin block, which was subjected to exhaustive systematic sectioning to obtain sections separated by 75 µm (8-25 per lymph node sample). Sections were mounted onto glass slides and immunostained with anti-CK18 antibody as described for lung sections.

*P-values* were calculated using Student's T-Tests, with two-tailed analyses.

### RESULTS

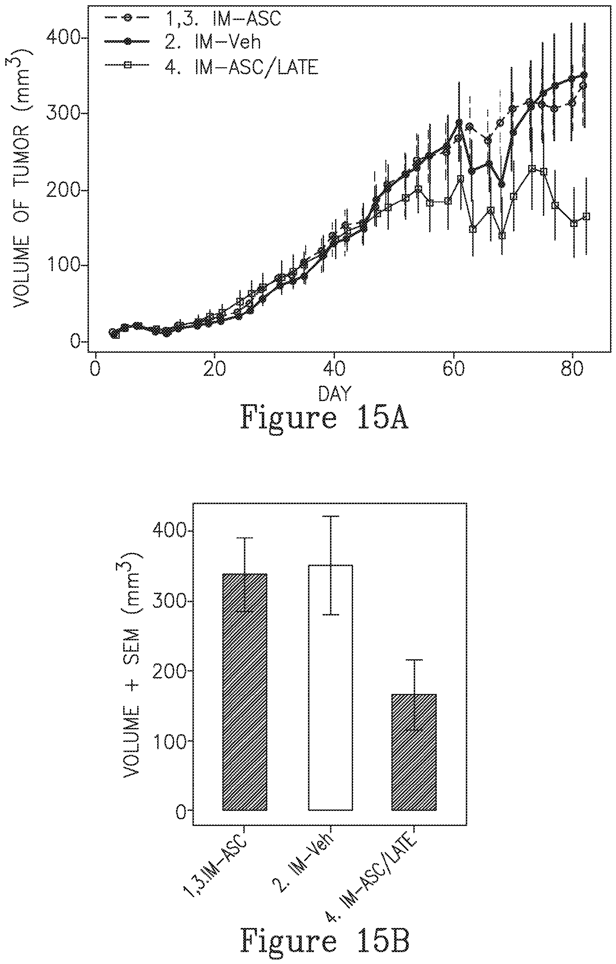

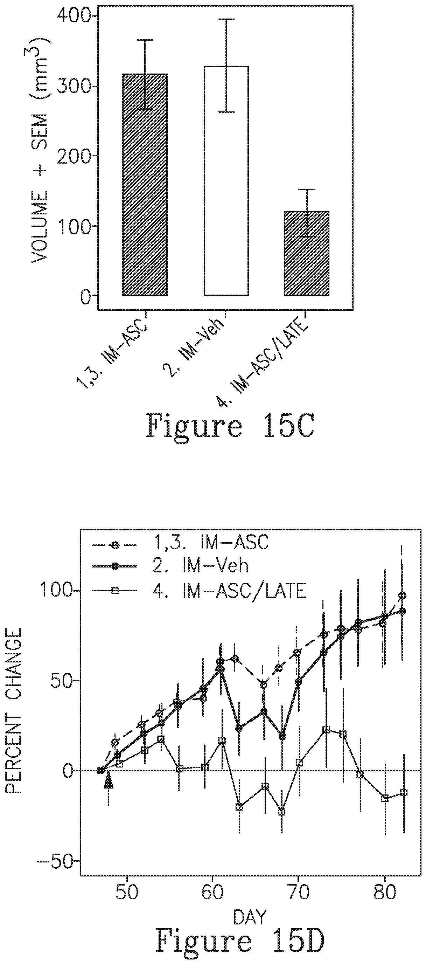

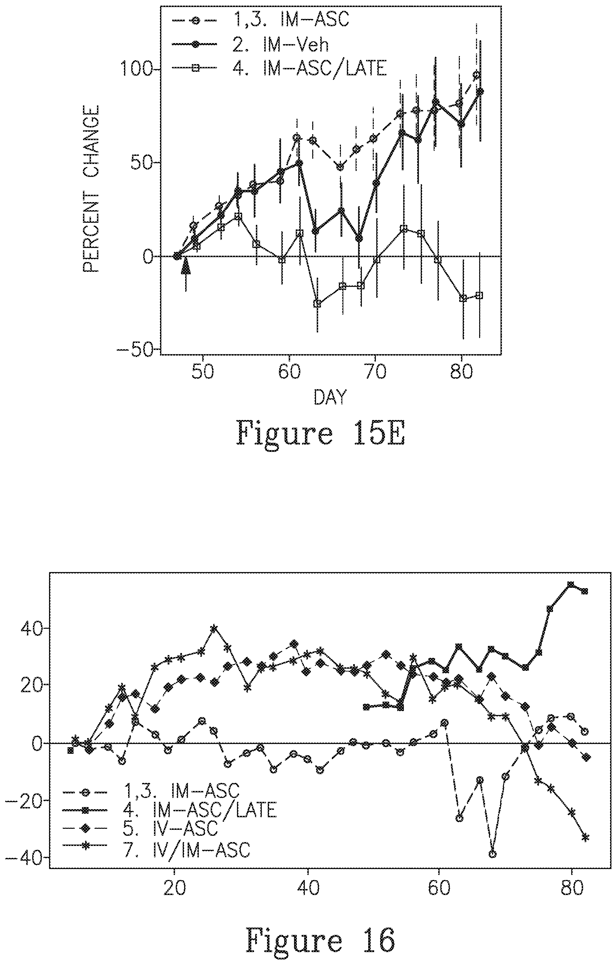

Late IM treatment with ASC (beginning at day 48) significantly slowed or halted tumor growth, which was apparent whether plotting mean tumor volume (**Figs. 15A-C**) or percent change in tumor volume from day 47 (**Figs. 15D-E**). In **Figs. 15C** and **E****,** outliers were removed to generate "trimmed" numbers. 30% of the mice in Group 4 exhibited complete remission by Day 84, compared to 0% in Group 2 and 5% in Groups 1 and 3.

IV-ASC-treatment initially inhibited tumor growth (**Fig. 16**), specifically a 34% or 29% inhibition of growth at day 38 when considering unmanipulated or trimmed means (**Figs**. **17A-B** **and** **C-D****,** respectively). Growth inhibition was not detected at later time points. However, tumors in the PlasmaLyte-treated group stopped growing after approximately day 55 (**Figs**. **17A** and **C**), which may have artefactually masked a lasting inhibition of tumor growth by IV-ASC treatment. Alternating IV/IM-ASC treatment yielded similar results to IV treatment.

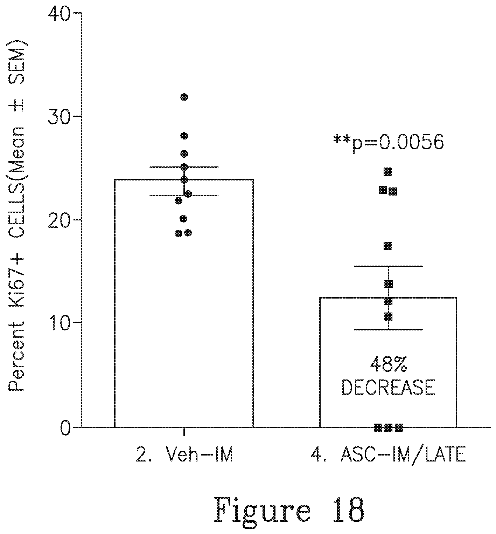

Histological analyses were performed on groups 2, 4, 5, and 6. Late IM treatment induced a statistically significant 48% decrease (p = 0.0056) in proliferating cells within the tumor (**Fig. 18**). No effect on proliferation was found in IV-treated mice.

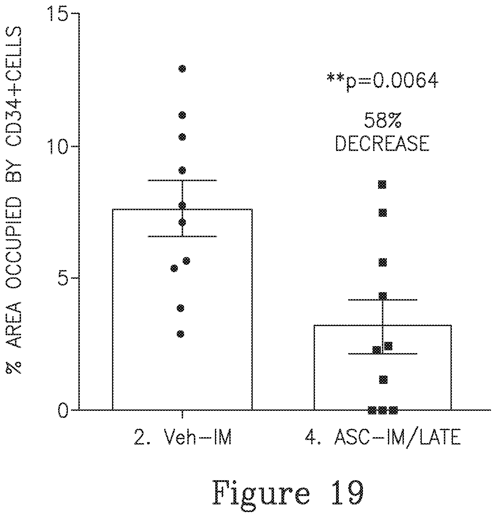

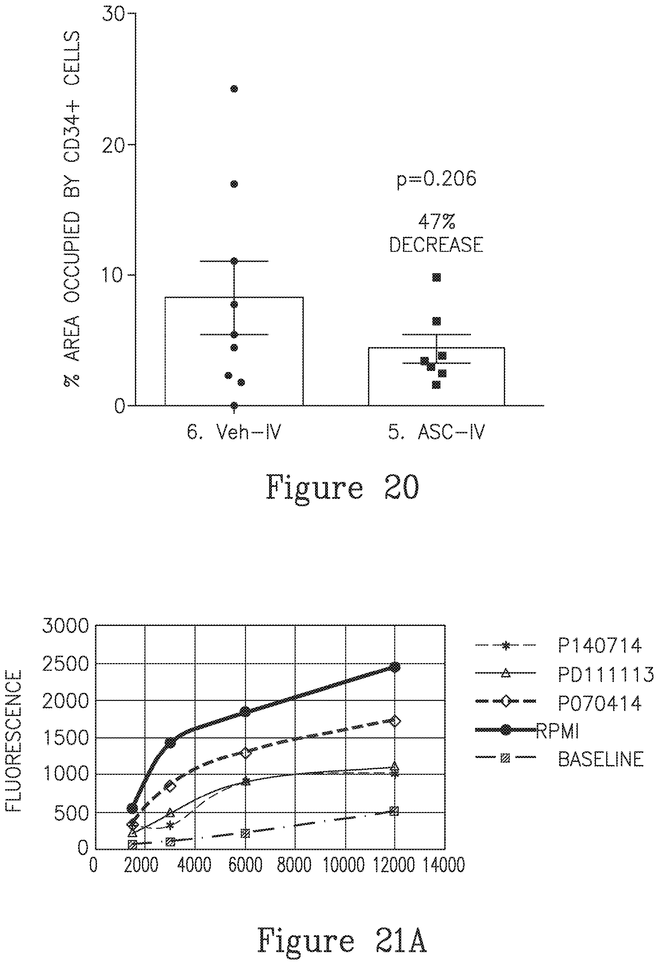

Late IM treatment also induced a statistically significant 58% decrease (p = 0.0064) in vascularization of the tumor (**Fig. 19**). IV-treated mice also showed a trend towards decreased vascularization (**Fig. 20**). Neither effect was correlated with tumor mass or proliferation, indicating an independent effect on vascularization. These data indicate that there are at least two separate MOAs that work together to inhibit tumor growth when pre-existing tumors are treated with ASC-IM.

IV-treated mice also exhibited a 15% increase in apoptosis within the tumor (p = 0.064), while no effect was seen with IM-treated mice.

Moreover, a complete absence of lung metastases was observed in the late-IM and IV-treated mice, while both control groups had them (**Table 27**). Axillary, inguinal, and lumbar lymph nodes were also observed for metastases. Late-IM-treated mice and IV-treated mice exhibited trends towards decreased axillary and lumbar metastases, respectively (**Table 28**).

**Table 27. Lung metastases.**

| Group | Percentage of mice with metastases | No. of metastases |
|---|---|---|
| 2 | 20 | 6 |
| 4 | 0 | 0 |
| 6 | 20 | 3 |
| 5 | 0 | 0 |

**Table 28. Lymph node metastases. LN denotes lymph node; LG denotes lung.**

| **Veh-IM** | | | **ASC-Late-IM** | | | **Veh-IV** | | | **ASC-IV** | | |
|---|---|---|---|---|---|---|---|---|---|---|---|
| tag | LN Met | LG Met | tag | LN Met | LG Met | tag | LN Met | LG Met | tag | LN Met | LG Met |
| 202 | lumbar | - | 224 | - | - | 206 | inguinal | - | 207 | - | - |
| 225 | - | - | 246 | - | - | 237 | axillary, lumbar, inguinal | √ | 210 | - | - |
| 236 | axillary lumbar | √ | 255 | - | - | 242 | - | - | 231 | - | - |
| 238 | axillary lumbar | √ | 268 | - | - | 250 | axillary | - | 235 | axillary | - |
| 266 | - | - | 269 | lumbar | - | 252 | - | - | 253 | axillary inguinal | - |
| 271 | - | - | 291 | axillary | - | 262 | - | - | 284 | - | - |
| 273 | - | - | 292 | lumbar | - | 283 | - | - | 300 | inguinal | - |
| 290 | - | - | 298 | lumbar | - | 296 | axillary, lumbar | √ | 409 | inguinal | - |
| 297 | axillary lumbar | - | 401 | lumbar | - | 402 | - | - | | | |
| 405 | - | - | 412 | - | - | 411 | - | - | | | |

### EXAMPLE 18: FURTHER IN-VITRO CONFIRMATION OF TUMOR CELL GROWTH INHIBITION BYASC-CM

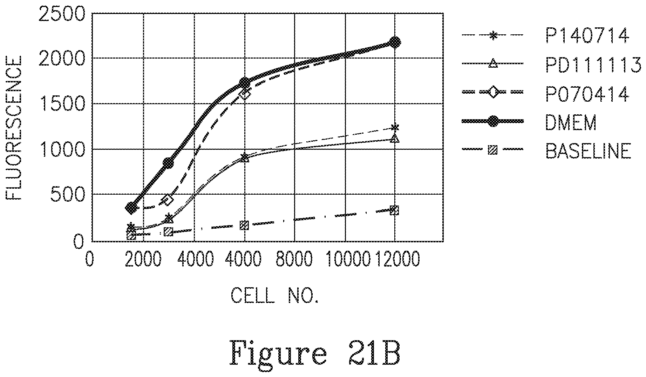

Three batches of ASC, prepared as described in Example 6 or Example 9, were tested. 500,000 ASC were incubated for 24 hr in cancer cell growth medium (RPMI or hi-glucose DMEM, as appropriate, without serum) to generate CM. Three cancer cell lines (NCI-H460 and MDA-MB-231, which are TRAIL sensitive; and MCF7, which is TRAIL insensitive) were seeded in 48 well-plates at initial densities of 1500, 3000, 6000 or 12000 cells/well. The following day, cells were washed and exposed to ASC-CM, after adding 5% FBS and 1% Glutamine to the CM to avoid non-specific growth inhibition via medium consumption, or were exposed to regular growth medium + 5% FBS, which was used as a control. After 3 days' incubation in ASC-CM or medium, cells were washed and frozen. Baseline plates seeded at each density were washed and frozen 1 day after cell seeding, then later processed in parallel with the other samples. Viable cells were quantified using CyQUANT GR. ASC-CM inhibited growth of NCI-H460 **(****Fig. 21A****)** and MDA-MB-231 **(****Fig. 21B****).**

### EXAMPLE 19: IN-VIVO TESTING OF ASC IN A METASTASES INHIBITION MODEL

Luciferase-labeled MDA-MB-231 human breast tumor cells (Yang *et al*) are injected into the mammary fat pad of NOD-SCID immunodeficient mice, and the mice are treated with ASC, given IV and IM, as described in the previous Example. The colonization of MDA-MB-231 tumor cells in the lung is monitored with an IVIS imaging system (PerkinElmer, American Fork, Utah).

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. In addition, citation or identification of any reference in this application shall not be construed as an admission that such reference is available as prior art to the invention.

### REFERENCES (Additional References are cited in the text)

Barretina, J. et al. (2012). "The Cancer Cell Line Encyclopedia enables predictive modelling of anticancer drug sensitivity." Nature 483(7391): 603-607.
Bonnomet A et al, A dynamic in vivo model of epithelial-to-mesenchymal transitions in circulating tumor cells and metastases of breast cancer. Oncogene. 2012 Aug 16;31(33):3741-53.
Clayton A et al, Analysis of antigen presenting cell derived exosomes, based on immuno-magnetic isolation and flow cytometry. J Immunol Methods. 2001;247(1-2):163-74.
Crescitelli R et al, Distinct RNA profiles in subpopulations of extracellular vesicles: apoptotic bodies, microvesicles and exosomes. J Extracell Vesicles. 2013 Sep 12;2.
Croft, D., et al. (2014). "The Reactome pathway knowledgebase." Nucleic Acids Res 42(Database issue): D472-477.
Dominici et al. Minimal criteria for defining multipotent mesenchymal stromal cells. The International Society for Cellular Therapy position statement. Cytotherapy. 2006; 8(4):315-7.
Forbes, S. A., et al. (2015). "COSMIC: exploring the world's knowledge of somatic mutations in human cancer." Nucleic Acids Research 43(D1): D805-D811
Friedrich J et al, Spheroid-based drug screen: considerations and practical approach. Nature Protocols 4(3): 309-324, 2009.
Ivascu A et al. Rapid generation of single-tumor spheroids for highthroughput cell function and toxicity analysis. J. Biomol. Screen 11: 922-932 (2006).
James MA et al, A novel, soluble compound, C25, sensitizes to TRAIL-induced apoptosis through upregulation of DR5 expression. Anticancer Drugs. 2015 Jun;26(5):518-30.
Jones LM et al, STAT3 Establishes an Immunosuppressive Microenvironment during the Early Stages of Breast Carcinogenesis to Promote Tumor Growth and Metastasis. Cancer Res. 2016 Mar 15;76(6):1416-28.
Kobayashi H et al, Lymphatic drainage imaging of breast cancer in mice by micro-magnetic resonance lymphangiography using a nano-size paramagnetic contrast agent. J Natl Cancer Inst. 2004 May 5;96(9):703-8.
Kobayashi K et al, Cytotoxic effects of benzbromarone and its 1'-hydroxy metabolite in human hepatocarcinoma FLC4 cells cultured on micro-space cell culture plates. Drug Metab Pharmacokinet. 2013;28(3):265-8.
Korff T et al. Integration of endothelial cells in multicellular spheroids prevents apoptosis and induces differentiation. J. Cell Biol. 143: 1341-1352 (1998).
Lyons AB. Analysing cell division in vivo and in vitro using flow cytometric measurement of CFSE dye dilution. J Immunol Methods. 2000 Sep 21;243(1-2):147-54.
Mathias RA et al, Isolation of extracellular membranous vesicles for proteomic analysis. Methods Mol Biol. 2009;528:227-42.
Morales P et al, Selective, nontoxic CB(2) cannabinoid o-quinone with in vivo activity against triple-negative breast cancer. J Med Chem. 2015 Mar 12;58(5):2256-64. doi: 10.1021/acs.jmedchem.5b00078. Epub 2015 Feb 20.
Morton JJ et al, Humanized Mouse Xenograft Models: Narrowing the Tumor-Microenvironment Gap. Cancer Res. 2016 Nov 1;76(21):6153-6158. Epub 2016 Sep 1.
Nakamura et al., Evaluation of drug toxicity with hepatocytes cultured in a micro-space cell culture system. J Biosci Bioeng. 2011 Jan;111(1):78-84.
Neve et al. "A Collection of Breast Cancer Cell Lines for the Study of Functionally Distinct Cancer Subtypes." Cancer cell 10.6 (2006): 515-527
Oh WK, Neoadjuvant therapy before radical prostatectomy in high-risk localized prostate cancer: defining appropriate endpoints. Urol Oncol. 2003 May-Jun;21(3):229-34.
Perche F et al, Cancer cell spheroids as a model to evaluate chemotherapy protocols. Cancer Biology & Therapy 13:12, 1205-1213, 2012.
Phung YT et al, Rapid Generation of In Vitro Multicellular Spheroids for the Study of Monoclonal Antibody Therapy, J Canc 2: 507-514, 2011.
Rahman et al. "TRAIL Induces Apoptosis in Triple-Negative Breast Cancer Cells with a Mesenchymal Phenotype." Breast cancer research and treatment 113.2 (2009): 217-230.
Ramsey SD et al, Integrating comparative effectiveness design elements and endpoints into a phase III, randomized clinical trial (SWOG S1007) evaluating oncotypeDX-guided management for women with breast cancer involving lymph nodes. Contemp Clin Trials. 2013 Jan;34(1):1-9.
Reich, M., et al. (2006). "GenePattern 2.0." Nat Genet 38(5): 500-501.
Rocha NS et al, (2002) Effects of fasting and intermittent fasting on rat hepatocarcinogeneis induced by diethylnitrosamine. Teratog Carcinog Mutagen. 22(2): 129-138.
Roux S et al, CD4+CD25+ Tregs control the TRAIL-dependent cytotoxicity of tumor-infiltrating DCs in rodent models of colon cancer. J Clin Invest. 2008 Nov;118(11):3751-61.
Ruggeri BA et al, Animal models of disease: pre-clinical animal models of cancer and their applications and utility in drug discovery. Biochem Pharmacol. 2014 Jan 1;87(1):150-61.
Walker JD et al, Oncolytic herpes simplex virus 1 encoding 15-prostaglandin dehydrogenase mitigates immune suppression and reduces ectopic primary and metastatic breast cancer in mice. J Virol. 2011 Jul;85(14):7363-71.
Yang S et al, Mouse models for tumor metastasis. Methods Mol Biol. 2012;928:221-8.
Zhang CH et al, Design, Synthesis, and Structure-Activity Relationship Studies of 3-(Phenylethynyl)-1H-pyrazolo[3,4-d]pyrimidin-4-amine Derivatives as a New Class of Src Inhibitors with Potent Activities in Models of Triple Negative Breast Cancer. J Med Chem. 2015 May 14;58(9):3957-74. Epub 2015 Apr 16.
Zhang F et al, ING5 inhibits cancer aggressiveness via preventing EMT and is a potential prognostic biomarker for lung cancer. Oncotarget. 2015 Jun 30;6(18):16239-52.
Zhang Y et al, Real-Time GFP Intravital Imaging of the Differences in Cellular and Angiogenic Behavior of Subcutaneous and Orthotopic Nude-Mouse Models of Human PC-3 Prostate Cancer. J Cell Biochem. 2016 Nov;117(11):2546-51.

## Claims

1. Adherent stromal cells (ASC) for use in a method of treating a breast carcinoma, wherein said ASC originate from placenta tissue.

2. The ASC for the use of claim 1, wherein said ASC have been obtained from a three-dimensional (3D) culture.

3. The ASC for the use of claim 2, wherein said 3D culture utilizes a medium whose composition is not varied over the course of said 3D culture.

4. The ASC for the use of claim 2, wherein one or more pro-inflammatory cytokines is added to an incubation medium of said 3D culture.

5. The ASC for the use of claim 4, wherein said 3D culture comprises: (a) incubating ASC in a 3D culture apparatus in a first growth medium, wherein no inflammatory cytokines have been added to said first growth medium; and (b) subsequently incubating said ASC in a 3D culture apparatus in a second growth medium, wherein said one or more pro-inflammatory cytokines have been added to said second growth medium.

6. The ASC for the use of claim 4, wherein said one or more pro-inflammatory cytokines comprise Tumor Necrosis Factor alpha (TNF-alpha) or Interferon-Gamma (IFN-gamma).

7. The ASC for the use of any of claims 2-6, wherein said 3D culture is performed in an apparatus that comprises a 3D bioreactor.

8. The ASC for the use of any of claims 2-7, wherein said 3D culture is performed in an apparatus that comprises a fibrous bed matrix, wherein said fibrous bed matrix comprises a synthetic adherent material.

9. The ASC for the use of any of claims 2-8, wherein said ASC have been incubated in a 2D adherent-cell culture apparatus prior to said 3D culture.

10. The ASC for the use of any of claims 1-9, wherein said ASC are at least predominantly maternal cells.

11. The ASC for the use of any of claims 1-9, wherein said ASC are at least predominantly fetal cells.

12. The ASC for the use of any one of claims 1-11, wherein said breast carcinoma is triple negative.

13. The ASC for the use of any of claims 1-12, wherein said ASC are administered systemically.

14. The ASC for the use of any of claims 1-12, wherein said ASC are administered intratumorally.

## Patentansprüche

1. Adhärente Stromazellen (ASC) zur Verwendung in einem Verfahren zur Behandlung eines Brustkarzinoms, wobei die ASC von Plazentagewebe stammen.

2. ASC für die Verwendung nach Anspruch 1, wobei die ASC von einer dreidimensionalen (3D-)Kultur erhalten wurden.

3. ASC für die Verwendung nach Anspruch 2, wobei die 3D-Kultur ein Medium verwendet, dessen Zusammensetzung im Lauf der 3D-Kultur nicht variiert.

4. ASC für die Verwendung nach Anspruch 2, wobei ein oder mehrere proentzündliche Zytokine einem Inkubationsmedium der 3D-Kultur zugegeben werden.

5. ASC für die Verwendung nach Anspruch 4, wobei die 3D-Kultur umfasst: (a) Inkubieren von ASC in einem 3D-Kulturgerät in einem ersten Wachstumsmedium, wobei keine entzündlichen Zytokine dem ersten Wachstumsmedium zugegeben wurden; und (b) anschließend Inkubieren der ASC in einem 3D-Kulturgerät in einem zweiten Wachstumsmedium, wobei das eine oder die mehreren proentzündlichen Zytokine dem zweiten Wachstumsmedium zugegeben wurden.

6. ASC für die Verwendung nach Anspruch 4, wobei das eine oder die mehreren proentzündlichen Zytokine Tumornekrosefaktor Alpha (TNF-Alpha) oder Interferon-Gamma (IFN-Gamma) umfassen.

7. ASC für die Verwendung nach einem der Ansprüche 2-6, wobei die 3D-Kultur in einem Gerät durchgeführt wird, das einen 3D-Bioreaktor umfasst.

8. ASC für die Verwendung nach einem der Ansprüche 2-7, wobei die 3D-Kultur in einem Gerät durchgeführt wird, das eine Faserbettmatrix umfasst, wobei die Faserbettmatrix ein synthetisches adhärentes Material umfasst.

9. ASC für die Verwendung nach einem der Ansprüche 2-8, wobei die ASC vor der 3D-Kultur in einem 2D-, adhärenten Zellkulturgerät inkubiert wurden.

10. ASC für die Verwendung nach einem der Ansprüche 1-9, wobei die ASC mindestens vorwiegend mütterliche Zellen sind.

11. ASC für die Verwendung nach einem der Ansprüche 1-9, wobei die ASC mindestens vorwiegend fötale Zellen sind.

12. ASC für die Verwendung nach einem der Ansprüche 1-11, wobei das Brustkarzinom dreifach negativ ist.

13. ASC für die Verwendung nach einem der Ansprüche 1-12, wobei die ASC systemisch verabreicht werden.

14. ASC für die Verwendung nach einem der Ansprüche 1-12, wobei die ASC intratumoral verabreicht werden.

## Revendications

1. Cellules stromales adhérentes (ASC) destinées à être utilisées dans un procédé de traitement d'un carcinome du sein, dans lesquelles lesdites ASC proviennent d'un tissu placentaire.

2. ASC destinées à être utilisées selon la revendication 1, dans lesquelles lesdites ASC ont été obtenues à partir d'une culture tri-dimensionnelle (3D).

3. ASC destinées à être utilisées selon la revendication 2, dans lesquelles ladite culture 3D utilise un milieu dont la composition ne varie pas au cours de ladite culture 3D.

4. ASC destinées à être utilisées selon la revendication 2, dans lesquelles une ou plusieurs cytokines pro-inflammatoires sont ajoutées à un milieu d'incubation de ladite culture 3D.

5. ASC destinées à être utilisées selon la revendication 4, dans lesquelles ladite culture 3D comprend : (a) l'incubation des ASC dans un appareil de culture 3D dans un premier milieu de croissance, des cytokines inflammatoires n'ayant pas été ajoutées audit premier milieu de croissance ; et (b) ensuite l'incubation desdites ASC dans un appareil de culture 3D dans un second milieu de croissance, lesdites une ou plusieurs cytokines pro-inflammatoires ayant été ajoutées audit second milieu de croissance.

6. ASC destinées à être utilisées selon la revendication 4, dans lesquelles lesdites une ou plusieurs cytokines pro-inflammatoires comprennent le facteur de nécrose tumorale alpha (TNF-alpha) ou l'interféron gamma (IFN-gamma).

7. ASC destinées à être utilisées selon l'une quelconque des revendications 2 à 6, dans lesquelles ladite culture 3D est effectuée dans un appareil qui comprend un bioréacteur 3D.

8. ASC destinées à être utilisées selon l'une quelconque des revendications 2 à 7, dans lesquelles ladite culture 3D est effectuée dans un appareil qui comprend une matrice de lit fibreux, ladite matrice de lit fibreux comprenant un matériau adhérent synthétique.

9. ASC destinées à être utilisées selon l'une quelconque des revendications 2 à 8, dans lesquelles lesdites ASC ont été incubées dans un appareil de culture de cellules adhérentes 2D avant ladite culture 3D.

10. ASC destinées à être utilisées selon l'une quelconque des revendications 1 à 9, dans lesquelles lesdites ASC sont au moins des cellules maternelles de manière prédominante.

11. ASC destinées à être utilisées selon l'une quelconque des revendications 1 à 9, dans lesquelles lesdites ASC sont au moins des cellules fœtales de manière prédominante.

12. ASC destinées à être utilisées selon l'une quelconque des revendications 1 à 11, dans lesquelles ledit carcinome du sein est triple négatif.

13. ASC destinées à être utilisées selon l'une quelconque des revendications 1 à 12, dans lesquelles lesdites ASC sont administrées systémiquement.

14. ASC destinées à être utilisées selon l'une quelconque des revendications 1 à 12, dans lesquelles lesdites ASC sont administrées de manière intratumorale.
